# EUROPEAN PATENT APPLICATION

(11) **EP 4 180 530 A1**
(43) Date of publication of application: **17.05.2023**
(21) Application number: 22216235.6
(22) Date of filing: 20.12.2019
(51) Int. Cl.: C12Q 1/18, G16C 20/64, G16C 20/70

(54) **ANTIMICROBIC SUSCEPTIBILITY TESTING USING MACHINE LEARNING**

(30) Priority: 31.12.2018 US 201862786678 P; 30.08.2019 US 201962894000 P
(62) Divisional of application: 19845643.6
(71) Applicant: Beckman Coulter, Inc., Brea, CA 92821 (US)
(72) Inventor: SEI, Katherine, Brea, 92821 (US); Chen, Qi, Brea, 92821 (US)
(74) Representative: Boult Wade Tennant LLP

(57) **Abstract**

An optimized testing method is used to determine minimum inhibitory concentration (MIC) of a particular antimicrobic for use on a sample. This may include iteratively imaging wells inoculated with the sample and containing various concentrations of the antimicrobic. The images are thereafter processed to determine various characteristic values that are provided as input to a machine learning model.

## Description

### BACKGROUND

Various types of tests related to patient diagnosis and therapy can be performed by analysis of the patient's microorganisms, or "microbes." Microbes are microscopic living organisms such as bacteria, fungi, or viruses, which may be single-celled or multicellular. Biological samples containing the patient's microorganisms may be taken from a patient's infections, bodily fluids or abscesses and may be placed in test panels or arrays, combined with various reagents, incubated, and analyzed to aid in treatment of the patient. Automated biochemical analyzers have been developed to meet the needs of health care facilities and other institutions to facilitate analysis of patient samples and to improve the accuracy and reliability of assay results when compared to analysis using manual operations and aid in determining effectiveness of various antimicrobials. An antimicrobial is an agent that kills microorganisms or inhibits their growth, such as antibiotics which are used against bacteria and antifungals which are used against fungi. However, with ever changing bacterial genera and newly discovered antimicrobials, the demand for biochemical testing has increased in both complexity and volume.

An important family of automated microbiological analyzers function as a diagnostic tool for determining both the identity of an infecting microorganism and of an antimicrobic effective in controlling growth of the microorganism. Automated microbiological analyzers function as a diagnostic tool for determining both the identity of an infecting microorganism and of an antimicrobic effective in controlling growth of the microorganism. In performing the diagnostic tests, identification and in vitro antimicrobic susceptibility patterns of microorganisms isolated from biological samples are ascertained. Conventional versions of such analyzers may place a small sample to be tested into a plurality of small sample test wells in panels or arrays that contain different enzyme substrates or antimicrobics in serial dilutions. Identification (ID) testing of microorganisms, and antimicrobic susceptibility testing (AST) for determining Minimum Inhibitory Concentrations (MIC) of an antimicrobic effective against the microorganism may utilize color changes, fluorescence changes, the degree of cloudiness (turbidity) in the sample test wells created in the arrays, or other information derived from the testing. Both AST and ID measurements and subsequent analysis may be performed by computer controlled microbiological analyzers to provide advantages in reproducibility, reduction in processing time, avoidance of transcription errors and standardization for all tests run in the laboratory.

In ID testing of a microorganism, a standardized dilution of the patient's microorganism sample, known as an inoculum, is first prepared in order to provide a bacterial or cellular suspension having a predetermined known concentration. This inoculum is placed in a plurality of test wells that may contain or thereafter be supplied with predetermined test media. Depending on the species of microorganism present, this media will facilitate changes in color, turbidity, fluorescence, or other characteristics after incubation. These changes are used to identify the microorganism in ID testing.

In AST testing, a plurality of test wells are filled with inoculum and increasing concentrations of a number of different antimicrobial agents, for example antibiotics. The different antimicrobial agents may be diluted in a growth medium or liquid medium to concentrations that include those of clinical interest. After incubation, the turbidity will be increased or unchanged in test wells where growth has not been inhibited by the antimicrobics in those test wells. The MIC of each antimicrobial agent is measured by lack of growth with respect to each concentration of antimicrobial agent. It follows that the lowest concentration of antimicrobial agent displaying a lack of growth is the MIC.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements and in which:
FIG. 1A depicts a portion of a diagrammatic view of an exemplary biological testing system;
FIG. 1B depicts another portion of the diagrammatic view of the biological testing system of FIG. 1A;
FIG. 2 depicts a perspective view of an exemplary incubator system and an exemplary optics system of the biological testing system of FIG. 1B;
FIG. 3 depicts a perspective view of the optics system of FIG. 2;
FIG. 4 depicts another perspective view of the optics system of FIG. 2 showing an XY stage of the optics system;
FIG. 5 depicts a diagrammatic view of portions of the optics system of FIG. 2;
FIG. 6 depicts a diagrammatic view of an exemplary computer system;
FIG. 7 depicts a chart of theoretical growth curves for a microbe;
FIG. 8 depicts a schematic view of an exemplary growth test well for use in the biological testing system of FIGS. 1A and 1B;
FIG. 9 depicts an exemplary image analysis cycle for use in the biological testing system of FIGS. 1A and 1B;
FIG. 10 depicts an exemplary raw image captured by the optics system of FIG. 2;
FIG. 11 depicts an exemplary enhanced image derived from the raw image of FIG. 10;
FIG. 12 depicts an exemplary gradient image derived from the raw image of FIG. 10;
FIG. 13 depicts an exemplary image enhancement flowchart;
FIG. 14 depicts an exemplary dynamic image enhancement flowchart;
FIG. 15 depicts an exemplary segmented image derived from the raw image of FIG. 10;
FIG. 16 depicts an exemplary image segmentation flowchart;
FIG. 17 depicts exemplary microbes having elongated elements;
FIG. 18 depicts exemplary microbes having non-elongated elements;
FIG. 19 depicts an exemplary optimized antimicrobic susceptibility testing method of the present invention;
FIG. 20 depicts an exemplary decision tree that could be used in determining MIC;
FIG. 21 depicts an exemplary decision tree that could be used in determining MIC;
FIG. 22 depicts an exemplary decision tree that could be used in determining MIC;
FIG. 23A depicts a portion of an exemplary decision tree that could be used in determining MIC;
FIG. 23B depicts a portion of an exemplary decision tree that could be used in determining MIC;
FIG. 24 depicts an exemplary structure for a neural network that could provide MIC predictions; and
FIG. 25 depicts an exemplary structure for a neural network that could make growth/inhibition predictions.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

It will be appreciated that any one or more of the teachings, expressions, versions, examples, etc. described herein may be combined with any one or more of the other teachings, expressions, versions, examples, etc. that are described herein. The followingdescribed teachings, expressions, versions, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

### I. Biological Testing System Hardware

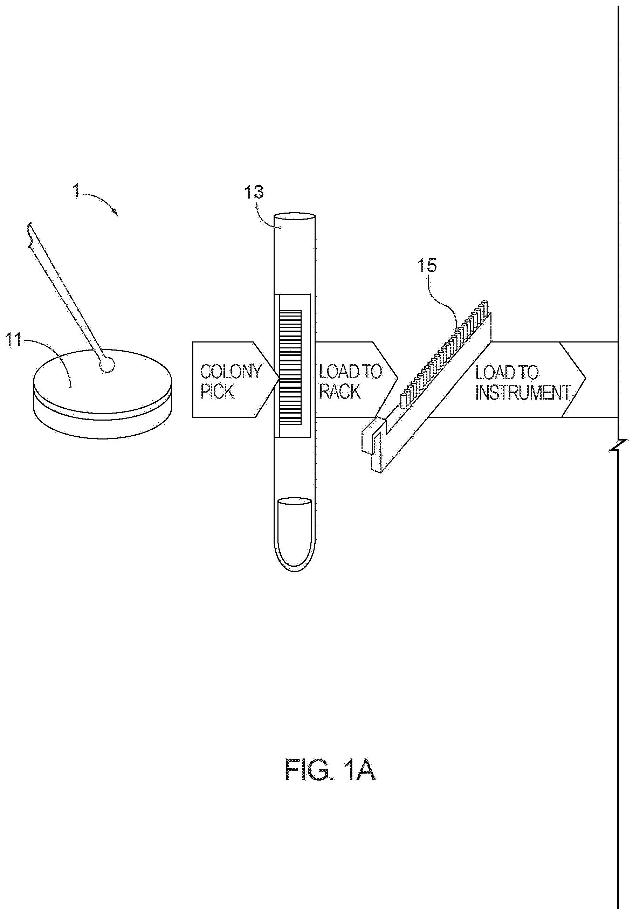

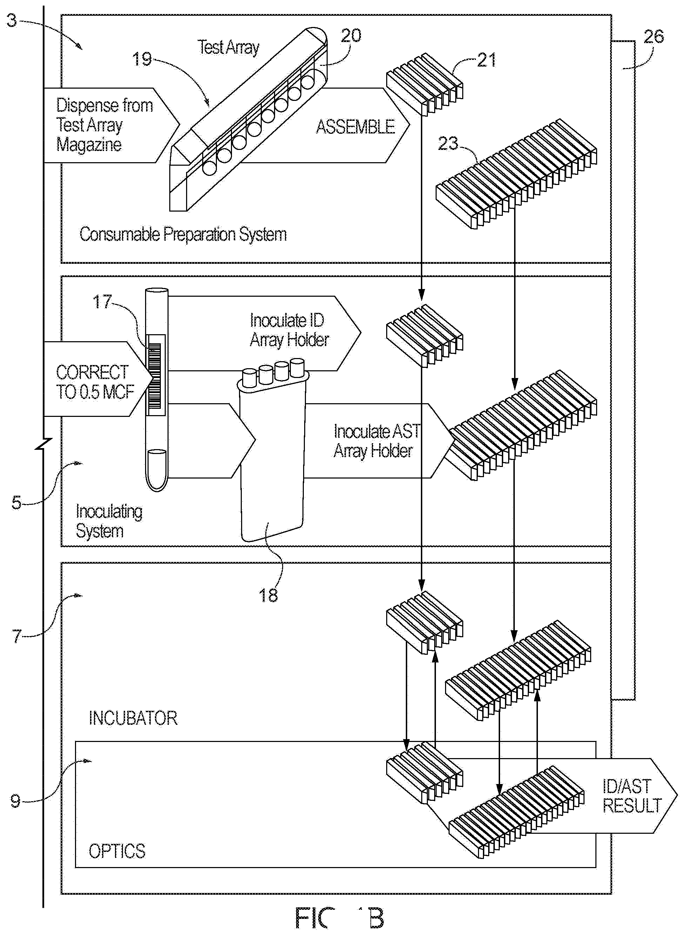

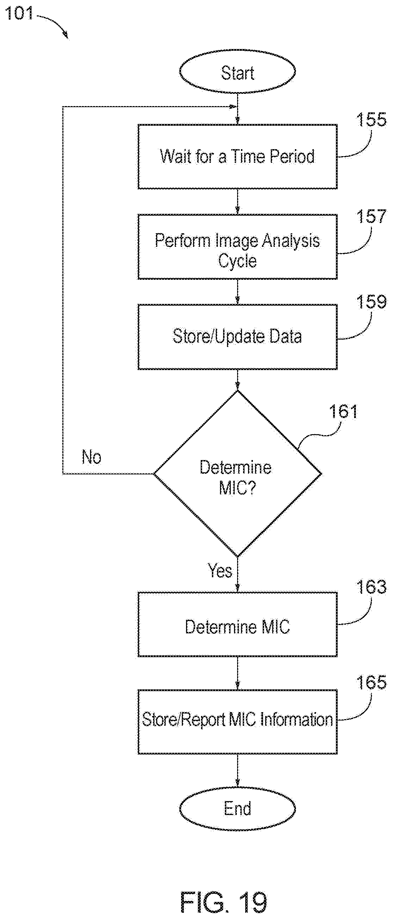

FIGS. 1A and 1B depict a diagrammatical example of various hardware components available in a biological testing system 1. Biological testing system 1 facilitates an optimized antimicrobial susceptibility testing (AST) method 101 (FIG. 19). Biological testing system 1 broadly includes a consumable preparation system 3, an inoculating system 5, an incubator system 7, and an optics system 9. The various systems within biological testing system 1 coordinate with each other and work automatically once loaded with adequate material by a user.

To operate biological testing system 1, the user first acquires an appropriate microbe sample. As shown in FIG. 1A, a microbe sample may be obtained from an agar plate 11 or, under certain circumstances, from a blood sample. Next, the user prepares an inoculum suspension by transferring the microbes into a tube containing a suitable liquid medium or broth. One such tube is shown in FIG. 1A as an inoculum 13. In some versions of biological testing system 1, the liquid medium or broth may be an approximately 0.5 mM phosphate buffered solution with small amounts of sodium and potassium chloride to aid in maintaining the viability of the microbes introduced into solution without adversely interfering with the MIC determination or other associated testing. The phosphate buffered solution may be used in both ID testing and AST testing to minimize the need for different inoculum across the two systems and leverage the efficiencies in having a single broth. Each inoculum 13 is placed into an inoculum rack 15 and the entire inoculum rack 15 is placed into inoculating system 5. Once in inoculating system 5, the inoculum in each inoculum 13 is adjusted if necessary to a standard turbidity value of 0.5 McFarland to create an inoculum 17. In some versions of biological testing system 1, a 1 microliter plastic loop or swab may be provided to the user to easily pick colonies from the agar plate and to minimize the amount of adjustment needed to bring the inoculum to the desired turbidity value. Once adjusted to the desired turbidity value, the inoculum is finalized. The finalized inoculum will be referred to hereinafter as inoculum 17, as depicted in FIG. 1B. Inoculum 17 may be further diluted into a 1:250 dilution and converted into an inoculum 18. As will be discussed in more detail below, the inoculum contained in each inoculum 17 is applied to an identification (ID) array holder 21, while the inoculum contained in each inoculum 18 is applied to an AST array holder 23. Both ID array holder 21 and AST array holder 23 are assembled by consumable preparation system 3 and provided to inoculating system 5 for use with inoculum 17 and inoculum 18.

Consumable preparation system 3 is loaded with magazines of test arrays 19, which may contain various antimicrobials or other agents required by biological testing system 1 disposed in a series of test wells 20. For example, test array 19 may comprise an antimicrobic dilution array or an identification array. Consumable preparation system 3 may also be loaded with bulk diluents (not shown) and/or various other elements for preparing and finalizing ID array holder 21 and AST array holder 23 and the inoculate therein. Primarily, consumable preparation system 3 operates to retrieve test arrays 19 as required and combine each retrieved test array 19 into either ID array holder 21 or AST array holder 23. Test arrays 19 may be selected and assembled by a robotic gripper (not shown) or other mechanical features as dictated by the prescribed testing. For example, a physician may order biological testing using the antibiotic amoxicillin. Test arrays 19 relating to amoxicillin testing are therefore retrieved and assembled into the appropriate ID array holder 21 and AST array holder 23. All or some portions of test array 19 may be formed of a styrene material to aid in reducing fluorescent crosstalk, fallout, and/or bubbles when digitally examining each test well 20.

Once inoculum 17, inoculum 18, ID array holder 21, and AST array holder 23 are assembled, inoculating system 5 dispenses the generally undiluted inoculum from inoculum 17 into test wells 20 of ID array holder 21 and the diluted inoculum from inoculum 18 into test wells 20 of AST array holder 23. The time between applying inoculum 17 to ID array holder 21 or inoculum 18 to AST array holder 23 and the start of logarithmic growth of the microbes disposed therein is known as "lag time." Lag time may be decreased by using enhanced broth such as a broth with yeast extract, vitamins, and/or minerals. Lag time may also be decreased by increasing the inoculum. In some versions of biological testing system 1, the amount of inoculum may be doubled to decrease the lag time by approximately 30 minutes without affecting the accuracy of the MIC determination. The dispensing may be accomplished via an elevator assembly 26 having an XY robot or XYZ robot (not shown) with a gripper (not shown) and pipettor (not shown), along with various circuitry, channels, and tubing as necessary. The XYZ robot is tasked with retrieving inoculum from inoculum racks 15 and dispensing the inoculum into test wells 20 of ID array holder 21 and AST array holder 23. Once ID array holder 21 and AST array holder 23 are sufficiently loaded with inoculum, each ID array holder 21 and AST array holder 23 are moved into incubator system 7 by way of an elevator assembly 26.

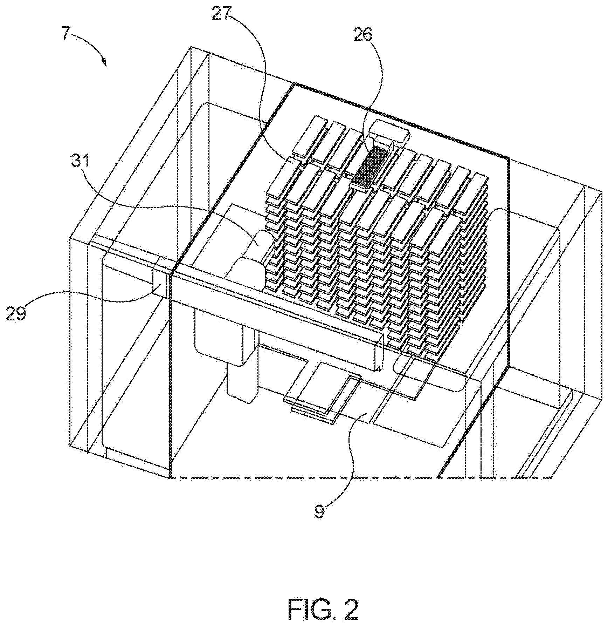

As shown in FIG. 2, incubator system 7 includes slots 27 for holding a large number of ID array holders 21 and AST array holders 23. Each array holder is placed into a corresponding slot 27 by an XYZ robot 29 using a gripper 31. XYZ robot 29 operates to move in any portion of the XYZ plane and position gripper 31 proximate the desired ID array holder 21 or AST array holder 23. While in incubator system 7, each array holder incubates in specific desired environmental conditions. For example, incubator system 7 may be set to incubate array holders at thirty-five degrees Celsius. At certain time intervals during the incubation, XYZ robot 29 retrieves a particular ID array holder 21 or AST array holder 23 and moves the selected array holder into the optics system 9.

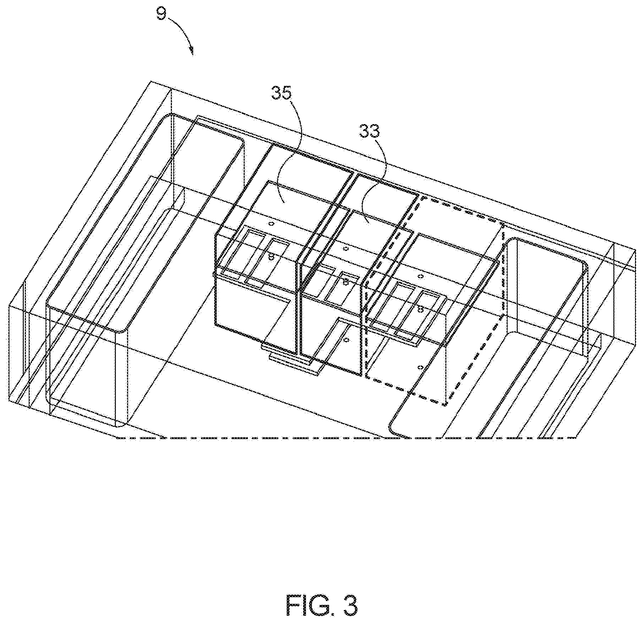

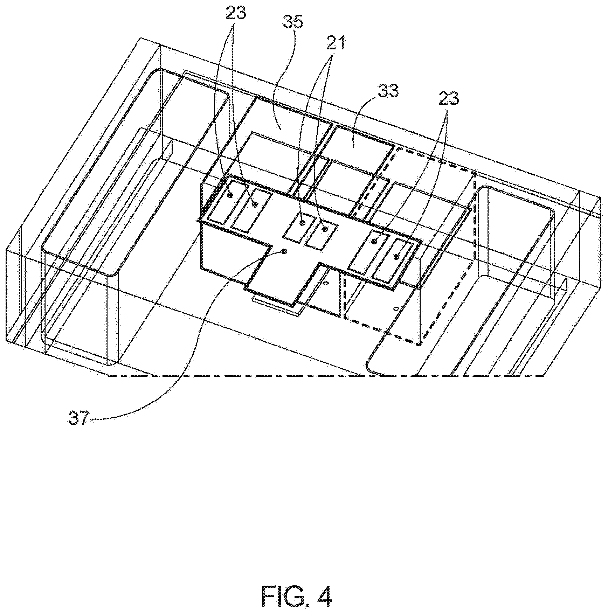

As shown in FIG. 2-4, optics system 9 includes features that are configured to observe, monitor, review, and/or capture images for each test well 20 of an ID array holder 21 or AST array holder 23. Specifically, each ID array holder 21 is monitored by an ID fluorimeter 33, and each AST array holder 23 is monitored by an AST camera 35. To accomplish the monitoring, XYZ robot 29 retrieves the particular array holder with gripper 31 and places the selected array holder onto an XY-stage 37. The XY-stage 37 moves in the XY plane to position the array holder under the associated monitoring element, namely, the ID array holders 21 are disposed under the ID fluorimeter 33 and the AST array holders 23 are disposed under the AST camera 35 for monitoring and observation in optics system 9. XY-stage 37 includes finely tuned motor control to allow each test well 20 of the associated array holder to be positioned accurately within the observation frame of either ID fluorimeter 33 or AST camera 35.

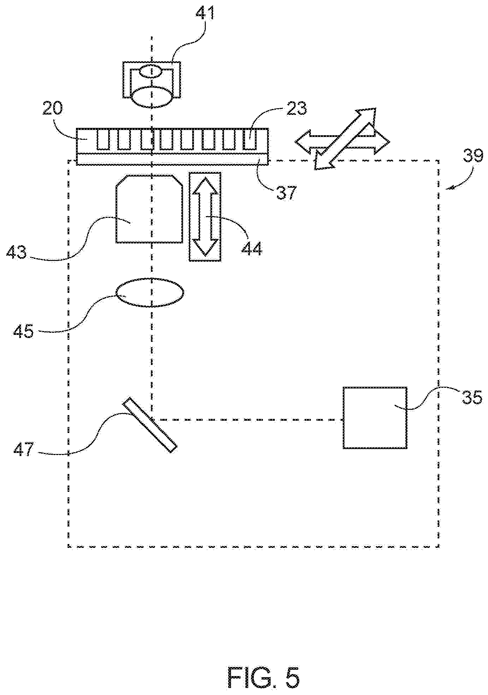

FIG. 5 illustrates an exemplary architecture for an AST optics portion 39 of optics system 9. AST optics portion 39 includes an illumination source 41, an objective lens 43, a tube lens 45, and a fold mirror 47. Illumination source 41 may comprise a condenser LED system for providing monochromatic illumination of each test well 20 of AST array holder 23. Objective lens 43 may comprise a Nikon 20x 0.45NA ELWD (extra-long working distance) objective lens, an Olympus 10X objective lens or any other suitable kind of lens. Objective lens 43 may comprise a 20x objective lens with each pixel covering about 0.33 microns. A 20x objective lens provides both the ability to detect a reasonable number of cells at the beginning of cell growth (around 100-200 cells) and the ability to detect cell morphology. Objective lens 43 may comprise a 10x objective lens and/or a 5MP camera for a larger dynamic range and/or a bigger sample of each test well 20 while maintaining enough resolution to count the microbes therein. In some exemplary embodiments of optics system 9, only one picture or image per test well 20 per pass is acquired by objective lens 43. Objective lens 43 may focus slightly off the bottom of test well 20 to eliminate background noise from the bottom of test well 20. In some versions of optics system 9, objective lens 43 is configured to focus approximately 5-10 microns from the bottom of test well 20. In some versions of optics system 9, objective lens 43 is configured to focus 8 microns from the bottom of test well 20. Objective lens 43 may also include a Z-stage 44 for allowing objective lens 43 to move in the Z-axis, relative to XY-stage 37. Thus, between XY-stage 37 moving AST array holder 23 in the XY plane and Z-stage 44 moving objective lens 43 in the Z-axis, each test well 20 of array holder 23 may be moved in any three-dimensional space to precisely align test wells 20 with the frame of AST camera 35. Tube lens 45 may be embodied in an achromatic tube lens. AST camera 35 may comprise a Sony IMX253 camera, various types of 5 megapixel cameras provided by other manufacturers such as Canon, Thorlabs, or Sentech, or any other suitable kind of camera. In some versions of optics system 9, XY-stage 37 and Z-stage 44 are replaced with an XYZstage to provide all three axes of three-dimensional movement of test wells 20.

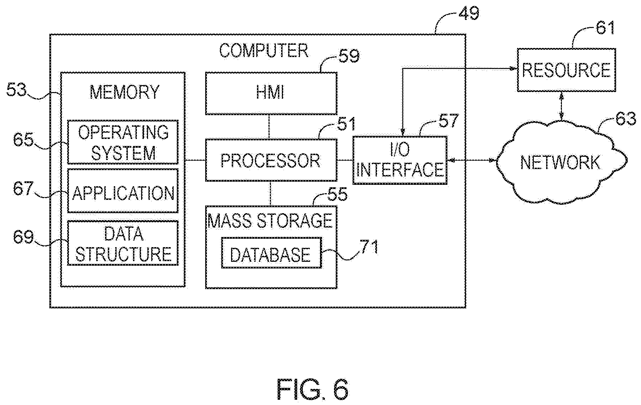

Referring now to FIG. 6, the various components of biological testing system 1 may incorporate one or more computing devices or systems, such as exemplary computer system 49. For example, any one of consumable preparation system 3, inoculating system 5, incubator system 7, and/or optics system 9 may incorporate one or more computing systems such as exemplary computer system 49. Alternatively, each of these subsystems of biological testing system 1 may function via commands from one overall computing system such as exemplary computer system 49.

Computer system 49 may include a processor 51, a memory 53, a mass storage memory device 55, an input/output (I/O) interface 57, and a Human Machine Interface (HMI) 59. Computer system 49 may also be operatively coupled to one or more external resources 61 via a network 63 or I/O interface 57. External resources may include, but are not limited to, servers, databases, mass storage devices, peripheral devices, cloud-based network services, or any other suitable computer resource that may be used by computer system 49.

Processor 51 may include one or more devices selected from microprocessors, micro-controllers, digital signal processors, microcomputers, central processing units, field programmable gate arrays, programmable logic devices, state machines, logic circuits, analog circuits, digital circuits, or any other devices that manipulate signals (analog or digital) based on operational instructions that are stored in memory 53. Memory 53 may include a single memory device or a plurality of memory devices including, but not limited, to read-only memory (ROM), random access memory (RAM), volatile memory, non-volatile memory, static random access memory (SRAM), dynamic random access memory (DRAM), flash memory, cache memory, or any other device capable of storing information. Mass storage memory device 55 may include data storage devices such as a hard drive, optical drive, tape drive, non-volatile solid state device, or any other device capable of storing information.

Processor 51 may operate under the control of an operating system 65 that resides in memory 53. Operating system 65 may manage computer resources so that computer program code embodied as one or more computer software applications, such as an application 67 residing in memory 53, may have instructions executed by the processor 51. In an alternative embodiment, processor 51 may execute application 67 directly, in which case the operating system 65 may be omitted. One or more data structures 69 may also reside in memory 53, and may be used by processor 51, operating system 65, or application 67 to store or manipulate data.

The I/O interface 57 may provide a machine interface that operatively couples processor 51 to other devices and systems, such as network 63 or external resource 61. Application 67 may thereby work cooperatively with network 63 or external resource 61 by communicating via I/O interface 57 to provide the various features, functions, applications, processes, or modules comprising embodiments of the invention. Application 67 may also have program code that is executed by one or more external resources 61, or otherwise rely on functions or signals provided by other system or network components external to computer system 49. Indeed, given the nearly endless hardware and software configurations possible, persons having ordinary skill in the art will understand that different versions of the invention may include applications that are located externally to computer system 49, distributed among multiple computers or other external resources 61, or provided by computing resources (hardware and software) that are provided as a service over network 63, such as a cloud computing service.

HMI 59 may be operatively coupled to processor 51 of computer system 49 in a known manner to allow a user to interact directly with the computer system 49. HMI 59 may include video or alphanumeric displays, a touch screen, a speaker, and any other suitable audio and/or visual indicators capable of providing data to the user. HMI 59 may also include input devices and controls such as an alphanumeric keyboard, a pointing device, keypads, pushbuttons, control knobs, microphones, etc., capable of accepting commands or input from the user and transmitting the entered input to the processor 51.

A database 71 may reside on mass storage memory device 55, and may be used to collect and organize data used by the various systems and modules described herein. Database 71 may include data and supporting data structures that store and organize the data. In particular, database 71 may be arranged with any database organization or structure including, but not limited to, a relational database, a hierarchical database, a network database, or combinations thereof. A database management system in the form of a computer software application executing as instructions on processor 51 may be used to access the information or data stored in records of the database 71 in response to a query, where a query may be dynamically determined and executed by operating system 65, other applications 67, or one or more modules.

### II. Optimized AST System and Method

In some versions, system 1 as discussed above may be used to facilitate some or all of the features provided in optimized AST method 101 such as shown in figure 19.

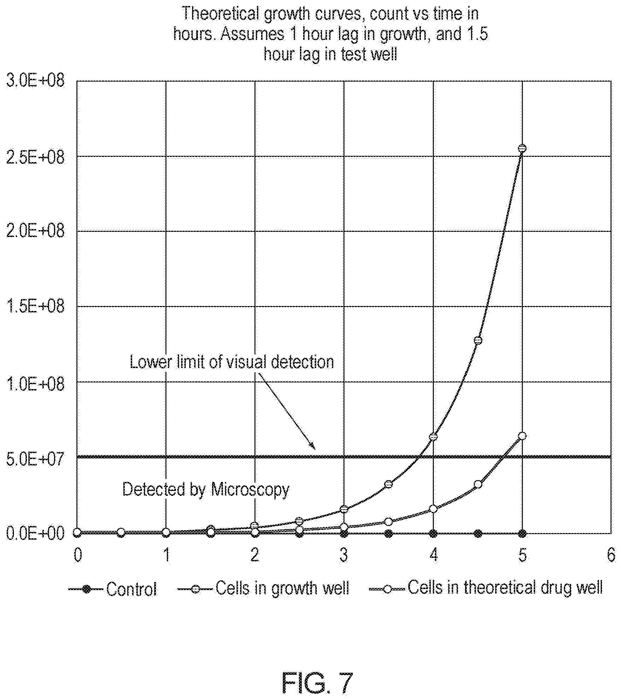

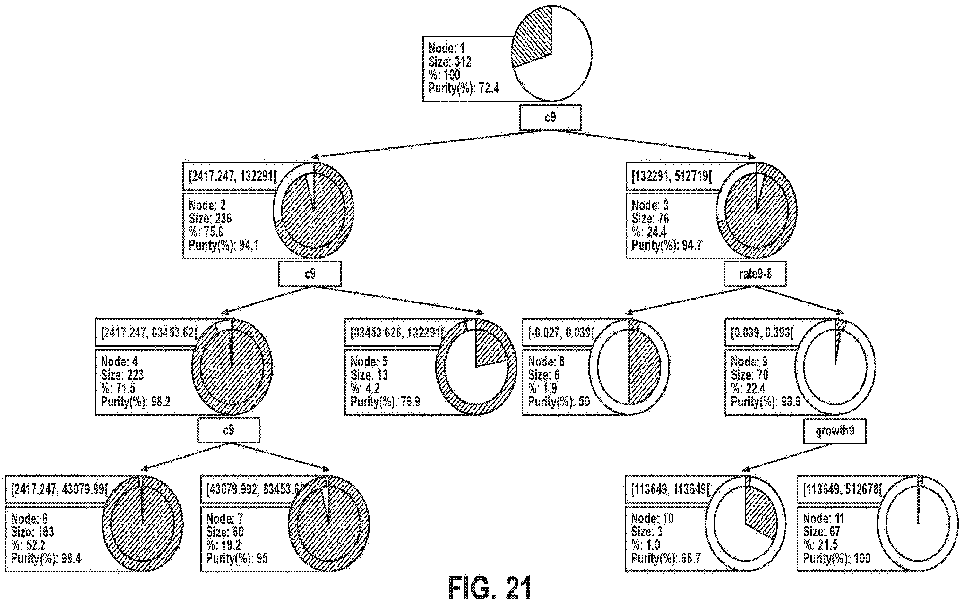

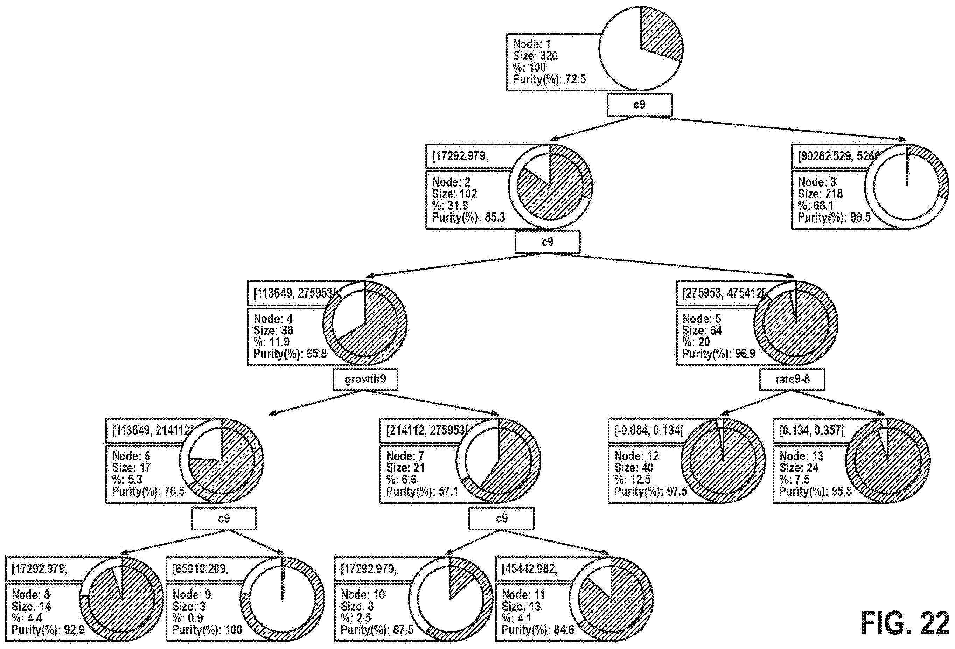

In some conventional processes, MIC is determined through manual visual inspection of test wells after waiting a period to allow the microbes to grow. However, as shown in FIG. 7, microbial sample growth within the test wells is not observable with the naked eye until between four to ten hours after the inoculum is disposed in the test wells. Traditional methods of determining MIC are limited by what a human can visually perceive relative to the growth of the microbes within the test wells. Further, the presence of antimicrobic dilution concentrations that are below the MIC concentration may slow the growth rate, and take even longer to perceive. As shown in FIG. 7, the first six to seven doublings of the microbial sample cannot be observed visually by the human eye. However, the information provided through these initial doublings is often indicative of the MIC. As shown in FIGS. 7-22, optimized AST method 101 utilizes digital microscopy to monitor the area and count of microbes in one more test wells from the point of inoculation and thus allows for more rapid and accurate detection of the MIC.

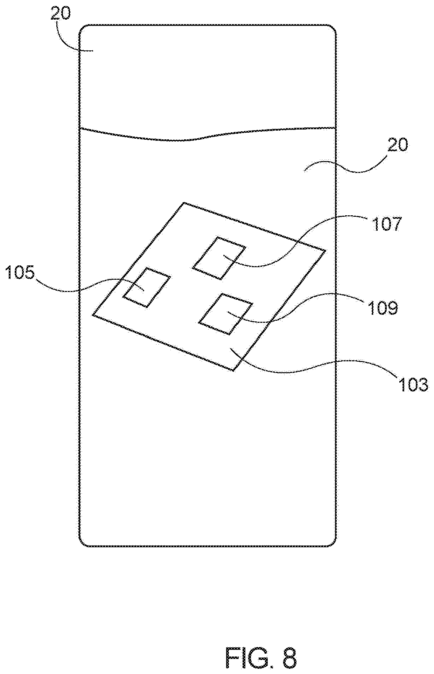

FIG. 8 provides an illustration of how data that can be used in an optimized AST method 101 can be captured from a test well. Test well 20 is embodied by a "384" style test well, having a clear viewing bottom. The volume of inoculum in test well 20 may be set to 20 microliters to reduce the amount of materials such as bulk diluents required by AST method 101 and/or minimize light artifacts and provide sampling of a consistent number of microbes from the 1:250 dilution of the 0.5 MacFarland inoculum. Decreasing the volume of inoculum in test well 20 generally increases the light artifacts. In some instances, capturing a single vertical plane 103 at 20x objective whereby AST camera 35 is set at 0.33 microns/pixel may be sufficient for sampling the inoculum and ensuring that each individual microbe is recognizable. However, these parameters are configurable and may change as desired by the user or the underlying needs of the system. Capturing three focal sites spaced about 5 microns apart within single vertical plane 103 may also provide a sufficient number of microbes in the sample to count for use in optimized AST method 101. These three focal sites are labeled site 105, site 107, and site 109 in FIG. 8. In an exemplary version of optimized AST method 101, each focal site is approximately 700 x 700 microns within single vertical plane 103, rather than capturing three sites in three different vertical planes. AST camera 35, optics system 9, and computer 49 are configured to capture an image of each focal site in consecutive time periods, manipulate each of these images, and thereafter use the data derived from these manipulated images to make a MIC determination.

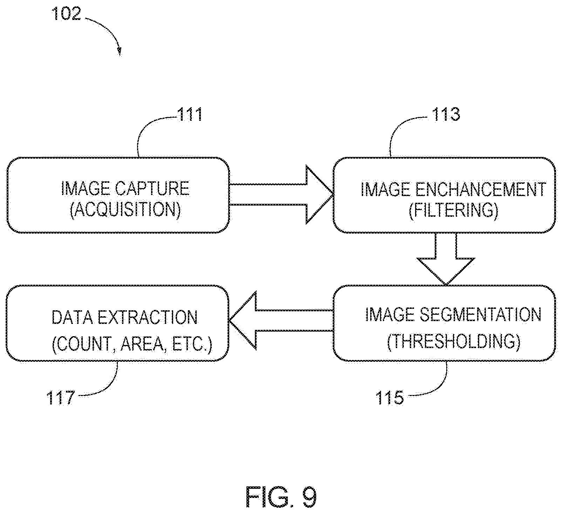

Image analysis cycle 102 is generally depicted in FIG. 9 and comprises an image capture step 111, an image enhancement step 113, an image segmentation step 115, and an object counting step 117. Optimized AST method 101 may include performing image analysis cycle 102 repetitively until a MIC is determined. Image analysis cycle 102 utilizes one or more instances of computer 49 and the various elements thereof to perform image capture step 111, image enhancement step 113, image segmentation step 115, and data extraction step 117, as well as any sub steps provided therein.

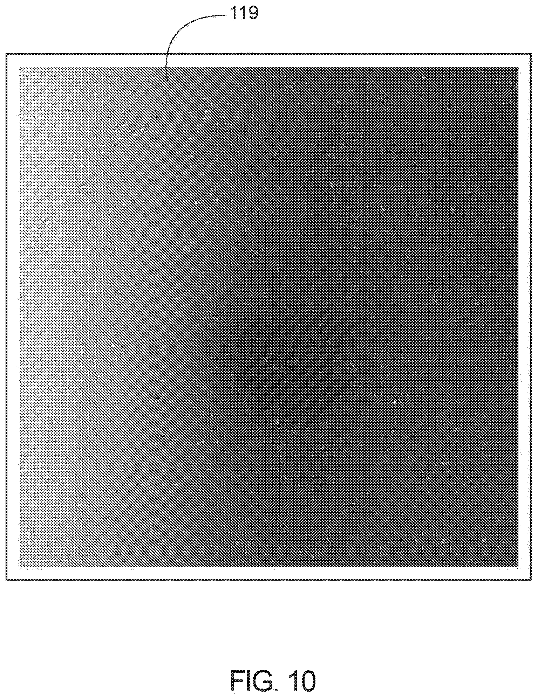

Image analysis cycle 102 begins with image capture step 111 and captures a raw image 119 (FIG. 10) of the inoculum via AST camera 35 of optics system 9 and stores raw image 119 in memory 53. While raw image 119 is depicted as a single image, raw image 119 may be a composite of several separate images taken in vertical plane 103, for example, a composite of site 105, site 107, and site 109. Raw image 119 may also be a composite of several images taken in different planes within the inoculum sample or may be a single image. As illustrated in FIG. 10, raw image 119 may include deficiencies such as uneven illumination. Uneven illumination may be a result of the meniscus created by the inoculum in combination with the walls of the associated test well 20, which may affect the path of illumination source 41. Uneven background intensity may also be caused by environmental issues such as plastic deformation or from a variety of other sources. After raw image 119 is captured in image capture step 111, image capture step 111 moves to image enhancement step 113.

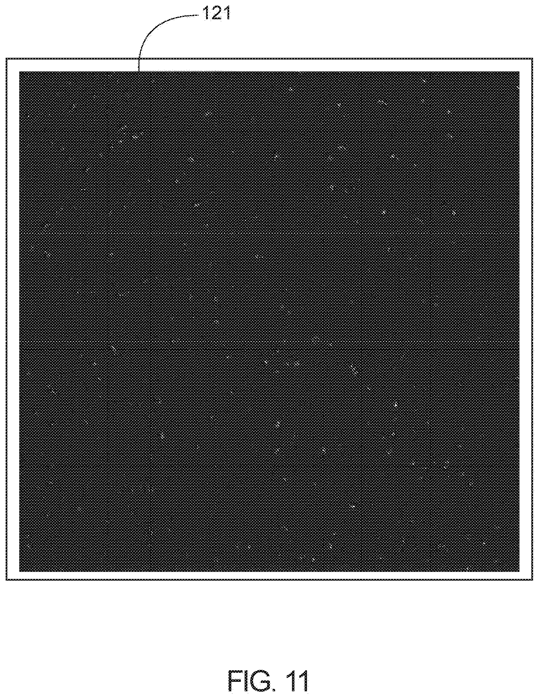

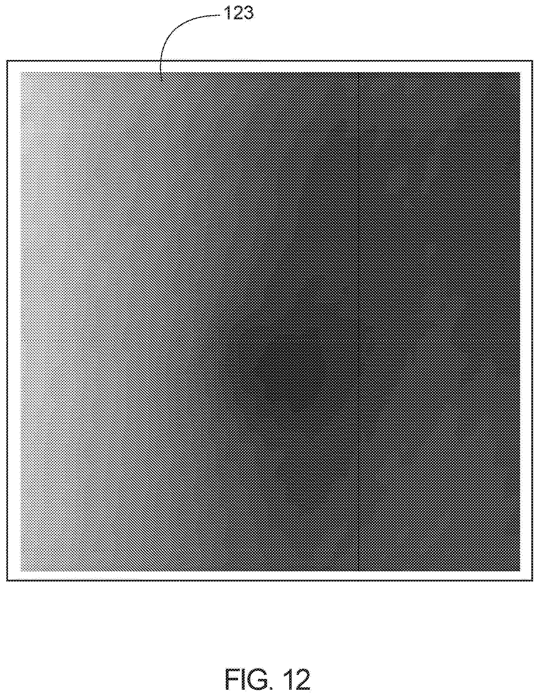

As shown in FIGS. 9, and 11-14, image enhancement step 113 processes raw image 119 to create an enhanced image 121 (FIG. 11), whereby enhanced image 121 is more suitable for the process of counting and recognizing microbes such as bacteria. During image enhancement step 113, one or more attributes of raw image 119 are modified. These attributes may include basic gray level transformations, noise filtering, and median filtering. For example, to resolve the problem of non-uniform illumination, a median filter may be applied to raw image 119 to arrive at a gradient image 123 (FIG. 12). This may be accomplished by selecting a pixel radius sufficient to provide a resulting image containing only the gradient of the background illumination of raw image 119. Gradient image 123 is then subtracted from raw image 119 to correct for the uneven illumination and generate enhanced image 121 free of uneven illumination.

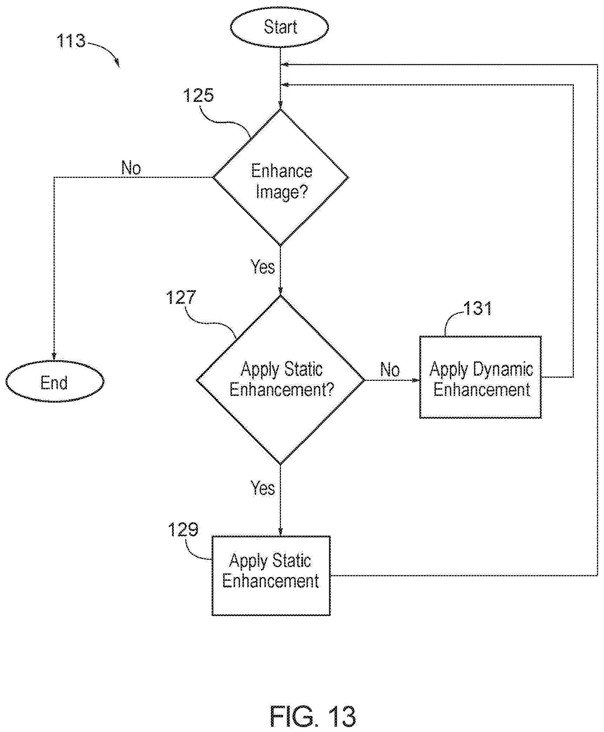

Image enhancement step 113 may apply image enhancements either statically, dynamically, or both. For example, the pixel radius for the median filter may be a statically set constant value or may be adaptively derived dynamically from characteristics of each raw image 119 captured through optics system 9. As shown in FIG. 13, some versions of image enhancement step 113 may include a step 125, whereby a determination is made as to whether the working image should undergo enhancement. If step 125 determines the working image should be enhanced, step 125 proceeds to a step 127. In step 127, a determination is made as to whether a static enhancement should be applied. If step 127 determines a static enhancement should be applied, step 127 proceeds to a step 129 where a static enhancement is applied. If step 127 determines a static enhancement should not be applied, step 127 proceeds to a step 131. In step 127, a static enhancement is applied to the working image and step 127 proceeds back to step 125. In step 131, a dynamic enhancement is applied to the working image and step 131 proceeds back to step 125. If step 125 determines the working image should not be further enhanced, step 125 proceeds to end.

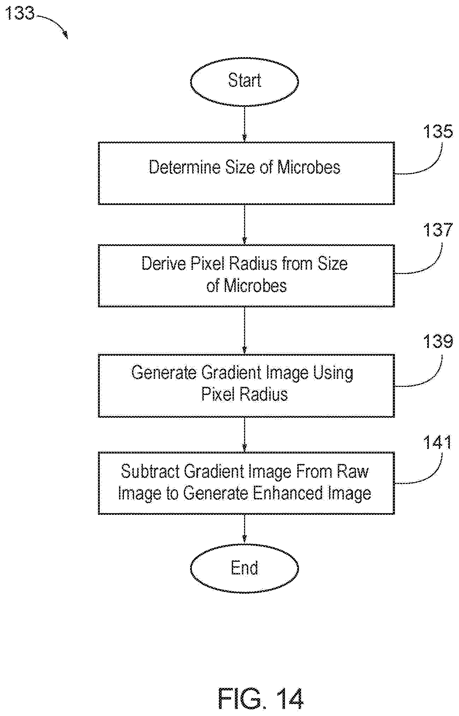

FIG. 14 illustrates an example of a method of dynamic image enhancement 133. Method of dynamic image enhancement 133 is directed to dynamically determining the appropriate pixel radius for use in a median filter enhancement. Method of dynamic image enhancement 133 begins with a step 135, whereby the size of the microbes depicted in raw image 119 is determined. The size of the microbes in raw image 119 may change depending on various circumstances and parameters associated with the inoculum and the overall optics system 9. Thus, while the literal size of the particular microbe being tested is generally constant in nature, the relative size of the microbes depicted in raw image 119 is dynamic and variable because of differences in parameters such as the lens objectification. Once step 135 determines the size of the microbes in raw image 119, step 135 proceeds to a step 137. In step 137, the pixel radius is derived from the determined size of the microbes. As a general example, if step 135 determines the maximum length of any given microbe in raw image 119 is five pixels, the pixel radius may be determined to be greater than five so that the filtered image only represents the gradient contained in the background. After step 135 derives the pixel radius based off the dynamically determined size of the microbes, step 135 proceeds to step 139. In step 139, gradient image 123 is generated based on processing raw image 119 with the derived pixel radius. After gradient image 123 is generated, step 139 moves to a step 141. In step 141, gradient image 123 is subtracted from raw image 119 to generate enhanced image 121. Thereafter, method of dynamic image enhancement 133 proceeds to end.

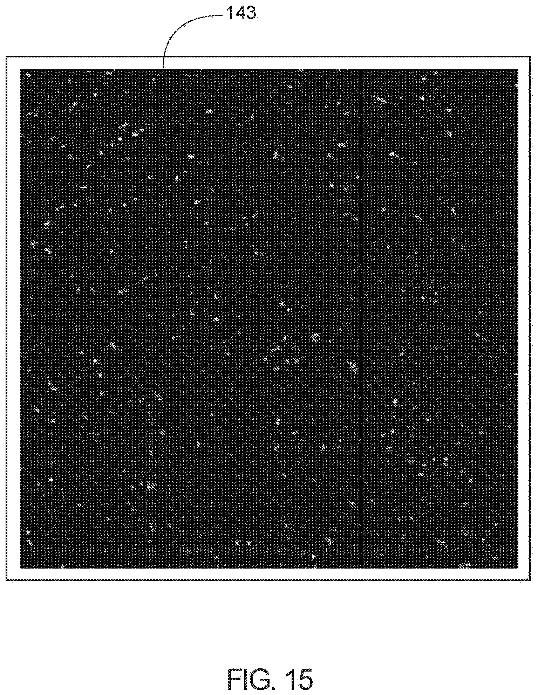

As shown in FIGS. 9, 15, and 16, image segmentation step 115 is used to partition the image into distinct regions containing pixels representing either the microbes as the foreground or the background. Image segmentation step 115 converts enhanced image 121 into a segmented image 143, as shown in FIG. 15. Image segmentation step 115 produces a binary image from enhanced image 121, where every pixel is equal to a value of either 0 or 1, where 0 refers to the background and 1 refers to a portion of a particular microbe.

Image artifacts such as noise may be removed by applying a noise reduction filter prior to applying the segmentation algorithm. Segmentation can be obtained using static threshold value or using an adaptive image thresholding method such as the Otsu cluster based thresholding algorithm. In this algorithm, the gray-level samples are clustered in two parts as background and foreground (object), or alternatively are modeled as a mixture of two Gaussians. The threshold value for the particular image thresholding algorithm used may be determined dynamically, depending on the overall image provided to image segmentation step 115 and the relative grayscale levels of the image. For example, inoculating system 5 or another element of system 1 may be configured to apply nigrosin to each test well 20 to enhance the image, as nigrosin does not attach to certain microbes such as bacteria. This may alter the relative greyscale levels in raw image 119 and require a different threshold value for the segmentation algorithm, as compared to a raw image 119 without nigrosin. In some versions of optimized AST method 101, threshold values may be determined dynamically by searching for edges within several areas of the image. These edges are the transition point between the background and a microbe. Thus, the threshold value can then be calculated as the average greyscale value for pixels on each side of the located edge.

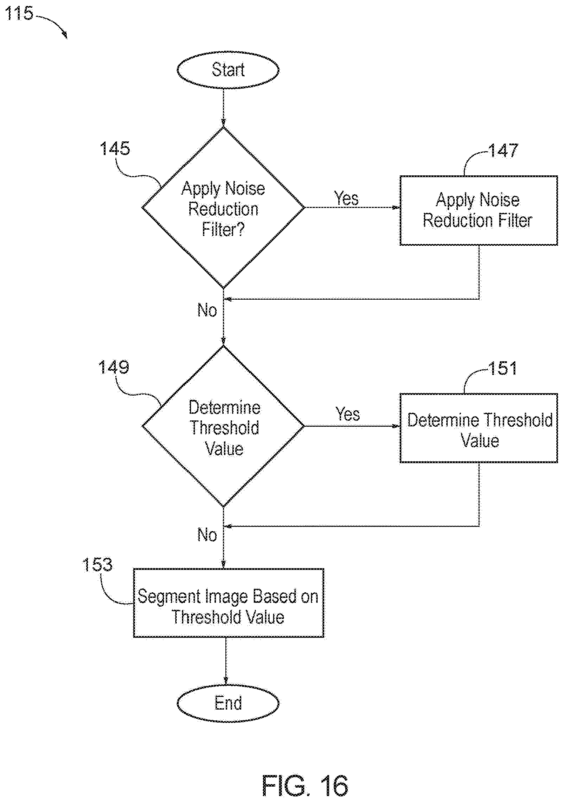

As shown in FIG. 16, some versions of image segmentation step 115 may begin with a step 145. In step 145, a determination is made regarding whether to apply a noise reduction filter to enhanced image 121. If step 145 determines a noise reduction filter should be applied, step 145 proceeds to a step 147 where the noise reduction filter is applied. Step 147 thereafter proceeds to a step 149. If step 145 determines that a noise reduction filter should not be applied, step 145 proceeds directly to step 149. In step 149, a decision is made regarding whether to dynamically determine a threshold value. If step 149 decides a threshold value should be dynamically determined, step 149 proceeds to a step 151 where the threshold value is determined. Step 149 thereafter proceeds to a step 153. If step 149 decides not to dynamically determine a threshold value, a static predetermined threshold value is used and step 149 proceeds directly to step 153. In step 153, enhanced image 121 is segmented using the selected threshold value and step 153 and thereafter image segmentation step 115 proceeds to end.

Once image segmentation step 115 generates segmented image 143, image segmentation step 115 proceeds to data extraction step 117. In data extraction step 117, the background and foreground pixels are considered to derive information regarding the number of microbes in the sample (Count - C), the area occupied by the microbes in the sample (Area - A), and the ratio between the area occupied by the microbes and the number of microbes (A/C). In some versions of data extraction step 117, the actual microbe count is compared with an average microbe count to determine if an error occurred within the image capture process. The comparison may incorporate a standard deviation with the average microbe count to generalize the microbe comparison.

Data extraction step 117 may be configured to derive information regarding the number of microbes in the image. In some embodiments of data extraction step 117, the number of foreground pixels in segmented image 143 may be counted in accordance with a predefined width and/or length to determine the number of microbes in the imaged portion of the inoculum. The counting algorithm may be divided into two separate algorithms, one for counting rod shaped microbes and one for counting spherical shaped microbes as the profile of the underlying microbes provides a corresponding different foreground pixel shape in segmented image 143. For example, the counting algorithm may be configured to consider a square of 2 × 2 pixels a microbe for counting purposes for spherical shaped microbes, or may consider a rectangle of 1 × 4 pixels a microbe for counting purposes for rod shaped microbes. Further, the counting algorithm may be configured to process both algorithms in order to capture the different three-dimensional orientations of rod shaped microbes. For example, if an elongated rod is positioned endwise towards AST camera 35, it will have a much different profile when viewed in two dimensions through AST camera 35. Therefore, both of the counting algorithms may be used during the counting phase of image analysis cycle 102. Alternatively, the counting algorithm may be configured to consider and count any foreground pixels surrounded by background pixels as a microbe.

Data extraction step 117 may be configured to derive information regarding the total area occupied by all of the microbes in the image. In some versions of data extraction step 117, the total number of foreground pixels in segmented image 143 may be counted and compared to the total number of background pixels in segmented image 143. Data extraction step 117 may express the area count information in any format, including as a percent such as 30%, or as a literal number of pixels such as "138 foreground pixels out of 450 total pixels" or "138 foreground pixels and 312 background pixels."

Data extraction step 117 may be configured to derive information regarding the ratio between the total count of microbes and the total area occupied by all of the microbes in the image. This information may be useful in determining whether the microbes are undergoing elongation over time. Elongation is a precursor to death and indicates the concentration of the antimicrobic dilution is negatively affecting the microbes. More specifically, elongation may occur when microbes such as bacteria encounter an effective amount of antibiotic drugs.

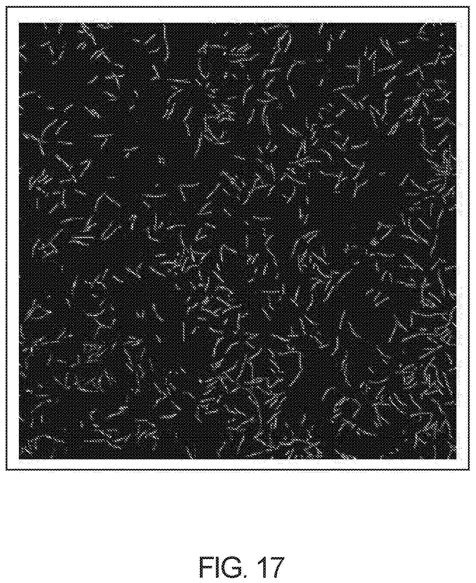

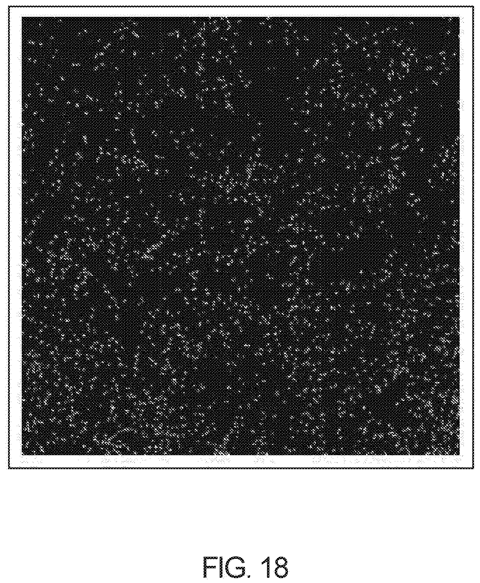

For example, FIGS. 17 and 18 depict microbes embodied by *E. coli* bacteria. FIG. 17 depicts the *E. coli* after being exposed to a dilution of the antibiotic ampicillin, while FIG. 18 depicts the *E. coli* free from any ampicillin exposure. FIG. 17 illustrates abnormal growth in the form of elongation as a result of the ampicillin exposure. This elongation is a precursor to the death of the bacteria and illustrates that the concentration of ampicillin is sufficient to neutralize the bacteria. FIG. 18 illustrates normal growth. It follows that the ratio between the total area occupied by all of the bacteria and the bacteria count will increase over time to represent each bacterium elongating. For example, if a particular image sample includes 100 bacteria and each bacterium measures approximately 4 pixels each, the area occupied by the 100 bacteria within the image is 400 pixels. In this image sample, the ratio of bacteria area to bacteria count is 4.0. Over time, if each bacterium in the sample elongates to approximately 12 pixels each, the area occupied by the 100 bacteria in a later image is 1200 pixel and the ratio of bacteria area to bacteria count in this later image is 12.0. This increase in ratio indicates that the concentration of ampicillin is effective in neutralizing the *E. coli* bacteria because the bacteria are elongating, which in turn indicates an impending death.

Once data extraction step 117 derives the desired information from segmented image 143, image analysis cycle 102 terminates. Optimized AST method 101 iteratively performs image analysis cycle 102 at set time intervals to determine how the microbes in each test well 20 are changing and reacting to the particular antimicrobic dilution pairing. Further, optimized AST method 101 iteratively performs image analysis cycle 102 on each test well 20 associated with the microbes to determine how the microbes are reacting to each concentration of the antimicrobic dilution. For example, presume the microbes being tested are *E. coli* bacteria and three test wells 20 are being tested, with each test well 20 having a 20-microliter solution therein. The first test well 20 may contain an antimicrobic dilution of 1 microgram per milliliter (µg/ml), the second test well 20 may contain an antimicrobic dilution of 2 µg/ml, and the third test well 20 may contain an antimicrobic dilution of 4 µg/ml. Optimized AST method 101 performs image analysis cycle 102 on each of the three test wells at each set time interval to determine (a) how each antimicrobic dilution is affecting the microbes; and (b) how each antimicrobic dilution is performing relative to the other antimicrobic dilutions. If the data indicates the 1 µg/ml antimicrobic dilution is as effective as the 2 and 4 µg/ml antimicrobic dilutions at neutralizing the microbes, the 1 µg/ml antimicrobic dilution is the MIC.

An exemplary version of optimized AST method 101 is illustrated in FIG. 19 and begins with a step 155. In step 155, the system waits for a set time period threshold to allow the microbes within the selected test well 20 enough time to provide new information regarding the growth rate or reaction to the particular antimicrobic dilution. Optimized AST method 101 may preferably be configured to utilize a time period of 30 minutes for this threshold, though different thresholds may be used in different embodiments and/or with different microorganisms. For example, some bacteria or yeast or other microbes may react very quickly and provide information relevant to making an MIC determination within an hour. In this scenario, optimized AST method 101 may be configured to perform image analysis cycle 102 every five minutes to capture data regarding the rapidly changing environment within test wells 20. Other microbes may react relatively slowly to antimicrobic dilutions, and therefore a time period threshold of one hour may be more appropriate. Once step 155 waits the specified time period threshold, step 155 moves to a step 157.

In step 157, one iteration of image analysis cycle 102 is performed on a particular microbe with a selected test well 20. As discussed above, an iteration of image analysis cycle 102 derives data regarding the growth rate of the microbes within the selected test well 20. After an iteration of image analysis cycle 102 is performed, step 157 moves to a step 159. In step 159, the data collected in step 157 is stored and/or updated in memory, which may be in the form of a database, a flat file, or any other similar memory or storage device. In some embodiments of optimized AST method 101, step 159 stores the data collected in step 157 in database 71 (FIG. 6). Once step 159 stores/updates the collected data, step 159 moves to a step 161.

In step 161, optimized AST method 101 determines whether enough data has been collected to determine a MIC. This could be done, for example, by determining whether the data collected regarding the test well matched the data used to train a machine learning classifier used in determining a MIC. If more data is needed to accurately determine a MIC, step 161 returns to step 155 and waits to perform another image analysis cycle 102 to collect more data at a future time interval. If step 161 determines a sufficient amount of data has been collected, step 161 proceeds to a step 163.

In step 163, the MIC is determined, and in step 165 it is reported (e.g., via HMI 59). The determination is based on the data collected during each iteration of image analysis cycle 102 for all of the antimicrobic dilutions for a microbial sample as well as for a control well where no antimicrobial agent was present. As described in the following section, this determination may involve applying the collected data to a machine learning classifier that had previously been trained to make growth predictions and then determining the MIC based on which of the test wells was predicted to exhibit growth or to be inhibited.

### III. Machine Learning for Determining Minimum Inhibitory Concentration (MIC)

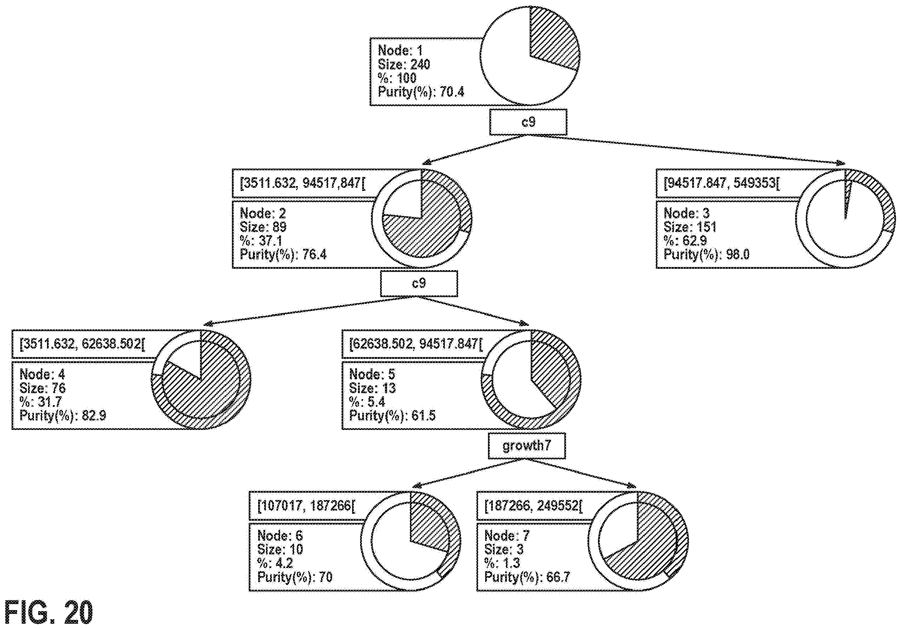

FIG. 20 depicts an exemplary decision tree classifier that could be used in some embodiments for determining MIC using the parameters set forth in table 1. Such a classifier could be created, for example, by collecting a set of training data by periodically (e.g., every 30 minutes, starting with 90 minutes after inoculation and ending with 4 hours after inoculation; every 30 minutes, starting with 90 minutes after inoculation and ending with 6 hours after inoculation; etc.) capturing images of a set of test wells that had been inoculated with a particular microbe and various concentrations of an antimicrobial agent, along with images of a control well that was inoculated with the microbe but did not include any antimicrobial agent, and extracting data from those images such as discussed previously in the context of figure 9. The captured data, or a subset thereof, along with observations of which wells showed growth (G) or inhibition (I) after 16 hours can then be provided to various algorithms, such as QUEST (Quick, Unbiased and Efficient Statistical Tree) or CHAID (Chi-squared Automatic Interaction Detector) to create decision trees that would make predictions of whether a particular well would exhibit growth (G) or inhibition (I). Figures 20-22 show examples of decision trees created in this manner using QUEST, based on the area covered after four hours (referred to in this embodiment as c9), percentage increase in area between 3.5 and 4 hours (calculated as area at 4 hours - area at 3.5 hours divided by area at 3.5 hours, and referred to in this embodiment as r9, or rate9-8), and growth in the control well at four hours (referred to in this embodiment as growth9, or g9) to make predictions after four hours of whether coagulase negative staphyloccoci would show growth or inhibition after 16 hours in the presence of various concentrations of daptomycin, erythromycin, and oxacillin. **T**he resulting decision trees are shown in figures 20 (for oxacillin), 21 (for daptomycin), and 22 (for erythromycin).

In general, the described approach can be used to create decision trees that would make growth predictions for a wide variety of microorganisms in the presence of varying concentrations of a wide variety of antimicrobial agents. **T**ables 1 and 2 list examples of classes of microorganisms and antimicrobials, as well as specific organisms and antimicrobials within those classes, that are believed to be suitable for use with decision tree classifiers of the types described above:

**Table 1**

| **Antimicrobial class** | **Specific antimicrobial** |
|---|---|
| Aminoglycoside | Amikacin |
| | Gentamicin |
| | Tobramycin |
| Ansamvcin | Rifampin |
| Beta-lactam, penicillin class | Penicillin |
| | Oxacillin |
| Beta-lactam + inhibitor combination | Amoxicillin- Clavulanic Acid |
| | Ampicillin-Sulbactam |
| | Ceftazidime-Avibactam |
| | Ceftolozane -Tazobactam |
| | Piperacillin-Tazobactam |
| | Ticarcillin-Clavulanic acid |
| Carbapenems | Doripenem |
| | Ertapenem |
| | Imipenem |
| | Meropenem |
| Cephem | Cefazolin |
| | Cefepime |
| | Cefotaxime |
| | Cefoxitin |
| | Ceftaroline |
| | Ceftazidime |
| | Ceftgriaxome |
| | Ceftizoxime |
| | Cefuroxime |
| Flouoroquinolones | Ciprofloxacin |
| | Levofloxacin |
| Folate Pathway Inhibitor | Trimethoprim-Sulfamethoxazole |
| Fosfomvcin | Fosfomvcin |
| Glycopeptide | Vancomycin |
| Lincosamide | Clindamycin |
| Lipopetide | Daptomycin |
| Macrolide | Erythromycin |
| Oxazolidinone | Linesolid |
| Tetracyclines | Tetracycline |

**Table 2**

| **Microorganism Class** | **Specific Microorganism** |
|---|---|
| Gram-Negative Fermenters | *Aeromonas hydrophilia* |
| | *Citrobacter freundii* |
| | *Citrobacter koseri* |
| | *Enterobacter aerogenes* |
| | *Enterobacter cloacae* |
| | *Eschericia coli* |
| | *Klebsiella oxytoca* |
| | *Klebsiella pneumoniae* |
| | *Morganelle morganii* |
| | *Proteus mirabilis* |
| | *Proteus penneri* |
| | *Proteus vulgaris* |
| | *Providencia stuartii* |
| | *Providendia rettgeri* |
| | *Salmonella paratyphi* |
| | *Salmonella typhi* |
| | *Serratia marcescens* |
| | *Shigella dysenteriae* |
| | *Shigella flexneri* |
| | *Shigella sonnei* |
| | *Vibrio chloerae* |
| | *Yersinia enterocolitica* |
| Gram-Negative Non Fermenters | *Acinetobacter baumannii* |
| | *Acinetobacer haemolyticus* |
| | *Acinetobacter lwoffii* |
| | *Moraxella species* |
| | *Pseudomonas aeruginosa* |
| | *Pseudomonas alcaligenes* |
| | *Pseudomonas fluorescens* |
| | *Pseudomonas putida* |
| | *Stenotrophamonas maltophilia* |
| Gram-Positive Micrococcacaeae | *Staphylococcus aureus* |
| | *Staphylococcus capitis* |
| | *Staphylococcus epidermidis* |
| | *Staphylococcus haemolyticus* |
| | *Staphylococcus saprophyticus* |
| | *Staphylococcus warneri* |
| | *Staphylococcus simulans* |
| | *Staphylococcus lugdenensis* |
| Gram-Positive Streptococcaceae | *Enterococcus avium* |
| | *Enterococcus faecalis* |
| | *Enterococcus faecium* |
| | *Enterococcus gallinarum* |
| | *Enterococcus raffinosus* |
| | *Streptococcus bovis* |
| | *Streptococcus pyogenes* |
| | *Streptococcus pneumoniae* |

It is believed that the above approach can also be used with microbes and antimicrobials not listed in tables 1 and 2. For example, it is believed that decision tree classifiers such as described above could be used to make growth predictions for microorganisms in the enterobacteriaceae family, the pseudomonas family, the acinetobacter family, the micrococcacaeae family and/or the streptococcaceae family.

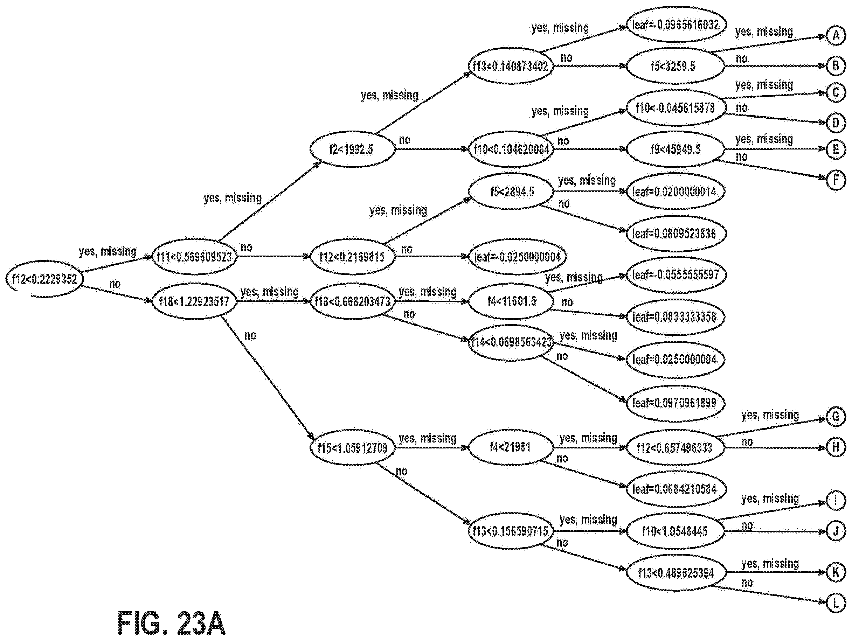

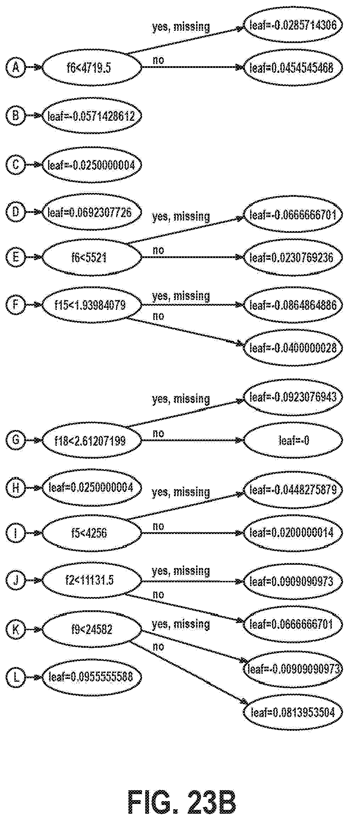

Variations on the above approach are also possible. For example, in some cases, rather than treating classifications directly as growth or inhibition predictions for the test well whose data is provided to a decision tree, they could be subjected to further processing to create a classification. For example, in some embodiments, when data is provided to a decision tree such as shown in FIGs. 23A and 23B, the number of leaves connected to their parent nodes by yes (i.e., inhibition) branches could be compared with the number of leaves connected to their parent nodes by no (i.e., growth) branches, and whichever number was greater could be treated as the growth/inhibition prediction for that well. However they were made, once predictions were available for each test well, the concentration in the test well with the lowest antimicrobial concentration that still produced a prediction of inhibition (I) could be treated as the MIC.

**Table 3: Exemplary factors for growth or inhibition decision tree**

| **Factors** | **Definitions** |
|---|---|
| F0 | Count at 120 minutes |
| F1 | Count at 150 minutes |
| F2 | Count at 180 minutes |
| F3 | Count at 210 minutes |
| F4 | Count at 240 minutes |
| F5 | Growth at 120 minutes |
| F6 | Growth at 150 minutes |
| F7 | Growth at 180 minutes |
| F8 | Growth at 210 minutes |
| F9 | Growth at 240 minutes |
| F10 | Rate at 150 minutes; (Count at 150 minutes-Count at 120 minutes)/Count at 120 minutes |
| F11 | Rate at 180 minutes; (Count at 180 minutes-Count at 150 minutes)/Count at 150 minutes |
| F12 | Rate 210; (Count at 210 minutes-Count at 180 minutes)/Count at 180 minutes |
| F13 | Rate 240; (Count at 240 minutes-Count at 210 minutes)/Count at 210 minutes |
| F14 | Rel 120; Count of growth (i.e., well inoculated with microorganisms but not containing any antimicrobial) well/count of drug well at 120 minutes |
| F15 | Rel 150; Count of growth well/count of drug well at 150 minutes |
| F16 | Rel 180; Count of growth well-count of drug well at 180 minutes |
| F17 | Rel 210; Count of growth well-count of drug well at 210 minutes |
| F18 | Rel 240; Count of growth well-count of drug well at 240 minutes. |

Other approaches are also possible, and will be immediately apparent to those of ordinary skill in the art. For example, in some embodiments, rather than training individual decision trees, training techniques such as XG Boost could be used to generate ensembles of multiple trees whose outputs could be combined to provide higher reliability predictions. There could be various numbers of trees in such an ensemble, from 2 trees to several hundred trees. During testing, when XG Boost software was used to create an ensemble of 500 decision trees that used the factors set forth in table 3 based on training data gathered using the gram negative bacteria of *A. baumannii, C. freundii cplx, E. aerogenes, E. cloacae, E. coli, K. oxytoca, K. pneumoniae, P. aeruginosa, P. fluorescens, S. marcescens, Salmonella sp., P. mirabilis, P. rettgeri* and the antibiotics of cefepime (a beta-lactam), cefotaxime, ceftazidime, gentamicin, levofloxacin, meropenem, and tetracycline, the predictions provided by the resulting ensemble after four hours were found to have the agreement set forth in table 4 with the actual growth/inhibition observations taken after 18 hours.

**Table 4: Accuracy measurements obtained during decision tree testing (with one indeterminable result in which two successive wells when ordered by concentration were provided an inhibit classification while the next well in terms of concentration provided a growth classification - a situation that, in production, would preferably be treated as indicating a technical error or growth that is on a borderline and should be confirmed by manual intervention after the full 16-18 hour growth period).**

| **Bias** | **Relative to external reference** |
|---|---|
| <=-2 | 0 |
| (i.e., the predicted MIC was less than the actual MIC, and there was at least one test well with a concentration of the antimicrobial that was both greater than the predicted MIC and less than the actual MIC) | |
| -1 | 12 |
| (i.e., the predicted MIC was less than the actual MIC, but there was no test well with a concentration of the antimicrobial that was both greater than the predicted MIC and less than the actual MIC) | |
| 0 | 132 |
| (i.e., agreement in MIC) | |
| 1 | 2 |
| (i.e., the predicted MIC was less than the actual MIC, but there was no test well with a concentration of the antimicrobial that was both less than the predicted MIC and greater than the actual MIC) | |
| >=2 | 2 |
| (i.e., the predicted MIC was greater than the actual MIC, and there was at least one test well with a concentration of the antimicrobial that was both less than the predicted MIC and greater than the actual MIC) | |
| Essential Agreement | 98% |
| (i.e., the predicted MIC matched the actual MIC, or, if there was disagreement, there were no test wells that had a concentration that was closer to the actual MIC than the predicted MIC was) | |
| Absolute Agreement | 88.6% |
| (i.e., the predicted and actual MIC matched) | |

Unexpectedly, during this testing, it was found that this ensemble was suitable for making growth predictions for each of the microorganisms tested, rather than a separate ensemble or tree being necessary for each microorganism or for each antimicrobial. As a result, in some embodiments a biological testing system may be configured with an ensemble of decision trees that would be used for any kind of gram negative bacteria regardless of the antimicrobial being tested or the particular gram negative bacteria in a sample, rather than using different decision trees or ensembles for each microorganism and antimicrobial. Of course, it is also possible that individual decision trees or ensembles could be trained on data for, and used to provide MIC predictions for, specific combinations of microorganisms and antimicrobials. Accordingly, the discussion above of applying decision tree classifiers and ensembles and the unexpected result that an ensemble classifier trained to use the parameters specified in table 3 could be applied to multiple microorganisms and antimicrobials should be understood as being illustrative only and should not be treated as limiting.

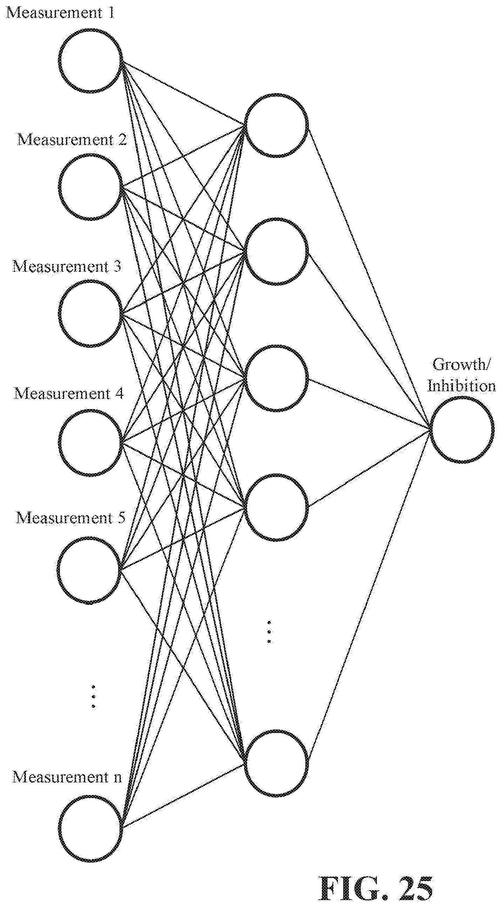

Other types of machine learning models could also be used to make MIC predictions. For example, in some embodiments, rather than using a decision tree such as shown in FIGs. 20-23B (or an ensemble of such trees), a neural network having a structure such as shown in FIG. 25 could be used. When a neural network of this type was trained to make growth/inhibition predictions using the area covered by microorganisms, the count of microorganisms (defined for purposes of this training as one micron objects), the ratio of area to count, and the ratio of area covered by microorganisms in the test well to the area covered by microorganisms in a growth (no antimicrobial) well from readings taken every 30 minutes from 0 to 4 hours using a digital microscope at 10X magnification, it was found to provide the results shown in table 5 when tested on 130 microorganism/antimicrobial combinations. The particular organisms and antimicrobials used in this test were *A. baumannii, C. freundii cplx, E. cloacae, E. coli, K. pneumoniae, K. oxytoca, P. aeruginosa, P. fluorescens, S. marcescens, Salmonella sp., P. mirabilis, P. rettgeri,* and Cefepime, Cefotaxime, Ceftazadime, Gentamicin, Levofloxacin, Meropenem, Tetracycline. The input nodes, output nodes, hidden layer code and response mapping code set for a neural network trained in this manner is set forth in table 6. Of the 130 microorganism/antimicrobial combinations tested, the neural network provided a G or I prediction that led to an interpretable MIC for 126 of the tests. Four test results were identified as uninterpretable because they included the sequence of I, I, G following a previous G, I, G pattern (e.g., 0.03 G, 0.06 G, 0.12 G, 0.25 G, 0.5 I, 1 G, 2 I, 4 I, 8 G, 16 I) which was (and in production could be) treated as indicative of a technical error or weak signal that would require manual intervention after a full 16-18 hour growth period.

**Table 5: Accuracy measurements obtained during testing of neural network G/I predictions.**

| **Bias** | **Relative to dried reference with known MIC** | **Relative to external reference** |
|---|---|---|
| <=-2 | 6 | 8 |
| (i.e., the predicted MIC was less than the actual MIC, and there was at least one test well with a concentration of the antimicrobial that was both greater than the predicted MIC and less than the actual MIC) | | |
| -1 | 21 | 23 |
| (i.e., the predicted MIC was less than the actual MIC, but there was no test well with a concentration of the antimicrobial that was both greater than the predicted MIC and less than the actual MIC) | | |
| 0 (i.e., agreement in MIC) | 90 | 76 |
| 1 | 7 | 16 |
| (i.e., the predicted MIC was less than the actual MIC, but there was no test well with a concentration of the antimicrobial that was both less than the predicted MIC and greater than the actual MIC) | | |
| >=2 | 2 | 3 |
| (i.e., the predicted MIC was greater than the actual MIC, and there was at least one test well with a concentration of the antimicrobial that was both less than the predicted MIC and greater than the actual MIC) | | |
| Essential Agreement | 94% | 91% |
| (i.e., the predicted MIC matched the actual MIC, or, if there was disagreement, there were no test wells that had a concentration that was closer to the actual MIC than the predicted MIC was) | | |
| Absolute Agreement | 71% | 60% |
| (i.e., the predicted and actual MIC matched) | | |

**Table 6: Exemplary neural network parameters and code.**

| |
|---|
| /^{∗}%INPUT: AC90 */ - i.e., the ratio of area to count of microorganisms in the test well after 90 minutes. |
| /*%INPUT: AC120 */ - i.e., the ratio of area to count of microorganisms in the test well after 120 minutes. |
| /*%INPUT: AC150 */ - i.e., the ratio of area to count of microorganisms in the test well after 150 minutes. |
| /*%INPUT: AC180 */ - i.e., the ratio of area to count of microorganisms in the test well after 180 minutes. |
| /*%INPUT: AC210 */ - i.e., the ratio of area to count of microorganisms in the test well after 210 minutes. |
| /*%INPUT: AC240 */ - i.e., the ratio of area to count of microorganisms in the test well after 240 minutes. |
| /*%INPUT: A90 */ - i.e., area (defined as total number of pixel covered in the field) covered by microorganisms in the test well after 90 minutes. |
| /*%INPUT: A120 */ - i.e., area covered by microorganisms in the test well after 120 minutes. |
| /*%INPUT: A150 */ - i.e., area covered by microorganisms in the test well after 150 minutes. |
| /*%INPUT: A180 */ - i.e., area covered by microorganisms in the test well after 180 minutes. |
| /*%INPUT: A210 *J - i.e., area covered by microorganisms in the test well after 210 minutes. |
| /*%INPUT: A240 *J - i.e., area covered by microorganisms in the test well after 240 minutes. |
| /*%INPUT: C90 */ - i.e., count of microorganisms (defined as 1 micron objects) in the test well after 90 minutes |
| /*%INPUT: C120 */ - i.e., count of microorganisms in the test well after 120 minutes |
| /*%INPUT: C150 */ - i.e., count of microorganisms in the test well after 150 minutes |
| /*%INPUT: C180 */ - i.e., count of microorganisms in the test well after 180 minutes |
| /*%INPUT: C210 */ - i.e., count of microorganisms in the test well after 210 minutes |
| /*%INPUT: C240 */ - i.e., count of microorganisms in the test well after 240 minutes |
| /*%INPUT: gac90 */ - i.e., the ratio of area to count in the growth (no antimicrobial) well after 90 minutes |
| /*%INPUT: gac120 */ - i.e., the ratio of area to count in the growth well after 120 minutes. |
| /*%INPUT: gac150 */ - i.e., the ratio of area to count in the growth well after 150 minutes |
| /*%INPUT: gac180 */ - i.e., the ratio of area to count in the growth well after 180 minutes. |
| /*%INPUT: gac210 */ - i.e., the ratio of area to count in the growth well after 210 minutes. |
| /^{∗}%INPUT: gac240 */ - i.e., the ratio of area to count in the growth well after 240 minutes. |
| /*%INPUT: gc90 */ - i.e., the count of microorganisms in the growth well after 90 minutes |
| /^{∗}%INPUT: gc120 */ - i.e., the count of microorganisms in the growth well after 120 minutes |
| /*%INPUT: gc150 */ - i.e., the count of microorganisms in the growth well after 150 minutes |
| /*%INPUT: gc180 */ - i.e., the count of microorganisms in the growth well after 180 minutes |
| /*%INPUT: gc210 */ - i.e., the count of microorganisms in the growth well after 210 minutes |
| /*%INPUT: gc240 */ - i.e., the count of microorganisms in the growth well after 240 minutes |
| /*%INPUT: rateac90 */ - i.e., ratio of area to count after 90 minutes less the ratio of area to count after 60 minutes divided by the ratio of area to count after 60 minutes. |
| /*%INPUT: rateac120 */ - i.e., ratio of area to count after 120 minutes less the ratio of area to count after 90 minutes divided by the ratio of area to count after 90 minutes |
| /*%INPUT: rateac150 */ - i.e., ratio of area to count after 150 minutes less the ratio of area to count after 120 minutes divided by the ratio of area to count after 120 minutes |
| /*%INPUT: rateac180 */ - i.e., ratio of area to count after 180 minutes less the ratio of area to count after 150 minutes divided by the ratio of area to count after 150 minutes |
| /*%INPUT: rateac210 */ - i.e., ratio of area to count after 210 minutes less the ratio of area to count after 180 minutes divided by the ratio of area to count after 180 minutes |
| /*%INPUT: rateac240 */ - i.e., ratio of area to count after 240 minutes less the ratio of area to count after 210 minutes divided by the ratio of area to count after 210 minutes |
| /*%INPUT: arate90 */ - i.e., the area covered by microorganisms at 90 minutes less the area covered by microorganisms at 60 minutes divided by the area covered by microorganisms at 60 minutes. |
| /*%INPUT: arate120 */ - i.e., the area covered by microorganisms at 120 minutes less the area covered by microorganisms at 90 minutes divided by the area covered by microorganisms at 90 minutes. |
| /*%INPUT: aarate150 */ - i.e., the area covered by microorganisms at 150 minutes less the area covered by microorganisms at 120 minutes divided by the area covered by microorganisms at 120 minutes. |
| /*%INPUT: arate180 */ - i.e., the area covered by microorganisms at 180 minutes less the area covered by microorganisms at 150 minutes divided by the area covered by microorganisms at 150 minutes. |
| /*%INPUT: arate210 */ - i.e., the area covered by microorganisms at 210 minutes less the area covered by microorganisms at 180 minutes divided by the area covered by microorganisms at 180 minutes. |
| /*%INPUT: arate240 */ - i.e., the area covered by microorganisms at 240 minutes less the area covered by microorganisms at 210 minutes divided by the area covered by microorganisms at 210 minutes. |
| /*%INPUT: crate90 */ - i.e., the count of microorganisms at 90 minutes, less the count of microorganisms at 60 minutes, divided by the count of microorganisms at 60 minutes. |
| /*%INPUT: crate120 */ - i.e., the count of microorganisms at 120 minutes, less the count of microorganisms at 90 minutes, divided by the count of microorganisms at 90 minutes. |
| /*%INPUT: crate150 */ - i.e., the count of microorganisms at 150 minutes, less the count of microorganisms at 120 minutes, divided by the count of microorganisms at 120 minutes. |
| /*%INPUT: crate180 */ - i.e., the count of microorganisms at 180 minutes, less the count of microorganisms at 150 minutes, divided by the count of microorganisms at 150 minutes. |
| /*%INPUT: crate210 */ - i.e., the count of microorganisms at 210 minutes, less the count of microorganisms at 180 minutes, divided by the count of microorganisms at 180 minutes. |
| /*%INPUT: crate240 */ - i.e., the count of microorganisms at 240 minutes, less the count of microorganisms at 210 minutes, divided by the count of microorganisms at 210 minutes. |
| /*%INPUT: crelg90 */ - i.e., the count of microorganisms in the growth well divided by the count of microorganisms in the test well after 90 minutes. |
| /*%INPUT: crelg120 */ - i.e., the count of microorganisms in the growth well divided by the count of microorganisms in the test well after 120 minutes. |
| /*%INPUT: crelg150 */ - i.e., the count of microorganisms in the growth well divided by the count of microorganisms in the test well after 150 minutes. |
| /*%INPUT: crelg180 */ - i.e., the count of microorganisms in the growth well divided by the count of microorganisms in the test well after 180 minutes. |
| /*%INPUT: crelg210 */ - i.e., the count of microorganisms in the growth well divided by the count of microorganisms in the test well after 210 minutes. |
| /*%INPUT: crelg240 */ - i.e., the count of microorganisms in the growth well divided by the count of microorganisms in the test well after 240 minutes. |
| /*%INPUT: arelg90 */ - i.e., the area covered by microorganisms in the growth well divided by the area covered by microorganisms in the test well after 90 minutes. |
| /*%INPUT: arelg120 */ - i.e., the area covered by microorganisms in the growth well divided by the area covered by microorganisms in the test well after 120 minutes. |
| /*%INPUT: arelg150 */ - i.e., the area covered by microorganisms in the growth well divided by the area covered by microorganisms in the test well after 150 minutes. |
| /*%INPUT: arelg180 */ - i.e., the area covered by microorganisms in the growth well divided by the area covered by microorganisms in the test well after 180 minutes. |
| /*%INPUT: arelg210 */ - i.e., the area covered by microorganisms in the growth well divided by the area covered by microorganisms in the test well after 210 minutes. |
| /*%INPUT: arelg240 */ - i.e., the area covered by microorganisms in the growth well divided by the area covered by microorganisms in the test well after 240 minutes. |
| /*%OUTPUT: target_G ^{∗}/ |
| /*%OUTPUT: target_I ^{∗}/ |
| LABEL target_G = 'Predicted: target=G'; |
| LABEL target_I = 'Predicted: target=I'; |
| /* Transformation Code */ |
| /* Hidden Layer Code */ |
| H1 = tanh(.5*(-0.76929772039729*AC90 + -0.517996995555927*AC120 + 0.213910040155609*AC150 + 0.340855993369109*AC180 + - 0.376320895999207*AC210 + -0.208692171049202*AC240 + - 0.0000859715871971256*A90 + -0.0000748964265814192*A120 + - 0.0000214755459177721*A150 + -0.0000150683732181355*A180 + - 0.0000132676978635083*A210 + -0.0000017095245443214*A240 + - 0.00302622289987604*C90 + 0.000383186617370712*C120 + - 0.00010108715828128*C150 + -0.0000092895701357271*C180 + 0.0000069320398064624*C210 + -0.0000131552306469186*C240 + 2.24453402415695*gac90 + -0.0654035630054382*gac120 + 0.746261432760748*gac150 + 1.04596894347035*gac180 + - 1.37805823921968*gac210 + -0.710709842225726*gac240 + - 0.000990040958059944*gc90 + 0.000247482342729981*gc120 + - 0.00011265390069385*gc150 + -0.0000007013178618312*gc180 + 0.0000237674497413957*gc210 + -0.0000519221165318845*gc240 + 1.58537807262008*rateac90 + -0.789928457719473*rateac120 + 8.49115550691271*rateac150 + 6.9049695671945*rateac180 + 5.58600547575419*rateac210 + 3.16053451738922*rateac240 + - 0.212081926513278*arate90 + -1.19262826834769*arate120 + - 2.7939524433148*aarate150 + 2.58569941234477*arate180 + 2.36019926387378*arate210 + -1.72961997725931*arate240 + - 1.16093025092512*crate90 + -1.08680344321621 * crate 120 + 3.84791043942434*crate150 + 1.68902661374424*crate180 + - 2.18647733372242*crate210 + 1.32154265308838*crate240 + 0.652443580928484*crelg90 + -0.0147678550589073*crelg120 + - 0.11137189647759*crelgl50 + -0.00410658919063058*crelg180 + - 0.0125555079348765*crelg210 + 0.00657204167790002*crelg240 + 0.337537063817937*arelg90 + -0.272090304200059*arelg120 + - 0.0509247123553928*arelg150 + -0.00746891903365038*arelg180 + - 0.000663876009395583*arelg210 + -0.0110159281466137*arelg240 + 10.8399000498195)); |
| H2 = tanh(.5*(-1.16976168725335*AC90 + -0.0103877404314782*AC120 + 0.0597313434595071*AC150 + 0.063813028812403*AC180 + 0.236600989973615*AC210 + 0.301349735222163*AC240 + 0.0000364279641799673*A90 + -0.0000347183910346567*A120 + - 0.0000034850198782841*A150 + -0.0000352201747640276*A180 + 0.0000063268515977127*A210 + 0.0000010817320379354*A240 + - |
| 0.000370544513273024*C90 + -0.0000030629381474385*C120 + 0.0000353234367720024*C150 + 0.000069496839662032*C180 + 0.0000291498822474206*C210 + -0.000001632731664515*C240 + 0.984062739064897*gac90 + 0.342619152823296*gac120 + 0.481270170268419*gac150 + 0.531994404757976*gac180 + - 0.487306273355606*gac210 + -0.546473184026972*gac240 + 0.000887486237158816*gc90 + 0.000133510624591607^{∗}gc120 + - 0.0000591682656498541*gc150 + 0.000193531001018708^{∗}gc180 + 0.0000455563784503271*gc210 + 0.0000149082512597672*gc240 + 1.70350222064765*rateac90 + 3.84842530127163*rateac120 + 3.25838583951833*rateac150 + -26.3685823457117*rateac180 + - 2.2673929694755*rateac210 + 6.30473430905984*rateac240 + 0.181409932326034*arate90 + -1.38185695328778*arate120 + 0.432085114414794*aarate150 + 1.69933981414079*arate180 + 1.36615939975567*arate210 + -1.19191992461766*arate240 + - 0.784012323438029*crate90 + 1.3 6579196906343 *crate 120 + - 1.52989242155914*crate150 + 1.71394211731483*crate180 + 2.26340878243152*crate210 + 1.77318566437615*crate240 + - 0.216291201958282*crelg90 + 0.462215614782652*crelg120 + - 0.0120326629626185*crelg150 + 0.0309832643761085*crelg180 + 0.00963006734043858*crelg210 + 0.00376994665498322*crelg240 + 0.469769245558646*arelg90 + 0.062982407301934*arelg120 + - 0.00642266022595118*arelg150 + -0.0122692122186592*arelg180 + 0.0164207145490414*arelg210 + -0.00991536762065987*arelg240 + - 13.2700545620113)); |
| H3 = tanh(.5*(-0.23125927742497*AC90 + 0.51487418372721 * AC120 + - 0.00276380038081496*AC150 + -0.21345122689864*AC180 + - 0.150659051113427*AC210 + -0.588720991231369*AC240 + 0.000145801876953261*A90 + -0.0000538328694348076*A120 + - 0.0000040895927944274*A150 + 0.0000205609951388703*A180 + 0.0000100739994479524*A210 + 0.0000009599798893615*A240 + - 0.0010189814587153*C90 + -0.000247974445009786*C120 + 0.000331409377243441*C150 + 0.0000520043906161321*C180 + - 0.0000144474356790896*C210 + 0.0000992238885446041*C240 + - 2.20718557722135*gac90 + 0.960165341577256*gac120 + 0.676314826623111*gac150 + 0.396749361951956*gac180 + 1.39645493925894*gac210 + 0.0186673963013697*gac240 + 0.00154951785724729*gc90 + -0.000441027278967922*gc120 + - 0.000190264868989225*gc150 + -0.0000351318986160641*gc180 + 0.0000013635727408041*gc210 + 0.0000058640918004133*gc240 + 4.29007934746452*rateac90 + -7.13496480329589*rateac120 + 5.15361172206318*rateac150 + -3.35074986946484*rateac180 + 21.6370986821571*rateac210 + -10.4652454880755*rateac240 + 1.13489397372379*arate90 + 0.158058031651436*arate120 + 1.29684987663805*aarate150 + 1.24879184134775*arate180 + |
| 0.695836955054086*arate210 + 3.98534113414407*arate240 + - 0.214813379406902*crate90 + -0.909401912314647*crate 120 + 0.422667490218204*crate 150 + 2.81399928794881*crate180 + - 1.44665707542257*crate210 + -2.39852954940785*crate240 + - 0.539488395661673*crelg90 + 0.216182839155341*crelg120 + - 0.09828851582002*crelg150 + 0.0456024134884955*crelg180 + 0.00497618634121935*crelg210 + 0.0418462502194032*crelg240 + - 0.0500135564197059*arelg90 + 0.0559897304567657*arelg120 + - 0.0155172872002849*arelg150 + 0.0113929533266592*arelg180 + 0.00411431863921262*arelg210 + 0.0000434203836803232*arelg240 + - 13.2624083931807)); |
| H4 = tanh(.5*(0.198032868712178^{∗}AC90 + 0.0962639169973812*AC120 + 0.540847351699363*AC150 + 0.549866827307403*AC180 + 0.282711194777991*AC210 + -0.620188769770503*AC240 + - 0.000252443669212807*A90 + 0.0000134416335806249*A120 + - 0.0000255176130971388*A150 + -0.0000065594374183819*A180 + 0.0000148101309638108*A210 + -0.0000034397568610409*A240 + 0.000399786393085689*C90 + -0.000258021857345837*C120 + - 0.000125404557662472*C150 + -0.0000804376876092228*C180 + - 0.0000049793301670883*C210 + -0.000068876513782153*C240 + - 1.55565752196062*gac90 + 0.09890442976911*gac120 + 0.165229888293428*gac150 + -0.927753531261078*gac180 + - 0.710561816656981*gac210 + -0.831601796505825*gac240 + - 0.000281033582431464*gc90 + 0.000212056941874682*gc120 + - 0.000175438020537987*gc150 + -0.0000248545198008954*gc180 + 0.0000482200110115973*gc210 + -0.000022842028173449*gc240 + 1.12834245595725*rateac90 + 0.672849335295135*rateac120 + - 17.9879860277004*rateac150 + 2.95853060716756*rateac180 + - 29.2860955820165*rateac210 + -1.46005874904402*rateac240 + - 1.41522012154113*arate90 + 0.228145513880728*arate120 + - 1.31343444925497*aarate150 + -1.12105335309574*arate180 + 0.419473717545843*arate210 + -1.03833261454141*arate240 + - 1.09334869304231 * crate 90 + -0.4457 6267 3 260642* crate 120 + - 0.663345546282067*crate150 + 0.646338128858341*crate180 + - 2.47640701269951*crate210 + -0.244547501161955*crate240 + 0.0399637127865421*crelg90 + -0.207816367449399*crelg120 + 0.0208984850480349*crelg150 + 0.128667977440571*crelg180 + - 0.0248561724805801*crelg210 + -0.00373508058841188*crelg240 + 0.738049496745409*arelg90 + -0.227984716018464*arelg120 + 0.0805909534054533*arelg150 + 0.0274226292877948*arelg180 + 0.0162581442105934*arelg210 + -0.00939963321474055*arelg240 + 26.0151472238864)); |
| H5 = tanh(.5*(0.504220265960324*AC90 + -0.0345693131884963*AC120 + - 0.329395791039983*AC150 + 0.484181730719927*AC180 + 0.172771910298875*AC210 + -0.593893953911198*AC240 + - |
| 0.000339299082187189*A90 + 0.0000368634041542546*A120 + 0.0000180340583880966*A150 + 0.0000120379915412919*A180 + 0.0000021479211035733*A210 + -0.0000042103542305605*A240 + - 0.00210436443826861*C90 + -0.000830309100665913*C120 + 0.000359298585013173*C150 + -0.0000096920558523461*C180 + - 0.0000541143349289768*C210 + -0.0000474785285765752*C240 + 0.32087644506994*gac90 + -1.55193918301016*gac120 + 0.409684725587271*gac150 + -0.538848947373655*gac180 + 0.600618695077408*gac210 + 0.468720303323398*gac240 + 0.00145402504941904*gc90 + 0.000111869532028829*gc120 + 0.000121939349655661*gc150 + 0.0000324309247413687*gc180 + - 0.0000619714372313147*gc210 + -0.0000275659225098164*gc240 + - 3.44408546484537*rateac90 + -7.16463910336051*rateac120 + 9.18428644579943*rateac150 + -3.09753823331883*rateac180 + 5.12148242089875*rateac210 + -10.6034041729504*rateac240 + 1.36506188136571*arate90 + -0.307376949658209*arate120 + - 1.110590347985*aarate150 + -1.63430602013457*arate180 + - 1.93205279709057*arate210 + 1.34499764104793*arate240 + 1.27340778002336*crate90 + -0.556356221081883*crate120 + 2.23490822106322*crate150 + 2.6016943528471*crate180 + 2.4427118925227*crate210 + -1.254126106528*crate240 + - 0.322127931327015*crelg90 + -0.166738720474011*crelg120 + - 0.0405585358436761*crelg150 + -0.13212351765273*crelg180 + - 0.0500662237217763*crelg210 + -0.000867709958905757*crelg240 + 0.205281380206447*arelg90 + -0.0755832922048061*arelg120 + 0.0121406277489595*arelg150 + -0.0112539190130659*arelg180 + - 0.00878172024721804*arelg210 + -0.0136994218079407*arelg240 + 2.82419572783062)); |
| H6 = tanh(.5*(-0.486979209622345*AC90 + 0.215826534309595*AC120 + 0.0646189471704605*AC150 + 0.485693119195833*AC180 + 0.699445540671657*AC210 + 0.351295443311533*AC240 + - 0.0000892980518365641*A90 + 0.0000551074778570986*A120 + - 0.00003894571294848*A150 + -0.0000034400250783069*A180 + 0.0000129989465287948*A210 + 0.0000026572091230457*A240 + - 0.000520878379404837*C90 + 0.000313537408408857*C120 + 0.0000452710843581886^{∗}C150 + -0.0000057465970155675*C180 + - 0.0000436905885619288*C210 + 0.0000008014556654991*C240 + - 1.57399461565226*gac90 + -0.562945638414176*gac120 + 0.765381407582662^{∗}gac150 + -0.0800023874747715^{∗}gac180 + - 0.888603517581868*gac210 + 0.257901393115448*gac240 + - 0.000262518808139924*gc90 + -0.0000190440865428215^{∗}gc120 + 0.0000638659691222125^{∗}gc150 + -0.000006096647467032^{∗}gc180 + 0.0000573611908287384*gc210 + -0.0000090107431977001*gc240 + - 4.24942933290467*rateac90 + -5.4189457686532*rateac120 + 3.29466232336089^{∗}rateac150 + 10.1618084021016*rateac180 + |
| 15.0372787532983*rateac210 + -9.92298879333266*rateac240 + - 0.228180801576929*arate90 + -0.30809132707945*arate120 + - 0.886482437215039*aarate150 + -0.642698436248999*arate180 + - 1.59338873565443*arate210 + -0.0537434739432932*arate240 + 0.426566305434244*crate90 + -0.610895090887034*crate120 + - 2.56982854399168*crate150 + -0.00626661203385239*crate180 + - 1.19545344375921*crate210 + 0.851116352798916*crate240 + 0.0749364858690202*crelg90 + 0.175986952110681*crelg120 + - 0.358636216035897*crelg150 + -0.0665321706099713*crelg180 + 0.0129498726922777*crelg210 + 0.00213901062235324*crelg240 + 0.14472358932098*arelg90 + -0.0055 1 940997279307*arelg 120 + - 0.134127859597497*arelg150 + -0.00601199681319052^{∗}arelg180 + 0.0128377852105935*arelg210 + 0.0177005689299152*arelg240 + 7.19822018309545)); |
| H7 = tanh(.5*(0.922800100119322*AC90 + -0.192116263666656*AC120 + - 0.261217011616777*AC150 + -0.114649990651609*AC180 + - 0.381991777858669*AC210 + 0.526792549809169*AC240 + 0.0000600146966875494*A90 + 0.0000356344650078005*A120 + - 0.0000247955123026378*A150 + -0.000023268538029893*A180 + 0.0000003198441898525*A210 + -0.0000006014879431384*A240 + - 0.000867427222781008*C90 + 0.00040088631695299*C120 + - 0.0000447995206455139*C150 + -0.0000480560361513427*C180 + - 0.0000038994797213073*C210 + -0.0000101572559034211*C240 + 0.819736371297127*gac90 + 1.29222165338821*gac120 + 0.949243181502809*gac150 + 1.56587672356456*gac180 + - 0.260098607103182*gac210 + 0.0672305614103147*gac240 + 0.00218955485493346*gc90 + 0.000494135155341308*gc120 + 0.000136728604296692*gc150 + 0.0000093154348986402*gc180 + - 0.0000287697805129201*gc210 + 0.0000131321140721615*gc240 + 8.8166942008963*rateac90 + -7.13606082073933*rateac120 + - 1.35104947173623*rateac150 + 24.6202538895043*rateac180 + - 6.42635789292955*rateac210 + 9.81179347883468*rateac240 + 1.24526140198831*arate90 + -0.474626854753247*arate120 + - 1.02822426804705*aarate150 + 0.414268781345753*arate180 + 0.825821541418975*arate210 + 1.73317820990469*arate240 + - 0.0121658101175875*crate90 + -0.204161954157909*crate120 + 0.264697157760856*crate150 + 2.30138713903102*crate180 + - 3.60883967051502*crate210 + 0.792644568002972*crate240 + - 0.136023183969718*crelg90 + -0.401620879231302*crelg120 + 0.283737941380147*crelg150 + -0.0244248319142184*crelg180 + 0.0305002294408631*crelg210 + -0.0146131595361351*crelg240 + 0.199112487433329*arelg90 + 0.255973672431188*arelg120 + - 0.0460374715686387*arelg150 + 0.00571098479339529*arelg180 + - 0.00492034912324138*arelg210 + -0.0166740084401507*arelg240 + - 32.3339192422534)); |
| H8 = tanh(.5*(0.0480151571944735*AC90 + 0.411143770013211*AC120 + - 0.256741730605656*AC150 + -0.627668314382401*AC180 + - 1.52631082438893*AC210 + -0.298169664388424*AC240 + - 0.0000112361073100364*A90 + 0.0000232050056584967*A120 + 0.0000094726202023403*A150 + 0.0000066372267988337*A180 + - 0.0000012271929337294*A210 + 0.000000603857933271*A240 + 0.000314246923774086*C90 + -0.000497991759511417*C120 + 0.0000850156198403374*C150 + -0.0000739170815296153*C180 + - 0.000015178246859892*C210 + -0.000017054616811818*C240 + - 0.868404777745801*gac90 + 1.72172539901414*gac120 + 0.172354401504524*gacl50 + 0.0941222214662907*gac180 + - 0.101387859159679*gac210 + 0.909839909256023*gac240 + - 0.001$4621983288096*gc90 + -0.0000979743137741817*gc120 + - 0.0000722260062132027*gc150 + 0.0000090617782679001*gc180 + 0.0000407516548677335*gc210 + -0.0000029009855952103*gc240 + 4.21536837567049*rateac90 + -5.07705379577422*rateac120 + 2.02518797773116^{∗}rateac150 + 17.9515899856501*rateac180 + 1.77488823817555^{∗}rateac210+ 10.3088596034954*rateac240 + - 0.167270008490919*arate90 + 0.415876311427769*arate120 + - 0.318828648294445^{∗}aarate150 + -0.0948375681041915*arate180 + - 2.04488678430714*arate210 + 5.06099305991178*arate240 + 0.613039390014797*crate90 + -1.26785418954062*crate120 + 1.28560574839448*crate150 + -2.42477628867248*crate180 + - 1.4032624092463*crate210 + 3.18750444127045*crate240 + - 0.803015787304634*crelg90 + 0.110351430061777^{∗}crelg120 + 0.0881780857901165*crelg150 + -0.108255720371317^{∗}crelg180 + 0.0228865223331244*crelg210 + 0.00442604766054714*crelg240 + 0.206089736156734*arelg90 + -0.0907827790334528^{∗}arelg120 + 0.0307087915984847*arelg150 + 0.0157307848147905^{∗}arelg180 + - 0.0128816556454105*arelg210 + 0.000918184056748312*arelg240 + 8.10627417213443)); |
| H9 = tanh(.5*(-0.679235556403322^{∗}AC90 + -0.010890357338895*AC120 + - 0.289421149518792*AC150 + -0.135612724903253*AC180 + 0.439684862235223*AC210 + -0.462466024640297*AC240 + - 0.0000911556456892915*A90 + 0.0000915832891336606*A120 + - 0.0000080006189754883*A150 + -0.0000048289767042734*A180 + - 0.0000090096070563177*A210 + -0.0000029782821678333*A240 + 0.00165347549324151*C90 + 0.000108104397665134*C120 + 0.0000072837790544504*C150 + 0.0000447225370295208*C180 + 0.000113254924527369*C210 + -0.00001250748069854*C240 + - 0.133944481010947*gac90 + 0.0745482494923264*gac120 + - 0.626994517893817*gac150 + -0.136249491757382*gac180 + 1.30849891574068*gac210 + -1.03780719604478*gac240 + 0.00160149023634695*gc90 + -0.000561165646851066*gc120 + 0.000159325833627974^{∗}gc150 + -0.0000421074849436144*gc180 + |
| 0.0000212753266592278*gc210 + 0.0000664689285347461*gc240 + 0.926828971202974*rateac90 + 1.26380359315643*rateac120 + - 12.9957082603335*rateac150 + -5.06359809797079*rateac180 + - 9.41465707185079*rateac210 + 10.3606186185046*rateac240 + 0.376466357651106*arate90 + 0.388708428688278*arate120 + - 1.58908882604381*aarate150 + 3.5982054250403*arate180 + - 3.43407199268327*arate210 + 0.320653022690422*arate240 + - 0.464291092432253*crate90 + -1.35100693033303*crate120 + - 1.1506076010921*crate150 + 0.928962715624808*crate180 + - 0.550654379880151*crate210 + 3.19698955324091*crate240 + 0.466333608035302*crelg90 + -0.348895316334491*crelg120 + 0.072416532934002*crelg150 + -0.0306291875675182*crelg180 + - 0.00787026003093296*crelg210 + 0.00613083460385819*crelg240 + - 0.127073345034367*arelg90 + -0.25921419654453*arelg120 + 0.044634272231463*arelg150 + -0.0135695726402604*arelg180 + - 0.0340339608445185*arelg210 + -0.0115610597409519*arelg240 + 8.79055343453187)); |
| H10 = tanh(.5*(-0.460411952497526*AC90 + -0.356249177135773*AC120 + 0.117223552175996*AC150 + -0.136720375927211*AC180 + 0.65627528917308*AC210 + 0.6609471265581*AC240 + 0.000248952849665384*A90 + -0.0000231661704039174*A120 + - 0.0000278081780284062*A150 + 0.0000045154425403604*A180 + - 0.0000019403362637502*A210 + 0.0000094182521601237*A240 + 0.00407860119836337*C90 + -0.000201829515972559*C120 + 0.0000273105474900875*C150 + 0.0000547525031180126*C180 + 0.0000648710601417554*C210 + 0.0000150184629453061*C240 + 1.74460656644976*gac90 + -1.17374412552961*gac120 + 1.02505104414661*gac150 + -0.106991862851777*gac180 + - 1.52843751578974*gac210 + -0.381954765860761*gac240 + - 0.00057773218701188*gc90 + -0.000810211632360701*gc120 + 0.000159051769990732*gc150 + -0.000113132871230933*gc180 + 0.0000103175010704463*gc210 + -0.0000440968231889992*gc240 + - 0.0755046139429835*rateac90 + 3.39471774222593*rateac120 + 3.04255686531225*rateac150 + 6.96395361231604*rateac180 + 17.8285513329132*rateac210 + -9.06468505353683*rateac240 + - 1.20457443977263*arate90 + 0.337308837466915*arate120 + - 0.14937889086361*aarate150 + -2.53403307434271*arate180 + 1.2504014473613*arate210 + 2.05856740006232*arate240 + - 0.862396950878627*crate90 + -1.7138081430$307*crate120 + - 0.114156738960385*cratel50 + -0.372141576914897*crate180 + 1.2451634658057*crate210 + -0.819510225637014*crate240 + - 0.593738467865856*crelg90 + -0.571092937025567*crelg120 + 0.0634006531661149*crelg150 + -0.041154968882571*crelg180 + - 0.00536074884429313*crelg210 + -0.0195612956522412*crelg240 + - 0.334880974334063*arelg90 + -0.123039819832497*arelg120 + |
| 0.0169234013333205*arelg150 + 0.00358183770093567^{∗}arelg180 + - 0.00956552678970241*arelg210 + 0.00820048360182057*arelg240 + 7.23927315654297)); |
| H11 = tanh(.5*(0.402702881985451*AC90 + -0.149568489278787*AC120 + - 0.364740963704104*AC150 + -0.140155842361263*AC180 + 0.56981472444305*AC210 + 0.181916045584535*AC240 + 0.000235200991419037*A90 + -0.000077649651360196*A120 + - 0.0000262415234320835*A150 + 0.0000074342867027753*A180 + - 0.0000043402926214745*A210 + -0.0000064278252295151*A240 + 0.0015442755718229*C90 + 0.000849859918584645*C120 + - 0.000263976603583677*C150 + 0.0000755250957399578*C180 + 0.0000362850759759775*C210 + -0.0000229131504524059*C240 + 3.2887721474413*gac90 + -1.62067340616259^{∗}gac120 + - 0.0132750399130472*gac150 + -0.424504637986779*gac180 + 0.318186118695249*gac210 + -0.70738$130391843*gac240 + - 0.000487982847511352*gc90 + -0.000241238557642085*gc120 + 0.0000458276572997962*gc150 + -0.0000806370364024034*gc180 + - 0.0000163431691345285*gc210 + -0.0000316465011046999*gc240 + 2.53341172273805*rateac90 + 3.8197930123849*rateac120 + 3.72765582784486*rateac150 + -7.7016528553468*rateac180 + - 6.19476083229564*rateac210 + 2.9448330431738*rateac240 + - 0.448720235465344*arate90 + -0.831828255279338*arate120 + 0.704453714963746*aarate150 + -0.64114948743251*arate180 + - 4.0467450144302*arate210 + -3.84470786762897*arate240 + 1.49664170463076*crate90 + -0.066915044762981*crate120 + 1.39233231317662*crate150 + -3.44834943279886*crate180 + 0.216707711026928*crate210 + 1.16906245402301*crate240 + - 0.11737414306435*crelg90 + -0.540525868750333*crelg120 + 0.023621247085068*crelg150 + -0.011013041137903*crelg180 + 0.0277242055781925*crelg210 + -0.00658646558598362*crelg240 + - 0.580316292477111*arelg90 + -0.0240646174267932*arelg120 + 0.0146141579851653*arelg150 + -0.0161994944754035*arelg180 + - 0.0134012490134956*arelg210 + 0.00293770123318858*arelg240 + 0.0616545046141657)); |
| H12 = tanh(.5*(0.290307944108739^{∗}AC90 + -0.361208428283953*AC120 + - 0.129261228962469*AC150 + 0.138920699338544*AC180 + 0.337280444559927*AC210 + 0.205118217890539*AC240 + - 0.0000752731079924833*A90 + -0.0000354605878144513*A120 + 0.0000264563990285212*A150 + 0.0000089900964031345*A180 + 0.0000011808429373295* A210 + 0.0000024955393192588*A240 + - 0.000669479447883587*C90 + 0.000329392859250592*C120 + - 0.0000466326870551753*C150 + 0.0000765682750809706*C180 + - 0.0000333794229133129*C210 + -0.0000349794696277753*C240 + 0.300291670095569*gac90 + 0.467252606331186*gac 120 + - 1.33180250539533*gac150 + 0.227677502522863*gac180 + |
| 0.237360539784565*gac210 + 0.329383259359264*gac240 + 0.000950692608175127*gc90 + 0.0000317427486435546*gc120 + - 0.0000644003112598646*gc150 + -0.000119644429282341*gc180 + - 0.0000538509366730599*gc210 + -0.0000408433095482209*gc240 + - 1.75601788031584*rateac90 + -9.82233662942838*rateac120 + - 12.8715251065264*rateac150 + 7.39911974093001*rateac180 + - 8.00536851353611*rateac210 + 9.22906470829051*rateac240 + 0.806674759661774*arate90 + -0.14574713267563*arate120 + 1.39976563499659*aarate150 + 0.806579900338269*arate180 + 0.746825172789227*arate210 + -2.26410279215533*arate240 + - 0.809785473501959*crate90 + -0.166990216171599*crate120 + - 0.490066949968967*crate150 + -0.209910959238311*crate180 + 2.75888500171999*crate210 + 1.63957011131702*crate240 + - 0.111275073771055*crelg90 + 0.036813688015239*crelg120 + - 0.124970176899258*crelg150 + 0.0939456482475218*crelg180 + - 0.00759024829353812*crelg210 + -0.0211474027594816*crelg240 + - 0.499372800997807*arelg90 + 0.152673229857122*arelg120 + 0.00723227276958711*arelg150 + -0.0143739252144746*arelg180 + 0.00404316672695174*arelg210 + -0.0180870531731817*arelg240 + 1.04014041001072)); |
| H13 = tanh(.5*(-0.082088679529523*AC90 + -0.498201135058846*AC120 + 0.229510398528249*AC150 + 0.11149670269337*AC180 + - 0.192715963490181*AC210 + 0.0979316987344983*AC240 + 0.000138252613337664*A90 + 0.0000363943848357707*A120 + - 0.0000128361989752603*A150 + 0.0000085702675917163*A180 + - 0.0000010170338930147*A210 + 0.000001155404769338*A240 + 0.000902434448662326*C90 + 0.000901810828698837*C120 + 0.000137734179138133*C150 + -0.0000802554992061388*C180 + - 0.0000664065829944779*C210 + -0.0000103429088325919*C240 + - 0.414518519047405*gac90 + 0.998995907271441*gac120 + 0.436780295899905*gac150 + -0.102036010262046*gac180 + 1.92137320722335*gac210 + -0.740987695294658*gac240 + - 0.00147817184609258*gc90 + 0.0000623065118158303*gc120 + - 0.00024467737249005*gc150 + 0.0000274411870324985*gc180 + - 0.00008516146294924*gc210 + 0.0000686288292874607*gc240 + 0.0121172566138649*rateac90 + 3.74487439167338*rateac120 + - 21.8394149398716*rateac150 + -13.652080748199*rateac180 + - 12.1663484733168*rateac210 + -27.2585567517384*rateac240 + 0.932573342845468*arate90 + -0.239535999002346*arate120 + 2.45079062186992*aarate150 + 1.37978554528791*arate180 + - 4.04317737415024*arate210 + 3.50969790494691*arate240 + 0.51024425315595*crate90 + -1.59205255799181*crate120 + 2.26575373288684*crate150 + -1.78584765185464*crate180 + - 1.71757768997192*crate210 + 2.25472320285531*crate240 + - 0.355263342044099*crelg90 + 0.648747544809873*crelg120 + |
| 0.0469668861581257*crelg150 + -0.0482307092824942*crelg180 + - 0.02918964939787*crelg210 + -0.0170452309981668*crelg240 + 0.179901898070414*arelg90 + -0.0637905746573216*arelg120 + - 0.0628305710313126*arelg150 + -0.0302407802948517*arelg180 + 0.00826295007587956*arelg210 + -0.000273457319989386*arelg240 + - 10.438201239948)); |
| H14 = tanh(.5*(-0.503084255562621*AC90 + -0.0582777058540796*AC120 + - 0.427988732484803*AC150 + 0.092477793714351*AC180 + 0.147448194523714*AC210 + 0.182596022911904*AC240 + 0.000019575353474254*A90 + 0.000105211591173437*A120 + 0.0000256867929190916*A150 + -0.0000044975073580685*A180 + 0.0000107510391043544*A210 + 0.0000010753364531966*A240 + 0.000560323422658683*C90 + -0.000596404939181252*C120 + 0.000233715969048373*C150 + 0.0000408597882914648*C180 + - 0.0000211145237018972*C210 + -0.0000520375842080883*C240 + - 0.449135683205585*gac90 + -1.35720301892484*gac120 + 0.661728129977262*gac150 + 0.917641371043244*gac180 + 0.740679737110286*gac210 + -0.989416254154832*gac240 + - 0.000408993504687311*gc90 + 0.000342358631207008*gc120 + 0.000367071413986256*gc150 + -0.0000328762673381467*gc180 + 0.0000583348559365413*gc210 + 0.0000059409525058682*gc240 + - 1.41557901148601*rateac90 + 0.00272136635448701*rateac120 + - 7.05250684974893*rateac150 + -7.76860624789065*rateac180 + - 11.2448377311843*rateac210 + 13.3424890311681*rateac240 + - 1.18803428014463*arate90 + -0.951990221699784*arate120 + 0.725495346642909*aarate150 + 0.637880938106726*arate180 + - 1.04811998683847*arate210 + 7.71392819640569*arate240 + - 0.590504471956235*crate90 + -1.03327592670624*crate120 + 0.254399586172258*crate150 + 1.5067072256674*crate180 + 1.81127861308939*crate210 + 3.27581190530405*crate240 + 0.179672489970462*crelg90 + 0.19001869520362*crelg120 + - 0.236494800307439*crelg150 + 0.031370224167366*crelg180 + 0.000817005163736975*crelg210 + 0.00964159330207101*crelg240 + - 0.173491679939736*arelg90 + -0.139571258137734*arelg120 + - 0.0686660392613448*arelg150 + 0.0209236836150803*arelg180 + - 0.00986151239983715*arelg210 + -0.00966538132277058*arelg240 + - 0.568602506474587)); |
| H15 = tanh(.5*(-0.701639352758717*AC90 + -0.196317261683387*AC120 + - 0.03944198327367*AC150 + -0.093482867192465*AC180 + 0.016541162495529*AC210 + 0.683447719173291*AC240 + 0.0000880799482607006*A90 + -0.0000185383456662717*A120 + 0.0000236379974154888*A150 + 0.0000209263261157999*A180 + 0.00000431309772582*A210 + 0.0000049010269104424*A240 + - 0.00107578972011044*C90 + -0.0000662386065738262*C120 + 0.0000507373163442933*C150 + -0.0000447494839619927*C180 + |
| 0.0000878034416308002*C210 + -0.0000010664275951598*C240 + - 0.220902181723904*gac90 + 1.04012558630981*gac120 + 0.431792043592326*gac150 + -0.0340300328275724*gac180 + - 1.27192882184924*gac210 + 2.38846120255999*gac240 + 0.00122492304563857*gc90 + 0.000227524131045*gc120 + - 0.000219715131561895*gc1450 + 0.000130790232014624*gc180 + 0.0000172897232429118*gc210 + -0.0000351539361534969*gc240 + - 4.39790837733915*rateac90 + -2.98415098415002*rateac120 + - 7.39380526430144*rateac150 + -17.629378230225*rateac180 + - 0.592439544430542*rateac210 + 9.64618030974514*rateac240 + - 0.168364309432635*arate90 + 0.423706579409284*arate120 + 2.34920566907184*aarate150 + 0.55709654672278*arate180 + 0.419384469462554*arate210 + -6.87378578301999*arate240 + - 0.0167798986502698*crate90 + -0.435811237731758*crate120 + - 2.50056890528587*crate150 + -1.50169896523926*crate180 + 0.409815553085915*crate210 + -1.068654947684*crate240 + - 0.284918894322071*crelg90 + 0.0142683894710452*crelg120 + 0.0765473607284735*crelg150 + -0.0234794952274257*crelg180 + - 0.000884931750656463*crelg210 + 0.022739757364922*crelg240 + 0.0316056854877905*arelg90 + -0.0812688988772043*arelg120 + 0.0401908571928191*arelg150 + -0.00329318275227653*arelg180 + 0.0203527027866734*arelg210 + -0.0102051020541605*arelg240 + - 15.4434225055068)); |
| /* Final Layer Code */ |
| THETA1=-0.815666476185441*H1 + 1.55676890989212*H2+ 0.860049356896419*H3 + -1.21446959158613*H4 + 0.950263414656696*H5 + - 0.138477205146025*H6 + 0.424501292822833*H7 + 1.15814998352325*H8 + 0.582156905753725*H9 + 1.61215825742042*H10 + -1.71215214271274*H11 + 0.125974808712058*H12 + 0.970813875797162*H13 + 1.78976759567777*H14 + - 3.5475707287426*H15 + 0.424946068234655; |
| /* Response Mapping Code */ |
| /* construction for probabilities for target */ |
| target_G = Exp(THETA1); |
| sum = 1 + target_G; |
| target_G = target_G/sum; |
| or Probability of growth is Exp(Theta1)/1+Exp(Theta1) |
| target_I = 1 - target_G; |
| or Probability of inhibit is (1-Probability of growth) |

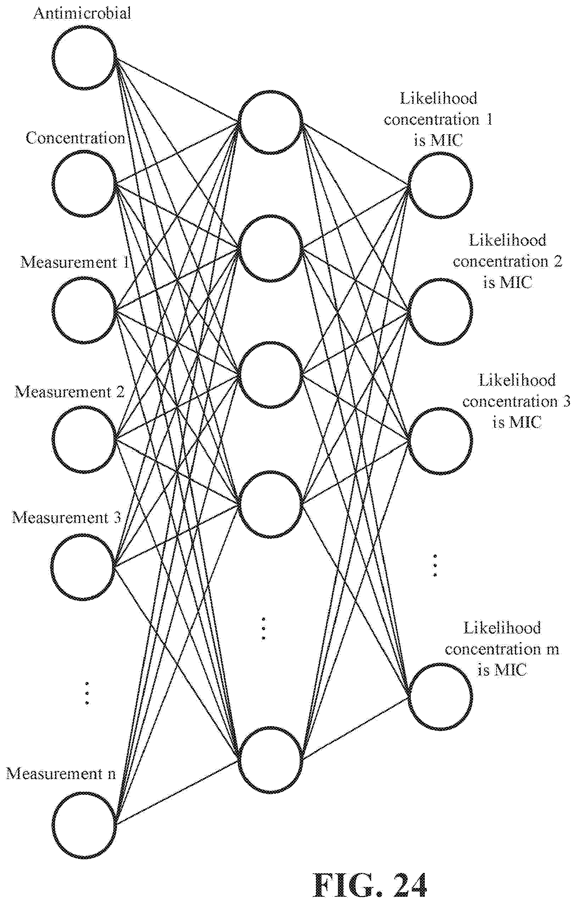

Variations are also possible in the way machine learning modules are used. For example, in some cases, rather than training a model to simply make a growth/inhibition prediction as described above, a model could be trained to make a MIC prediction directly. FIG. 24 provides an example of a structure for a neural network that could be used to make this type of prediction.

In some embodiments, to use a neural network such as illustrated in FIG. 24 to make a MIC prediction, a test well containing some concentration of an antimicrobial could be inoculated with a microorganism. Data could then be gathered by taking measurements in a manner similar to that described above in the context of the decision tree and neural network classifiers of FIGS. 20-23B and 25. These measurements, along with the identity and concentration of the antimicrobial in the test well could then be provided as inputs to the neural network's input nodes. The values provided on the network's output nodes could then be treated as indications of the likelihood that the concentrations corresponding to the output nodes were the MIC for the microorganism added to the test well. For example, when using a network which had been trained to provide a likelihood that a first concentration is the MIC on a first output node, a likelihood that a second concentration is the MIC on a second output node, and a likelihood that a third concentration is the MIC on a third output node, the concentration corresponding to the output node with the highest value (which might not be the same as the concentration of the antimicrobial in the test well from which the data was gathered) could be treated as the MIC.

In some embodiments, rather than using values of the output nodes of a network such as shown in FIG. 24 as directly indicating the MIC, values from such output nodes may be used as inputs to another function that would provide a (potentially more reliable) MIC value. For example, in some embodiments, separate sets of measurements could be gathered from each of a plurality of test wells having different concentrations of the same antimicrobial, and those sets of measurements could each be provided as input to a neural network such as illustrated in FIG. 24. Each of those neural networks would then provide a prediction of the most likely MIC, and those predictions could be provided as input to another function (e.g., a statistical function, such as a mode or median identification function) that would provide a prediction that would be treated as the true MIC for the sample. To illustrate, consider table 7, below, which shows how a predicted MIC was obtained from the outputs of a plurality of neural networks during testing.

**Table 7: Exemplary MIC derivation from output of multiple neural networks.**

| **Org** | **Drug** | **Dilution (µg/ml)** | **Predicted MIC (µg/ml)** | **Explanation** |
|---|---|---|---|---|
| | | | | In this example, 11 drug concentrations were tested, and each drug concentration well has a predicted MIC for this specimen for this drug, i.e., each well is predicting the MIC. |
| P. fluorescens | Caz (i.e., Ceftazadime) | 0.06 | **2** | The median of the dilutions is used to make the final predicted MIC, which in this case is 2. Note that the 4 dilution predicted a very different MIC, but the median of all the dilutions for this specimen predicts a final MIC of 2. The actual MIC for this example was 2. |
| P. fluorescens | Caz | 0.12 | **2** | |
| P. fluorescens | Caz | 0.25 | **2** | |
| P. fluorescens | Caz | 0.5 | **2** | |
| P. fluorescens | Caz | 1 | **2** | |
| P. fluorescens | Caz | 2 | **2** | |
| P. fluorescens | Caz | 4 | **32** | |
| P. fluorescens | Caz | 8 | **2** | |
| P. fluorescens | Caz | 16 | **2** | |
| P. fluorescens | Caz | 32 | **2** | |
| P. fluorescens | Caz | 64 | **2** | |

During testing, it was unexpectedly found that using this approach allowed a single neural network having the input nodes, output nodes, hidden layer code, and response mapping code set forth in table 8 to be used to create MIC predictions that had the essential and absolute agreements set forth in table 9 for data covering the species of *A*. *baumannii, C. freundii, E. aerogenes, E. cloacae, E. coli, K. oxytoca, K. pneumonia, P. mirabilis, P. aeruginosa, P. fluorescens, P. mirabilis, P. rettgeri, S. marcescens,* and *Salmonella* and the antimicrobials of Cefepime, Cefotaxime, Ceftazidime, Gentamicin, Levofloxacin, Meropenem, and Tetracycline.

**Table 8: Exemplary neural network parameters and code.**

| |
|---|
| /*%INPUT: Drug */ |
| /*%INPUT: Dilution */ |
| /*%INPUT: A60 */ - i.e., area covered by microorganisms in the test well after 60 minutes |
| /*%INPUT: A90 */ - i.e., area covered by microorganisms in the test well after 90 minutes |
| /*%INPUT: A120 */ - i.e., area covered by microorganisms in the test well after 120 minutes |
| /*%INPUT: A150 */ - i.e., area covered by microorganisms in the test well after 150 minutes |
| /*%INPUT: A180 */ - i.e., area covered by microorganisms in the test well after 180 minutes |
| /*%INPUT: A210 */ - i.e., area covered by microorganisms in the test well after 210 minutes |
| /*%INPUT: AC60 */ - i.e., ratio of area to count of microorganisms in the test well after 60 minutes |
| /*%INPUT: AC90 */ - i.e., ratio of area to count of microorganisms in the test well after 90 minutes |
| /*%INPUT: AC120 */ - i.e., ratio of area to count of microorganisms in the test well after 120 minutes |
| /*%INPUT: AC150 */ - i.e., ratio of area to count of microorganisms in the test well after 150 minutes |
| /*%INPUT: AC180 */ - i.e., ratio of area to count of microorganisms in the test well after 180 minutes |
| /*%INPUT: C60 */ - i.e., count of microorganisms in the test well after 60 minutes |
| /*%INPUT: C90 */ - i.e., count of microorganisms in the test well after 90 minutes |
| /*%INPUT: C120 */ - i.e., count of microorganisms in the test well after 120 minutes |
| /*%INPUT: C150 */ - i.e., count of microorganisms in the test well after 150 minutes |
| /*%INPUT: C180 */ - i.e., count of microorganisms in the test well after 180 minutes |
| /*%INPUT: C210 */ - i.e., count of microorganisms in the test well after 210 minutes |
| /*%INPUT: gac60 */ - i.e., the ratio of area to count in the growth (no antimicrobial) well after 60 minutes |
| /*%INPUT: gac90 */ - i.e., the ratio of area to count in the growth well after 90 minutes |
| /*%INPUT: gac120 */ - i.e., the ratio of area to count in the growth well after 120 minutes |
| /*%INPUT: gac150 */ - i.e., the ratio of area to count in the growth well after 150 minutes |
| /*%INPUT: gac180 */ - i.e., the ratio of area to count in the growth well after 180 minutes |
| /*%INPUT: gac210 */ - i.e., the ratio of area to count in the growth well after 210 minutes |
| /*%INPUT: ga60 */ - i.e., the area covered by microorganisms in the growth well after 60 minutes |
| /*%INPUT: ga90 */ - i.e., the area covered by microorganisms in the growth well after 90 minutes |
| /*%INPUT: ga120 */ - i.e., the area covered by microorganisms in the growth well after 120 minutes |
| /*%INPUT: ga150 */ - i.e., the area covered by microorganisms in the growth well after 150 minutes |
| /*%INPUT: ga180 */ - i.e., the area covered by microorganisms in the growth well after 180 minutes |
| /*%INPUT: ga210 */ - i.e., the area covered by microorganisms in the growth well after 210 minutes |
| /*%INPUT: gc60 */ - i.e., the count of microorganisms in the growth well after 60 minutes |
| /*%INPUT: gc90 */ - i.e., the count of microorganisms in the growth well after 90 minutes |
| /*%INPUT: gc120 */ - i.e., the count of microorganisms in the growth well after 120 minutes |
| /*%INPUT: gc150 */ - i.e., the count of microorganisms in the growth well after 150 minutes |
| /*%INPUT: gc180 */ - i.e., the count of microorganisms in the growth well after 180 minutes |
| /*%INPUT: gc210 */ - i.e., the count of microorganisms in the growth well after 210 minutes |
| /*%INPUT: rateac60 */ - i.e., the rate of change in the ratio of the area to count in the test well between 30 minutes and 60 minutes |
| /*%INPUT: rateac90 */ - i.e., the rate of change in the ratio of the area to count in the test well between 60 minutes and 90 minutes |
| /*%INPUT: rateac120 */ - i.e., the rate of change in the ratio of the area to count in the test well between 90 minutes and 120 minutes |
| /*%INPUT: rateac150 */ - i.e., the rate of change in the ratio of the area to count in the test well between 120 minutes and 150 minutes |
| /*%INPUT: rateac180 */ - i.e., the rate of change in the ratio of the area to count in the test well between 150 minutes and 180 minutes |
| /*%INPUT: rateac210 */ - i.e., the rate of change in the ratio of the area to count in the test well between 180 minutes and 210 minutes |
| /*%INPUT: arate60 */ - i.e., the rate of change in the area covered by microorganisms in the test well between 30 and 60 minutes |
| /*%INPUT: arate90 */ - i.e., the rate of change in the area covered by microorganisms in the test well between 60 and 90 minutes |
| /*%INPUT: arate120 */ - i.e., the rate of change in the area covered by microorganisms in the test well between 90 and 120 minutes |
| /*%INPUT: arate150 */ - i.e., the rate of change in the area covered by microorganisms in the test well between 120 and 150 minutes |
| /*%INPUT: arate180 */ - i.e., the rate of change in the area covered by microorganisms in the test well between 150 and 180 minutes |
| /*%INPUT: arate210 */ - i.e., the rate of change in the area covered by microorganisms in the test well between 180 and 210 minutes |
| /*%INPUT: crate60 */ - i.e., the rate of change in the count of microorganisms in the test well between 30 and 60 minutes |
| /*%INPUT: crate90 */ - i.e., the rate of change in the count of microorganisms in the test well between 60 and 90 minutes |
| /*%INPUT: crate120 */ - i.e., the rate of change in the count of microorganisms in the test well between 90 and 120 minutes |
| /*%INPUT: crate150 */ - i.e., the rate of change in the count of microorganisms in the test well between 120 and 150 minutes |
| /*%INPUT: crate180 */ - i.e., the rate of change in the count of microorganisms in the test well between 150 and 180 minutes |
| /*%INPUT: crate210 */ - i.e., the rate of change in the count of microorganisms in the test well between 180 and 210 minutes |
| /*%OUTPUT: _384_Wella_16 */ |
| /*%OUTPUT: _384_Wella_32 */ |
| /*%OUTPUT: _384_Wella_64 */ |
| /*%OUTPUT: _384_Wella_0_015 */ |
| /*%OUTPUT: _384_Wella_0_03 */ |
| /*%OUTPUT: _384_Wella_0_06 */ |
| /*%OUTPUT: _384_Wella_0_12 */ |
| /*%OUTPUT: _384_Wella_0_25 */ |
| /*%OUTPUT: _384 _Wella_0_5 */ |
| /*%OUTPUT: _384_Wella_1 */ |
| /*%OUTPUT: _384_Wella_16 */ |
| /*%OUTPUT: _384_Wella_2 */ |
| /*%OUTPUT: _384_Wella_32 */ |
| /*%OUTPUT: _384_Wella_4 */ |
| /*%OUTPUT: _384_Wella_64 */ |
| /*%OUTPUT: _384_Wella_8 */ |
| LABEL _ 384 _Wella_16 = 'Predicted: _384 _Wella=>16'; |
| LABEL _384_Wella _32 = 'Predicted: _384_Wella=>32'; |
| LABEL _384_Wella_64 = 'Predicted: _384_Wella=>64'; |
| LABEL _384_Wella_0_015 = 'Predicted: _384_Wella=0.015'; |
| LABEL _384_Wella_0_03 = 'Predicted: _384_Wella=0.03'; |
| LABEL _384_Wella_0_06 = 'Predicted: _384 _Wella=0.06'; |
| LABEL _ 384 _Wella_0_12 = 'Predicted: _384_Wella=0.12'; |
| LABEL _384_Wella_0_25 = 'Predicted: _384_Wella=0.25'; |
| LABEL _384_Wella_0_5 = 'Predicted: _384_Wella=0.5'; |
| LABEL 384 _Wella_1 = 'Predicted: _384_Wella=1'; |
| _ LABEL _384_Wella_16 = 'Predicted: _384_Wella=16'; |
| LABEL _384_Wella_2 = 'Predicted: _384_Wella=2'; |
| LABEL _384_Wella_32 = 'Predicted: _384_Wella=32'; |
| LABEL _384_Wella_4 = 'Predicted: _384_Wella=4'; |
| LABEL _384_Wella_64 = 'Predicted: _384_Wella=64'; |
| LABEL _384_Wella_8 = 'Predicted: _384_Wella=8'; |
| /* Transformation Code */ |
| /* Hidden Layer Code */ |
| H1 = tanh(.5*(-4.55424782042312*((Drug="Caz")-(Drug="Te")) + 0.561975394827427*((Drug="Cft")-(Drug="Te")) + 0.0178103737876131*((Drug="Cpe")-(Drug="Te")) + - 1.59884814313403*((Drug="Gm")-(Drug="Te")) + 0.61872932107782*((Drug="Lvx")-(Drug="Te")) + - 0.650013026061434*((Drug="Mer")-(Drug="Te")) + - 0.00473985600700199*Dilution + -0.0000716019647183412*A60 + - 0.0000496961955513778*A90 + -0.0000253388937638214*A120 + 0.0000002625720252509*A150 + 0.0000008135744354486*A180 + - 0.0000016030731967939*A210 + 0.034159663173171*AC60 + 0.111113620704464*AC90 + 0.237265127841344*AC120 + 0.0748089111348722*AC150 + -0.173903547451588*AC180 + - 0.000537534413848004*C60 + 0.000227298093885187*C90 + - 0.0000225506747816601*C120 + 0.0000336839791648029*C150 + 0.0000120243838767326*C180 + 0.0000118233859922471*C210 + 0.154976341424827*gac60 + 0.983974915521153*gac90 + - 0.447122406957271*gac120 + -0.269318472182602*gac150 + 0.205299434139941*gac180 + 0.209080414377943*gac210 + - 0.0000249890921998936*ga60 + -0.000106683501653873*ga90 + - 0.0000573518349266986*ga120 + 0.0000112700528774361*ga150 + 0.0000062370793938525*ga180 + 0.0000102252304637016*ga210 + 0.000607865815098916*gc60 + -0.00112731867552478*gc90 + - 0.000170816207571062*gc120 + 0.0000913554430601127*gc150 + 0.000047848346180216*gc180 + 0.0000719894997435259*gc210 + 0.143490719651955*rateac60 + 1.50175536774982*rateac90 + - 0.740706769918225*rateac120 + 0.6149718662759*rateac150 + - 1.43111557400087*rateac180 + -1.52054993688704*rateac210 + - 0.101386106775082*arate60 + 0.0720031400412117*arate90 + - 0.260603183827185*arate120 + 0.556939744261285*arate150 + 0.180120245786776*arate180 + 1.57836686855497*arate210 + - 0.264585955131948*crate60 + -0.0628933363990231*crate90 + |
| 0.17303028608517*crate120 + -0.148087907126341*crate150 + - 0.263263165167484*crate180 + -0.740568144611469*crate210 + - 7.96353021152842)); |
| H2 = tanh(.5*(-0.68561118062642*((Drug="Caz")-(Drug="Te")) + 0.555260885062402*((Drug="Cft")-(Drug="Te")) + - 0.8384434001 1 5728*((Drug="Cpe")-(Drug="Te")) + 2.3 8287 6 1710493 2* ((Drug=" Gm")- (Drug=" Te")) + - 1.40477324042629*((Drug="Lvx")-(Drug="Te")) + 1.61856342230398*((Drug="Mer")-(Drug="Te")) + - 0.00780026996521446*Dilution + 0.000077134884918636*A60 + 0.0000582395331377104*A90 + 0.0000162454225108147*A120 + 0.0000013496856129347*A150 + 0.0000004593551294793*A180 + 0.0000025227790819147*A210 + -0.0541260037619333*AC60 + 0.286834856881596*AC90 + -0.00353803998793339*AC120 + - 0.0254078892948951*AC150 + 0.0203917629618599*AC180 + - 0.00059836525765408*C60 + 0.0000273686165686692*C90 + - 0.0000561396758203549*C120 + -0.0000620351813944231 *C150 + - 0.0000276652360592924*C180 + 0.000000614621390308*C210 + 0.746206788435514*gac60 + -0.953055430696509*gac90 + - 0.842254861314274*gac120 + 0.520171255037211*gac150 + 0.480164569785779*gac180 + 0.438374629338116*gac210 + - 0.000117709921973074*ga60 + -0.000126006982199379*ga90 + - 0.0000084514396820874*ga120 + -0.000001856742155838*ga150 + - 0.0000010051155999557*ga180 + -0.000004594689928305*ga210 + - 0.00135400660831467*gc60 + 0.0000086957568844701*gc90 + 0.00024309576627734*gc120 + 0.000023914463948012*gc150 + - 0.0000127980245255971*gc180 + -0.0000487757258043663*gc210 + 1.62464200581121*rateac60 + 0.218720703227948*rateac90 + - 3.78461908606459*rateac120 + 4.19632599947592*rateac150 + 2.6177235440444*rateac180 + 0.206346497073155*rateac210 + 0.069179126357424*arate60 + -0.0102773745154557*arate90 + 0.109773502090162*arate120 + -0.145295153869756*arate150 + - 0.544016029657339*arate180 + 0.86998970704758*arate210 + - 0.268257471215735*crate60 + 0.0133413599358641*crate90 + 0.565612688322558*crate120 + -0.428843978388716*crate150 + - 0.970751181414429*crate180 + -0.262344502677242*crate210 + 2.17167746121481)); |
| H3 = tanh(.5*(-0.849902021126176*((Drug="Caz")-(Drug="Te")) + 0.5214458547463*((Drug="Cft")-(Drug="Te")) + 0.154368071276156*((Drug="Cpe")-(Drug="Te")) + 1.14930851314019*((Drug="Gm")-(Drug="Te")) + 1.26097742744338*((Drug="Lvx")-(Drug="Te")) + - 0.715264029301468*((Drug="Mer")-(Drug="Te")) + 0.00608536676016285*Dilution + 0.0000929963415937734*A60 + - 0.0000398597023193154*A90 + -0.000007070601766385*A120 + |
| 0.0000018365334254452*A150 + 0.0000045425153382006*A180 + - 0.0000004641202569268*A210 + 0.0667294074647125*AC60 + - 0.0280524072789369*AC90 + -0.0735120291203193*AC120 + - 0.052412721336399*AC150 + 0.0234590799033993*AC180 + 0.000684923047519617*C60 + -0.0000388419541403617*C90 + 0.0000355547421034935*C120 + -0.0000322186384352275*C150 + - 0.0000170293799157941*C180 + -0.0000301939941014876*C210 + - 0.894267787909701*gac60 + -0.697487940856172*gac90 + 0.0276523241360457*gac120 + 0.701383069304954*gac150 + 0.567122499486888*gac180 + -0.296768488952879*gac210 + 0.000324733558203196*ga60 + -0.0000406333656056838*ga90 + 0.0000213467692539717*ga120 + 0.0000168133677820647*ga150 + 0.0000006931171347735*ga180 + -0.0000036475061200401*ga210 + 0.00321919766231493*gc60 + 0.000500942242053422*gc90 + 0.000164995103995309*gc120 + 0.0000079310141236369*gc150 + - 0.0000280782299153558*gc180 + -0.0000377589627231411*gc210 + - 2.14920465938481*rateac60 + 0.829230761390482*rateac90 + - 1.08506059591287*rateac120 + -1.58193272400227*rateac150 + 0.0105355590410335*rateac180 + 0.205074809799282*rateac210 + 0.0854999843654999*arate60 + 0.177513418871244*arate90 + - 0.136276873620921*arate120 + -0.249201353706554*arate150 + - 0.152124790407909*arate180 + 0.00326146260583867*arate210 + - 0.0157777849811834*crate60 + -0.145705862460054*crate90 + - 0.42350622674 1 246 * crate 120 + -0.940565745772838*crate150 + 0.269673736451992*crate180 + 0.484753578796856*crate210 + 2.86057492222363)); |
| H4 = tanh(.5*(0.566735533016229*((Drug="Caz")-(Drug="Te")) + 0.739995849432032*((Drug="Cft")-(Drug="Te")) + - 1.23639330752971*((Drug="Cpe")-(Drug="Te")) + - 1.0226406462841*((Drug="Gm")-(Drug="Te")) + 0.0877615170450854*((Drug="Lvx")-(Drug="Te")) + - 3.90469452281071*((Drug="Mer")-(Drug="Te")) + 0.0192871827157567*Dilution + -0.000105168964233511*A60 + 0.0000152604179055053*A90 + 0.0000167102958217824*A120 + 0.0000005913984277249*A150 + -0.0000025176744757095*A180 + - 0.0000017948482859893*A210 + 0.246645873588222*AC60 + - 0.180191560587275*AC90 + -0.0552663834493609*AC120 + - 0.176734689795167*AC150 + -0.257747996485977*AC180 + - 0.000261787531516134*C60 + 0.000502081396951872*C90 + 0.000207083085415683*C120 + 0.0000058179752669871*C150 + 0.0000073970362849283*C180 + 0.0000103109777099969*C210 + 0.780092078722023*gac60 + -0.934246806295149*gac90 + 0.0430857363698831*gac120 + -0.0822811701001966*gac150 + 0.935887969141324*gac180 + -0.247241109321854*gac210 + 0.000184725504212636*ga60 + -0.0000752611522895085*ga90 + - |
| 0.0000221392941841813*ga120 + -0.0000202817096055973*ga150 + 0.0000034769788349079*ga180 + -0.0000023141659036314*ga210 + - 0.00158160467867741*gc60 + -0.000277188758656556*gc90 + - 0.000108594426098581*gc120 + -0.0000183513535267909*gc150 + 0.0000466052574607684*gc180 + 0.0000071261723043269*gc210 + - 2.12933344561129*rateac60 + 1.58177763325417*rateac90 + 1.27742034008295*rateac120 + -1.3857903056685*rateac150 + 1.86031741420219*rateac180 + 2.16929699257178*rateac210 + - 0.0519236176933813*arate60 + -0.0906895387384921*arate90 + 0.0273595464359536*arate120 + 0.426900583149606*arate150 + 0.44221653715769*arate180 + 0.564983937588253*arate210 + 0.164586696177753*crate60 + -0.042291870323269*crate90 + - 0.179924926509974*crate120 + 0.422004783242264*crate150 + 0.419400375584186*crate180 + -0.389979174299435*crate210 + 0.142590316198447)); |
| H5 = tanh(.5*(1.36596890423224*((Drug="Caz")-(Drug="Te")) + - 1.4475303400525*((Drug="Cft")-(Drug="Te")) + - 0.0251420856825452*((Drug="Cpe")-(Drug="Te")) + 0.189288553257507*((Drug="Gm")-(Drug="Te")) + - 0.669669801690211*((Drug="Lvx")-(Drug="Te")) + - 0.548292275872848*((Drug="Mer")-(Drug="Te")) + 0.00366502373312106*Dilution + -0.000128264019434088*A60 + - 0.0000449923000349789*A90 + 0.00003085329309746*A120 + 0.0000077338470802818*A150 + -0.0000018544110951683*A180 + - 0.0000007373589459167*A210 + 0.108502920628512*AC60 + 0.000622038543139749*AC90 + -0.130159194994696*AC120 + - 0.0993057353726485*AC150 + -0.116582001782146*AC180 + - 0.00097741563122961*C60 + -0.000111621804762964*C90 + 0.000109113250417542*C120 + -0.0000039410672524806*C150 + - 0.0000201441147472907*C180 + -0.0000144029765859724*C210 + - 1.40148747217075*gac60 + 0.793237168292246*gac90 + 0.0637720006487575*gac120 + 0.0292303997974253*gac150 + - 0.544111149184768*gac180 + -0.991540191797356*gac210 + 0.00012934154827996*ga60 + 0.000286891834474019*ga90 + 0.000046394632988084*ga120 + 0.0000047142102462053*ga150 + - 0.0000000014446654119*ga180 + 0.0000051622912236681*ga210 + - 0.00111813675334933*gc60 + 0.000766101623 156741*gc90 + - 0.0000604905098967774*gc120 + -0.0000645182827331432*gc150 + - 0.0000550338667179665*gc180 + -0.000009261386179533*gc210 + - 0.59790451054046*rateac60 + 0.241301321757376*rateac90 + - 0.297666683425254*rateac120 + -0.224799176804934*rateac150 + - 1.21748598829765*rateac180 + -5.40330597845438*rateac210 + - 0.358240538553932*arate60 + 0.404702021129394*arate90 + - 0.214725908694327*arate120 + -0.712731973820259*arate150 + - 0.18808974300813*arate180 + 0.0192607205470072*arate210 + - |
| 0.31740386871432*crate60 + -0.0640791664468304*crate90 + - 0.442311584989805*crate120 + -0.559457145368139*crate150 + - 0.322713426069697*crate180 + 0.15698545668711*crate210 + 11.75472487994)); |
| H6 = tanh(.5*(-0.672295615530724*((Drug="Caz")-(Drug="Te")) + 0.193888936122221*((Drug="Cft")-(Drug="Te")) + 1.27965850995998*((Drug="Cpe")-(Drug="Te")) + 1.43584991741796*((Drug="Gm")-(Drug="Te")) + - 0.394668783943975*((Drug="Lvx")-(Drug="Te")) + - 1.92772102730093*((Drug="Mer")-(Drug="Te")) + - 0.00379876578612891*Dilution + -0.000152649204716171*A60 + - 0.000073836758859624*A90 + -0.000012506895922238*A120 + 0.000000025107631799*A150 + -0.0000017882926632326*A180 + 0.0000008825237709848*A210 + 0.0645520035518182*AC60 + 0.150812382699767*AC90 + 0.153081285921259*AC120 + - 0.0283783649518231*AC150 + 0.0621259914252017*AC180 + - 0.0000810179069734667*C60 + -0.000520673630977109*C90 + - 0.0000277619997209515*C120 + 0.0000394225404894188*C150 + 0.0000293722877935603*C180 + 0.0000297343707845281*C210 + - 0.0774341834992982*gac60 + 0.613982792628955*gac90 + 1.02691139493504*gac120 + 1.31055912685482*gac150 + 0.431786411939769*gac180 + 0.98204318012007*gac210 + 0.000156928155732835*ga60 + 0.000224841958096158*ga90 + 0.0000149381592900022*ga120 + -0.0000134781747323539*ga150 + - 0.0000068886356502239*ga180 + 0.0000036757829527683*ga210 + 0.000109873629729478*gc60 + 0.000999150276035897*gc90 + - 0.000196220347728132*gc120 + -0.000114206790348538*gc150 + 0.00004983640341336*gc180 + 0.000085859444621443*gc210 + - 0.0906762924075144*rateac60 + 0.0274914378696596*rateac90 + - 1.07109657923677*rateac120 + -0.973358474430359*rateac150 + - 2.51540054416557*rateac180 + -1.4399681484816*rateac210 + - 0.0827429707534192*arate60 + 0.703184641550041*arate90 + - 0.479336011817217*arate120 + 0.586033717305062*arate150 + - 0.322352993983728*arate180 + 0.178650668996729*arate210 + - 0.207043565399784*crate60 + -0.0912679139962499*crate90 + 0.156939994539754*crate120 + -0.031405854404432*crate150 + - 0.79686701448911*crate180 + 0.630482194492482*crate210 + - 30.5218726041071)); |
| H7 = tanh(.5*(1.1371218452932*((Drug="Caz")-(Drug="Te")) + - 0.688936276977778*((Drug="Cft")-(Drug="Te")) + 1.84408537431566*((Drug="Cpe")-(Drug="Te")) + - 0.266414843699841*((Drug="Gm")-(Drug="Te")) + 2.09762593929503*((Drug="Lvx")-(Drug="Te")) + 1.59951852642801*((Drug="Mer")-(Drug="Te")) + 0.00530341659337711*Dilution + 0.000211034857860013*A60 + |
| 0.0000031897685544787*A90 + -0.0000153885682551032*A120 + 0.0000027398257274534*A150 + 0.0000064046677120069*A180 + 0.0000034242874261186*A210 + 0.0219409056260999*AC60 + 0.0170011021279569*AC90 + 0.170568190483146*AC120 + - 0.0904316053935454*AC150 + -0.13396859079975*AC180 + 0.000459630018793247*C60 + 0.0000822392244407297*C90 + - 0.000115413739058612*C120 + -0.0000129539109812691 *C150 + 0.0000011343164442289*C180 + -0.0000046466754112982*C210 + 2.26604865625884*gac60 + -0.623119763632109*gac90 + - 0.782499874137105*gac120 + 0.717504032727906*gac150 + - 0.399675813393717*gac180 + -0.732423095675785*gac210 + - 0.0000892786539658582*ga60 + 0.0000210289770491398*ga90 + 0.0000439193531793834*ga120 + 0.0000205582654104853*ga150 + - 0.0000016822907841864*ga180 + -0.0000023915920304265*ga210 + - 0.00201873298219092*gc60 + 0.000474646423129823*gc90 + 0.000373648263174738*gc120 + 0.0000336378389904502*gc150 + - 0.000037428492127897*gc180 + -0.0000149271423203322*gc210 + 0.645262329974964*rateac60 + -0.038663482599977*rateac90 + 0.894127465677666*rateac120 + -0.199756904203992*rateac150 + - 0.595643136147713*rateac180 + -2.0515476318619*rateac210 + 0.321183744839553*arate60 + -0.636504965186765*arate90 + 0.00566443723588453*arate120 + -0.0372528235629735*arate150 + - 0.137427322027206*arate180 + -0.0113374364674697*arate210 + 0.152288070906777*crate60 + 0.204035645000945*crate90 + 0.313628874282519*crate120 + -0.272902121080525*crate150 + - 0.172426324446118*crate180 + -0.234476472633933*crate210 + - 3.14191245841849)); |
| H8 = tanh(.5*(0.932764290558989*((Drug="Caz")-(Drug="Te")) + - 0.430402407760004*((Drug="Cft")-(Drug="Te")) + - 1.63438259363168*((Drug="Cpe")-(Drug="Te")) +-0.663708358224236*((Drug="Gm")-(Drug="Te")) + - 1.6810592439831*((Drug="Lvx")-(Drug="Te")) + 3.40494754663491*((Drug="Mer")-(Drug="Te")) + 0.0105430153940886*Dilution + -0.0000047831715004749*A60 + 0.0000421904026795931*A90 + 0.0000006475945580586*A120 + - 0.0000019648908886368*A150 + -0.0000035189322583634*A180 + 0.0000011832949498502*A210 + -0.00216744401393596*AC60 + - 0.0237101002588567*AC90 + -0.0443790471455064 * AC 120 + 0.0775280919484203*AC150 + -0.0193596720243765*AC180 + - 0.0010836920714677*C60 + 0.000116585300361968*C90 + - 0.0000023924692978101*C120 + 0.0000279757856647576*C150 + - 0.0000058399857117745*C180 + 0.0000107999506015439*C210 + - 0.226703453909654*gac60 + 0.948857207270458*gac90 + 0.566857916658987*gac120 + -0.058750753103414*gac150 + - 0.526320935962934*gac180 + -0.831024167729636*gac210 + - |
| 0.000188600926595037*ga60 + -0.000189598261520102*ga90 + 0.0000542685174477922*ga120 + 0.0000373178022445471*ga150 + 0.0000051226052732194*ga180 + -0.0000059853740157876*ga210 + 0.000368284669281717*gc60 + -0.00127218407920371*gc90 + 0.000180889619113105*gc120 + 0.000115688129932239*gc150 + - 0.0000424765812784238*gc180 + -0.0000951083702420526*gc210 + 0.301809839290138*rateac60 + 0.274840639790434*rateac90 + - 0.991762557330826*rateac120 + -1.19727828897446*rateac150 + - 1.17485190827342*rateac180 + -1.23914993976581*rateac210 + 0.0100951969443878*arate60 + -0.079960346271416*arate90 + 0.26071327164437*arate120 + -0.157955374455068*arate150 + 0.0098586757338883*arate180 + 0.317293621166267*arate210 + - 0.151452225482903*crate60 + -0.0843266579043151*crate90 + 0.296826838950813*crate120 + 0.305057664106348*crate150 + - 0.291846650634007*crate180 + 0.284773335411612*crate210 + 4.69389450931259)); |
| H9 = tanh(.5*(1.54958795535049*((Drug="Caz")-(Drug="Te")) + 0.495200078929188*((Drug="Cft")-(Drug="Te")) + - 0.14394453196633*((Drug="Cpe")-(Drug="Te")) + 1.48793514031965*((Drug="Gm")-(Drug="Te")) + - 1.60567236478778*((Drug="Lvx")-(Drug="Te")) + - 1.10353969091776*((Drug="Mer")-(Drug="Te")) + - 0.00257014116306035*Dilution + -0.000203819055532813*A60 + 0.0000296529803069037*A90 + 0.000011381520740921*A120 + - 0.0000084313665221906*A150 + -0.0000050523055890294*A180 + 0.0000037961546454979*A210 + -0.15680884397583*AC60 + - 0.0352083783384521*AC90 + -0.00473290780306647*AC120 + 0.145904228223169*AC150 + 0.106063415284739*AC180 + - 0.000134464458535032*C60 + 0.0000219599516690428*C90 + 0.0000766493829395313*C120 + -0.0000017347179201079*C150 + 0.0000227459514028102*C180 + 0.0000363314945218217*C210 + - 0.240659415926932*gac60 + -0.938243665011072*gac90 + - 0.374849299811987*gac120 + 0.21029217051649*gac150 + 0.584229596056013*gac180 + 0.83758811051175*gac210 + - 0.000151822495437797*ga60 + -0.000190742718472707*ga90 + - 0.0000596231902686967*ga120 + -0.0000023926196757104*ga150 + 0.0000008268525606537*ga180 + 0.0000050293085175949*ga210 + - 0.00245757027887857*gc60 + -0.000151631685195015*gc90 + - 0.000123202833484231*gc120 + 0.0000468159220982533*gc150 + - 0.0000196885367707044*gc180 + -0.0000137592691418769*gc210 + 2.96646863349999*rateac60 + -0.34622356526555*rateac90 + - 2.84011481743832*rateac120 + -1.1706679139977*rateac150 + 0.219755457992586*rateac180 + 4.83902484936668*rateac210 + - 0.146055809635628*arate60 + 0.0321460752914305*arate90 + - 0.0523173996860528*arate120 + 0.375417722577054*arate150 + - |
| 0.00393496498271822*arate180 + -0.584903755571126*arate210 + 0.356094403448173*crate60 + -0.0954315921795354*crate90 + - 0.0714644028534081*crate120 + 0.600060257745956*crate150 + 0.0853348893683977*crate180 + -0.328270697379932*crate210 + 2.76638089153125)); |
| H10 = tanh(.5*(0.711034775761523*((Drug="Caz")-(Drug="Te")) + 0.300671569281717*((Drug="Cft")-(Drug="Te")) + - 1.64587193893686*((Drug="Cpe")-(Drug="Te")) + - 1.81369887821046*((Drug="Gm")-(Drug="Te")) + 0.511798973257844*((Drug="Lvx")-(Drug="Te")) + 1.59456988047293*((Drug="Mer")-(Drug="Te")) + 0.00343851820730032*Dilution + -0.000188528983664629*A60 + - 0.0000309465243151169*A90 + -0.000041167940241115*A120 + - 0.0000084030620455804*A150 + -0.0000014063972672092*A180 + 0.0000024205523848494*A210 + 0.0516817173720267*AC60 + - 0.0967466653931919*AC90 + 0.129766740449898*AC120 + 0.0269698506089658*AC150 + 0.226791672863994*AC180 + - 0.000767885142905625*C60 + 0.00091579868217373*C90 + 0.000140855446834701*C120 + -0.0000044006160910948*C150 + 0.0000043251559375038*C180 + 0.0000077349729759489*C210 + 0.295414369450498*gac60 + -0.0308310745879374*gac90 + - 0.986137050376739*gac120 + -1.28619599966129*gac150 + 0.0895076453894242*gac180 + 1.48648262513835*gac210 + 0.0000088903827579182*ga60 + -0.000214738343138672*ga90 + 0.000023451058725205*ga120 + -0.0000180478098172938*ga150 + - 0.0000016250383936217*ga180 + 0.0000024018093034203*ga210 + 0.000599918332133457*gc60 + -0.00054781997317827*gc90 + 0.000624944338782551*gc120 + 0.000162445263447744*gc150 + - 0.0000000806068615833*gc180 + -0.000037239768829028*gc210 + 1.30716838139155*rateac60 + -0.410602683324547*rateac90 + - 0.883977031995655*rateac120 + -1.54826864448454*rateac150 + - 3.23730304245087*rateac180 + 0.179614096676577*rateac210 + - 0.0933058131946383*arate60 + -0.125377124438232*arate90 + - 0.310718057811251*arate120 + 0.508259413250422*arate150 + - 0.120481666109283*arate180 + 0.442205241034251*arate210 + 0.102979189246549*crate60 + -0.112442653246022*crate90 + 0.423721072986163*crate120 + 0.0787359687849293*crate150 + 0.262369796678796*crate180 + -0.606132740549201*crate210 + - 0.440807766160079)); |
| H11 = tanh(.5*(-0.442444284821728*((Drug="Caz")-(Drug="Te")) + - 0.707698499743234*((Drug="Cft")-(Drug="Te")) + - 0.969964208081883*((Drug="Cpe")-(Drug="Te")) + - 1.36459208874893*((Drug="Gm")-(Drug="Te")) + 1.49154993300449*((Drug="Lvx")-(Drug="Te")) + - 0.0379019305535625*((Drug="Mer")-(Drug="Te")) + |
| 0.00406687400848588*Dilution + -0.0000711793362459282*A60 + - 0.000009958466095487*A90 + 0.0000140621244035269*A120 + 0.0000101377333398228*A150 + 0.0000026662104626123*A180 + 0.0000036197274024637*A210 + 0.182835432875596*AC60 + - 0.0724423771311082*AC90 + 0.066778621032633*AC120 + - 0.0879298668086381*AC150 + -0.213172351687223*AC180 + - 0.000174828648102129*C60 + 0.000128375322144125*C90 + 0.0000243303119690779*C120 + 0.0000272493695563039*C150 + 0.000004220949977599*C180 + 0.0000080657000055476*C210 + 1.57591021861805*gac60 + 1.17029679434017*gac90 + 0.130407200071658*gac120 + 0.166558062687711*gac150 + 0.627723955884869*gac180 + 0.183514848329699*gac210 + 0.000351311200377612*ga60 + -0.0000629540877900904*ga90 + - 0.0000376505347749398*ga120 + -0.0000039925929851477*ga150 + 0.0000024159429502305*ga180 + 0.0000113425894147932*ga210 + 0.00101133932797758*gc60 + -0.000316292538412536*gc90 + 0.0000154226380152071*gc120 + 0.0000587132875943865*gc150 + 0.0000061862169693888*gc180 + 0.0000626086027371988*gc210 + - 0.612189051720599*rateac60 + 0.849031035024636*rateac90 + 0.456804311241401*rateac120 + 0.36691474012399*rateac150 + - 0.950473344091367*rateac180 + -0.410287089127733*rateac210 + - 0.181943440757191*arate60 + 0.0311741312229526*arate90 + 0.0504356213043603*arate120 + -0.010670647899239*arate150 + - 0.0982120067352041*arate180 + -0.0283891113310224*arate210 + - 0.0414485829604609*crate60 + -0.0437558384459912*crate90 + - 0.441081946224582*crate120 + 0.148749534521391*crate150 + - 0.101672388377872*crate180 + 0.265785008955112*crate210 + - 24.4811066513508)); |
| H12 = tanh(.5*(0.803714729445973*((Drug="Caz")-(Drug="Te")) + 2.90272297865265*((Drug="Cft")-(Drug="Te")) + - 2.45142115988862*((Drug="Cpe")-(Drug="Te")) + 1.96304928603422*((Drug="Gm")-(Drug="Te")) + - 1.71916189459251*((Drug="Lvx")-(Drug="Te")) + 1.12742215709736*((Drug="Mer")-(Drug="Te")) + 0.0074133208641436*Dilution + 0.000128780224758327*A60 + - 0.0000028220735721713*A90 + -0.000008537787243153*A120 + - 0.0000084591934326991*A150 + -0.0000048575057173566*A180 + - 0.0000018427352080614*A210 + -0.204124992995516*AC60 + 0.000833744506492574*AC90 + -0.0122389469626728*AC120 + 0.0554302535614537*AC150 + -0.0419076945572957*AC180 + - 0.000780788527281608*C60 + -0.000330504437532441*C90 + - 0.0000261519894994234*C120 + 0.0000800460924117741*C150 + 0.0000587870877293544*C180 + 0.0000466448395375917*C210 + 0.793863788241171*gac60 + 1.31502846290121*gac90 + - 0.153252740831981*gac120 + -0.179660565196688*gac150 + |
| 0.178330027260202*gac180 + 1.98604677011118*gac210 + - 0.000717857704328019*ga60 + 0.0000373188544671989*ga90 + 0.0000114202370302307*ga120 + 0.0000149443178382111*ga150 + 0.0000005302560500952*ga180 + 0.0000063267221564855*ga210 + - 0.00473524548182845*gc60 + -0.000262268415192743*gc90 + - 0.0000610099411281652*gc120 + 0.0000147931178927658*gc150 + - 0.0000078515215585892*gc180 + -0.0000134221282625086*gc210 + - 1.00854829993428*rateac60 + -0.503378679088597*rateac90 + - 0.625700264288145*rateac120 + -3.4495193185417*rateac150 + - 1.60008893843818*rateac180 + 0.42344259390747*rateac210 + 0.0342370283319816*arate60 + 0.235787496643151*arate90 + - 0.216060560415023*arate120 + 0.0225140128946343*arate150 + 0.187424292630901*arate180 + -0.505654003942949*arate210 + - 0.0468603279426172*crate60 + -0.0126634506083568*crate90 + 0.228029523263575*crate120 + 0.291401873953503*crate150 + 0.14202386334287*crate180 + -0.222221199454057*crate210 + - 16.7705318555362)); |
| H13 = tanh(.5*(-0.479797778318367*((Drug="Caz")-(Drug="Te")) + - 3.08951059068334*((Drug="Cft")-(Drug="Te")) + 1.25395772753422*((Drug="Cpe")-(Drug="Te")) + 1.29710916138866*((Drug="Gm")-(Drug="Te")) + 1.40391961733115*((Drug="Lvx")-(Drug="Te")) + - 0.326470683149212*((Drug="Mer")-(Drug="Te")) + - 0.00717352141180871*Dilution + 0.000057546385761008*A60 + 0.0000199568092502381*A90 + -0.0000073256114381153*A120 + 0.00000361230384191*A150 + 0.0000049421965550231*A180 + 0.0000006374877569545*A210 + -0.18462565121864*AC60 + - 0.0502499394016354*AC90 + 0.0746206505277938*AC120 + - 0.0782643874100686*AC150 + 0.0638579190393944*AC180 + - 0.000531289663864084*C60 + -0.00015268462966863*C90 + - 0.000108155360798308*C120 + -0.0000423096425713795*C150 + 0.0000037889070893654*C180 + 0.0000009257116116501*C210 + 0.73754348960741*gac60 + 0.0395625981333150*gac90 + 0.92117626606737*gac120 + 0.158343529486522*gac150 + 0.188008881716948*gac180 + -0.998633671469417*gac210 + - 0.0000583065333931441*ga60 + -0.000160013434125953*ga90 + 0.0000017330397940256*ga120 + 0.0000078019802958759*ga150 + 0.0000119207232391501*ga180 + -0.0000079145845519208*ga210 + - 0.000230967164840802*gc60 + 0.0000574373015138514*gc90 + 0.000140003360535301*gc120 + 0.00015350969893837*gc150 + 0.0000921297845913627*gc180 + -0.0000480980186192081*gc210 + 0.826494314003736*rateac60 + -0.333804276586628*rateac90 + - 0.0184590498021609*rateac120 + -3.80724981063126*rateac150 + - 2.57595497047839*rateac180 + -0.83847283016147*rateac210 + - 0.0867497091418313*arate60 + -0.180108997338579*arate90 + - |
| 0.408847106609092*arate120 + 0.155684341229394*arate150 + 0.198511133739555*arate180 + 0.154221900268227*arate210 + 0.405680212363798*crate60 + -0.240917531814249*crate90 + 0.0120244451218207*crate120 + 0.135706870229268*crate150 + 0.239969821251013*crate180 + -1.50684015211326*crate210 + - 3.81443651340348)); |
| H14 = tanh(.5*(1.2687825547767*((Drug="Caz")-(Drug="Te")) + - 1.77495468033 1 96*((Drug="Cft")-(Drug="Te")) + 2.330761727139*((Drug="Cpe")-(Drug="Te")) + - 0.801970864091597*((Drug="Gm")-(Drug="Te")) + 0.93061483431914*((Drug="Lvx")-(Drug="Te")) + 3.0347276207568*((Drug="Mer")-(Drug="Te")) + 0.00905333848716299*Dilution + -0.0000387927605955953*A60 + 0.0000425020249267229*A90 + 0.0000179147024949906*A120 + 0.0000042501508283453*A150 + -0.0000004732549511066*A180 + 0.0000002535786941546*A210 + -0.094856419298611*AC60 + - 0.050224092687271*AC90 + -0.0254092324932619*AC120 + - 0.00735035227571449*AC150 + 0.083654035105146*AC180 + 0.000248664738334494*C60 + 0.0000485589600160344*C90 + - 0.0000048911122509804*C120 + -0.0000169904253312522*C150 + - 0.0000169794266576861*C180 + -0.0000032713956182908*C210 + - 0.649981551436503*gac60 + 0.104128819009085*gac90 + 1.01011191649086*gac120 + 0.617929673656436*gac150 + - 0.0452143630652486*gac180 + -0.149835902002351*gac210 + 0.000130267561631577*ga60 + 0.000102598971853121*ga90 + 0.0000291130119568452*ga120 + 0.0000145526434452325*ga150 + 0.0000064692360546298*ga180 + 0.0000019265490583274*ga210 + 0.00118129865648602*gc60 + 0.000326830739834696*gc90 + - 0.0000223771755732627*gc120 + 0.0000492633798171483*gc150 + 0.0000402403455925824*gc180 + 0.0000169121652699485*gc210 + - 1.19582258918063*rateac60 + 0.523898296470249*rateac90 + - 1.06182394409601*rateac120 + -0.300703513226787*rateac150 + - 1.04965674384329*rateac180 + -1.84825390902025*rateac210 + 0.0538854448306092*arate60 + 0.0642629726552263*arate90 + - 0.135652062725095*arate120 + -0.184594099901545*arate150 + 0.114795199720034*arate180 + 0.107199252295908*arate210 + 0.0695429066614269*crate60 + 0.00317542203045142*crate90 + - 0.152332407003631*crate120 + 0.281042810307315*crate150 + 0.322853121806986*crate180 + 0.118913554939934*crate210 + - 13.1390945606885)); |
| H15 = tanh(.5*(1.27367049547503*((Drug="Caz")-(Drug="Te")) + 1.56061785957055*((Drug="Cft")-(Drug="Te")) + 3.72863678606863*((Drug="Cpe")-(Drug="Te")) + - 1.59786140518307*((Drug="Gm")-(Drug="Te")) + - 1.05687955446878*((Drug="Lvx")-(Drug="Te")) + - |
| 1.97045506866885*((Drug="Mer")-(Drug="Te")) + - 0.000860141524092481*Dilution + -0.0000108780340069828*A60 + 0.0000396959706989585*A90 + 0.000003624907672025*A120 + - 0.0000074379558207228*A150 + -0.000000313155339151*A180 + - 0.0000005865791547041*A210 + 0.0155099688137843*AC60 + - 0.0128259899116445*AC90 + 0.0874300434884509*AC120 + 0.0592438162087635*AC150 + -0.063242927503991*AC180 + - 0.000217669695766135*C60 + 0.000523913321589052*C90 + 0.0000423483956282264*C120 + -0.000007039184331871*C150 + - 0.0000104333548883157*C180 + 0.0000077531559860528*C210 + 0.487020771298581*gac60 + 0.184531057815411^{∗}gac90 + 0.834704632032675*gac120 + -0.6516480753481*gac150 + 0.0065778245828488*gac180 + 2.03421986568327*gac210 + 0.000076762907309766*ga60 + -0.0000921995473470863*ga90 + - 0.0000265700461080635*ga120 + -0.0000080409910083577*ga150 + 0.0000052123476138161*ga180 + 0.0000092221186352395*ga210 + - 0.000312967845137245*gc60 + -0.00100556246759271*gc90 + - 0.000270811937385767*gc120 + -0.0000028927335542928*gc150 + 0.0000318310629956187*gc180 + 0.000036422736097534*gc210 + - 0.402748701216039*rateac60 + 0.823370530423392*rateac90 + - 0.0134438219591159*rateac120 + -2.95215490554213*rateac150 + - 0.738261980037303*rateac180 + 1.78112412922792*rateac210 + - 0.234581136393705*arate60 + -0.0327864707915357*arate90 + 0.130216441052494*arate120 + -0.199573142998811*arate150 + 0.461457632067167*arate180 + -1.02098772703318*arate210 + 0.510126717608042*crate60 + 0.0485746169094956*crate90 + - 0.24797987524124*crate120 + 0.0907370998544347*crate150 + 0.502830660339717*crate180 + 0.876072243217604*crate210 + - 18.431304844771)); |
| /* Final Layer Code */ |
| THETA1=0.448109860001125*H1 + 0.585274409538855*H2 + - 1.54287550794778*H3 + 1.45742147077605*H4 + 1.77562294942118*H5 + - 6.27761390089231*H6 + 1.1869960498014*H7 + -0.0289132613265069*H8 + 1.89243099163383*H9 + -0.851863697286872*H10 + 1.23313558662471*H11 + -0.568286680705328*H12 + -1.92828192408443*H13 + 6.7225460781964*H14 + 5.43623384810481*H15 + -5.27950687714991; |
| THETA2=2.30309836952028*H1 + -3.74719493583621*H2 + - 2.76207876588187*H3 + 5.59186639435255*H4 + -1.66353075325961*H5 + - 2.52913726227958*H6 + 1.16273912049144*H7 + 2.84714189062433*H8 + 2.00899061664253*H9 + -8.44310624847262*H10 + 0.267663159322204*H11 + -9.72738804283792*H12 + -0.721025742263901*H13 + 0.320259886332635*H14 + 13.5927358164804*H15 + -6.56451795560769; THETA3=-3.8623554281867*H1 + -5.76215425161039*H2 + 6.77873813988771*H3 + 6.26131185999666*H4 + 1.60271712946071*H5 + - |
| 1.47732990297188*H6 + -5.03884098577123*H7 + 1.2426174583394*H8 + 2.12181741672179*H9 + 1.6180515530581*H10 + -1.2374246197232*H11 + 6.76196968535522*H12 + -5.59514883791286*H13 + 1.47858483830336*H14 + 2.22825551252485*H15 + -3.92663939474312; |
| THETA4=-2.99504697080743*H1 + -3.63416069443166*H2 + - 1.35993667950606*H3 + -1.94315832465739*H4 + 2.00465700177323*H5 + 0.55157487578422*H6 + -3.53229998627745*H7 + 1.62351529189416*H8 + 0.533363853848544*H9 + -8.24641026318202*H10 + -1.28733015098744*H11 + -4.1235171485767*H12 + 3.30366156780676*H13 + 9.28452918001928*H14 + - 3.70769483123128*H15 + -5.23919275888148; |
| THETA5=9.93146113114612*H1 + -8.75422665086103*H2 + - 4.41441206671862*H3 + -12.8509962164756*H4 + 4.80229829629299*H5 + - 5.17287246992593*H6 + -3.89112021257176*H7 + -3.57207229596685*H8 + - 1.90437396686423*H9 + 2.21496347208363*H10 + -1.48219137258818*H11 + - 0.0844681788197313*H12 + -0.227756566083175*H13 + - 1.56736305162217*H14 + 4.75042203396162*H15 + -3.49398122163591; THETA6=-6.05155134896019*H1 + -5.02878111089484*H2 + - 3.52322016343898*H3 + -7.13056864375409*H4 + 4.82325004263704*H5 + 2.05459109296963*H6 + -4.25539985850237*H7 + 7.40480622849679*H8 + - 0.663080033927679*H9 + -2.93161928675978*H10 + -1.09238929577345*H11 + 4.80542199691869*H12 + -5.97385725774731*H13 + -2.26650163477075*H14 + 7.06130660653269*H15 + -4.12847648431594; |
| THETA7=-7.32464794504945*H1 + 2.21926679873624*H2 + - 3.616238958143*H3 + -2.38427908132804*H4 + 3.71938433704259*H5 + 1.28477391119291*H6 + -1.63545931374813*H7 + -4.91481101589252*H8 + 0.135668807094692*H9 + -3.75399646937016*H10 + 2.94632461028069*H11 + 2.24856992679719*H12 + -0.762308891908463*H13 + 4.06179556781823*H14 + 4.31044746203816*H15 + -4.205856501667; |
| THETA8=-3.98073723921275*H1 + -3.63720322464119*H2 + 0.703591064496045*H3 + -5.2891081844093*H4 + -5.69081418141929*H5 + 3.76295649706744*H6 + -2.18400055112138*H7 + -3.8290552625935*H8 + - 4.16279614658787*H9 + -7.2913348840839*H10 + -7.10604808492075*H11 + 7.60680735505048*H12 + 6.18892463813744*H13 + -1.46443692734238*H14 + -5.92558826337853*H15 + -3.69716770596592; |
| THETA9=-10.4771017521003*H1 + 5.26270062881992*H2 + - 6.06759742131325*H3 + 6.07368892574189*H4 + 1.11396593044705*H5 + 9.54368205556282*H6 + 1.91896134997294*H7 + -12.2752339661305*H8 + 3.59481225184609*H9 + -0.509815538470254*H10 + -5.88157184146634*H11 + -5.77753456787085*H12 + 7.17834666326727*H13 + 6.78457617404532*H14 + -3.2517866785037*H15 + -1.35643401257472; |
| THETA10=2.29838206677043*H1 + 1.30718550797906*H2 + 1.96958763690255*H3 + 5.29916505626869*H4 + -0.576300273157774*H5 + 7.54451316799609*H6 + -3.67779539570955*H7 + -0.593114903710494*H8 + - 4.08883774586049*H9 + -2.38765061813365*H10 + -8.32690809171206*H11 + |
| -1.71550825942372*H12 + 6.9245633272652*H13 + -6.65455408016999*H14 + 6.35444527953592*H15 + 0.627065990860323; |
| THETA11=7.22289179530831*H1 + 1.50008415959505*H2 + - 5.92414292157721*H3 + 3.87027246814306*H4 + 6.03337659960812*H5 + - 3.18476963931444*H6 + -2.60139597331539*H7 + 8.40648966942569*H8 + - 0.476383315816606*H9 + 1.33709716396634*H10 + -6.21020880633463*H11 + -0.785358897958367*H12 + 4.38011280096845*H13 + 2.38979752643681*H14 + 15.9016779091557*H15 + -3.82281402620396; |
| THETA12=7.01732697853405*H1 + -5.60071326445127*H2 + 1.07079073697737*H3 + -2.08527551488845*H4 + 5.87679258056935*H5 + 2.76739613867928*H6 + 4.04054481318299*H7 + 6.48596236298034*H8 + 7.18978287702057*H9 + -11.1547550322708*H10 + 0.748434862255111*H11 + -3.50971511625884*H12 + 1.59339788377001*H13 + -9.93400364114984*H14 + 3.21230244752724*H15 + -0.994143492549278; |
| THETA13=-5.46270766369035*H1 + 5.09978812997716*H2 + 0.401702577985853*H3 + 5.97026341273713*H4 + 1.62473886247562*H5 + 6.32663511579362*H6 + -7.16603207141861*H7 + 4.83272638261094*H8 + 2.38377095612739*H9 + -5.41299735132255*H10 + 6.00802968994889*H11 + - 3.60653626661512*H12 + 4.32010366915005*H13 + -0.223820623398087*H14 + 1.03905425614484*H15 + -6.50045625223307; |
| THETA14=-3.66518324270255*H1 + 0.54633167491805*H2 + - 2.33429220359834*H3 + -0.932528960233431*H4 + 0.765229125800119*H5 + - 8.51755307788349*H6 + -10.5715731968488*H7 + 3.44067987215287*H8 + 0.14152409253156*H9 + 1.20080772104079*H10 + 7.95493209713549*H11 + - 0.234782356426792*H12 + 11.0738232745697*H13 + 4.95960571582252*H14 + -4.93664114239205*H15 + -2.19561286338951; |
| THETA15=-9.82936887384502*Hl + 1.28867466830731*H2 + - 2.69802680165775*H3 + 7.32056712552724*H4 + 9.94179568870123*H5 + 4.18927875457526*H6 + -3.69272962983193*H7 + -8.19006404100712*H8 + 1.35116855140603*H9 + 5.37866609295208*H10 + 5.34936862522398*H11 + 1.14146969239163*H12 + -7.17385271765782*H13 + 1.81355386361619*H14 + 2.20526645328883*H15 + -4.40567200268083; |
| /* Response Mapping Code */ |
| /* construction for probabilities for _384_Wella */ |
| MIC >16 = Exp(THETA1); |
| MIC >32 = Exp(THETA2); |
| MIC >64 = Exp(THETA3); |
| MIC 0.015 = Exp(THETA4); |
| MIC 0.03 = Exp(THETA5); |
| MIC 0.06 = Exp(THETA6); |
| MIC 0.12 = Exp(THETA7); |
| MIC 0.25 = Exp(THETA8); |
| MIC 0.5 = Exp(THETA9); |
| MIC 1 = Exp(THETA10); |
| MIC 16 = Exp(THETA11); |
| MIC 2 = Exp(THETA12); |
| MIC 32 = Exp(THETA13); |
| MIC 4 = Exp(THETA14); |
| MIC 64 = Exp(THETA15); |
| The final calculation for the probability is listed below |
| sum = 1 + _MIC>16 + MIC >32 + MIC >64 + _MIC 0.015 + _MIC 0.03 + _MIC 0.06 + MIC 0.12 + MIC 0.25 + MIC 0.5 + MIC 1 + MIC 16 + MIC 2 + MIC 32 + MIC 4 + MIC 64; |
| Probability of MIC>16 = _MIC>16/sum; |
| Probability of MIC>32 = _MIC>32/sum; |
| Probability of MIC >64 = _MIC>64/sum; |
| Probability of MIC 0.015 = MIC 0.015/sum; |
| Probability of MIC 0.03 = _MIC 0.03/sum; |
| Probability of MIC 0.06 = _MIC 0.061sum; |
| Probability of MIC 0.12 = _MIC 0.12/sum; |
| Probability of MIC 0.25 = _MIC 0.25/sum; |
| Probability of MIC 0.5 = _MIC 0.5/sum; |
| Probability of MIC 1 = MIC 1/sum; |
| Probability of MIC 16 = _MIC 16/sum; |
| Probability of MIC 2 = MIC 2/sum; |
| Probability of MIC 32 = _MIC 32/sum; |
| Probability of MIC 4 = _MIC 4/sum; |
| Probability of MIC 64 = MIC 64/sum; |
| For a given test well, the MIC is the one with the highest probability. |

**Table 9: Accuracy measurements obtained during testing of neural network MIC predictions.**

| **Bias** | **Relative to dried reference with known MIC** | **Relative to frozen reference with known MIC** |
|---|---|---|
| <=-2 | | |
| (i.e., the predicted MIC was less than the actual MIC, and there was at least one output node that corresponded to a MIC between the predicted MIC and the actual MIC) | 0 | 2 |
| -1 | | |
| (i.e., the predicted MIC was less than the actual MIC, but there was no output node that corresponded to a MIC between the predicted MIC and the actual MIC) | 0 | 19 |
| 0 | | |
| (i.e., agreement in MIC) | 130 | 83 |
| 1 | | |
| (i.e., the predicted MIC was greater than the actual MIC, but there was no output node that corresponded to a MIC between the predicted MIC and the actual MIC) | 0 | 24 |
| >=2 | | |
| (i.e., the predicted MIC was greater than the actual MIC, and there was at least one output node that corresponded to a MIC between the predicted MIC and the actual MIC) | 0 | 2 |
| Total drug/org combinations | 130 | 130 |
| Essential Agreement | | |
| (i.e., output node for predicted MIC was same as or adjacent to output node for actual MIC) | 100.0% | 96.9% |
| Absolute Agreement | | |
| (i.e., output node for predicted MIC was same as output node for actual MIC) | 100.0% | 63.8% |

As set forth above, neural networks can be used to model growth patterns of a bacterial isolate over time at various drug concentrations. As long as the bacteria grows in the well with no drug, then a neural network can be created to use the count and area of the growth well and at the various drug concentrations to make an early prediction of the MIC. Such networks can make predictions of MIC growth within the first six hours (or within the first 3.5-4 hours for the networks discussed in the context of tables 6 and 9) that had a high degree of agreement with actual MIC read at 16-24 hours. This can be applied to gram negative bacteria (including Enterobacteriaceae, Pseudomonas species, Acinetobacter species, Stenotrophamonas species, and Haemophilus species) and gram positive bacteria (including staphylococcus species, enterococcus species, and streptococcus species), and with drug classes of penicillins, beta-lactam, beta-lactam with inhibitor, carbapenems, cephalosporins, monobactams, tetracyclines, aminoglycosides, macrolides, glycopeptids, lincosamides, oxazolidinones, quinolones, folate inhibitors, and ansamycins.

After a model such as a decision tree classifier, ensemble of decision trees, neural network or other type of model has been created it, potentially with a plurality of other models (e.g., in embodiments in which specific models, such as the decision trees of figures 20-22 are used for specific microbe-antimicrobial combinations) would preferably be deployed to a computer system such as shown in figure 6 to be used in accelerating MIC determinations relative to what would be possible with traditional visual analysis. Then, when a sample was being analyzed on a machine controlled by or integrated with such a computer system, images and data corresponding to the images and data used to train the models could be captured and extracted using techniques such as described herein, and that data could be used to generate MIC predictions. In some variations, this may include identifying microorganisms in a sample (e.g., using a classifier such as described in Venkatesh Vijaykumar, Classifying Bacterial Species using Computer Vision and Machine Learning, International Journal of Computer Applications Vol. 151, No. 8, October, 2016 at 23-26, the disclosure of which is hereby incorporated by reference) and selecting an appropriate decision tree or other type of model. In other variations, this type of model selection may be omitted and instead a model that had been trained to make predictions across a variety of microorganisms and/or antimicrobials could be used.

### IV. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A method comprising: (a) incubating a first plurality of test mixtures in a plurality of test wells 20 using an incubator subsystem 7 of a biological testing system 1, wherein: (i) each test mixture from the first plurality of test mixtures is inoculated using a first biological sample; (ii) each test mixture from the first plurality of test mixtures has a concentration of a first antimicrobial agent; (iii) in each test mixture from the first plurality of test mixtures, the concentration of the first antimicrobial solution in that test mixture differs from the concentration of the first antimicrobial solution in each other test mixture from the first plurality of test mixtures; (iv) the same first biological sample is used to inoculate each test mixture from the first plurality of test mixtures; and (v) the first plurality of test mixtures comprises a growth mixture having a concentration of the first antimicrobial agent of zero; (b) at a plurality of imaging times, wherein each of the imaging times takes place after incubation has begun, for each test mixture from the first plurality of test mixtures, capturing an image of that test mixture using an AST camera 35; (c) obtaining a plurality of machine learning outputs by a processor 51 of a computer system 49 performing steps comprising, for each test mixture from the first plurality of test mixtures whose concentration of the first antimicrobial agent is greater than zero: (i) determining a plurality of characteristic values for that test mixture, wherein: (A) each of the characteristic values corresponds to a parameter from a plurality of parameters; and (B) the plurality of characteristic values for that test mixture are determined based on images captured of that test mixture at the plurality of imaging times; and (ii) providing the plurality of characteristic values determined for that test mixture to a machine learning model; and (d) the processor 51 of the computer system 49 generating a MIC prediction for the first biological sample based on the plurality of machine learning outputs.

### Example 2

The method of Example 1, wherein: (a) the plurality of imaging times comprises an earliest imaging time separated from onset of incubation by a first duration; (b) each imaging time from the plurality of imaging times except for the earliest imaging time is separated from its preceding imaging time by a second duration; and (c) the second duration is shorter than the first duration.

### Example 3

The method of Example 2, wherein the first duration is 90 minutes, and the second duration is 30 minutes.

### Example 4

The method of any of Examples 1-3, wherein the plurality of imaging times comprises a latest imaging time separated from the onset of incubation by four hours.

### Example 5

The method of any of Examples 1-4, wherein, for each test mixture from the first plurality of test mixtures whose concentration of the first antimicrobial agent is greater than zero: (a) the plurality of characteristic values for that test mixture comprises a first set of characteristic values and a second set of characteristic values; (b) the first set of characteristic values is based on images captured at a first time from the plurality of imaging times; (c) the second set of characteristic values is based on images captured at a second time from the plurality of imaging times; and (d) the plurality of parameters comprises a set of parameters, wherein each parameter from the set of parameters corresponds to one value from the first set of characteristic values and to one value from the second set of characteristic values.

### Example 6

The method of Example 5, wherein, for each test mixture from the first plurality of test mixtures whose concentration of the first antimicrobial agent is greater than zero, the plurality of characteristic values for that test mixture comprises, for each imaging time from the plurality of imaging times, a rate of change value for each parameter from the set of parameters.

### Example 7

The method of any of Examples 5-6, wherein, for each test mixture from the plurality of test mixtures whose concentration of the first antimicrobial agent is greater than zero: (a) the plurality of characteristic values comprises a growth set of characteristic values; (b) each parameter from the set of parameters corresponds to one characteristic value from the growth set of characteristic values for each imaging time from the plurality of imaging times; and (c) the characteristic values from the growth set of characteristic values are based on images captured of the growth mixture at the plurality of imaging times.

### Example 8

The method of any of Examples 5-7, wherein the set of parameters comprises microbial count.

### Example 9

The method of any of Examples 1-8, wherein: (a) the method further comprises: (i) incubating a second plurality of text mixtures, wherein: (A) each test mixture from the second plurality of test mixtures is inoculated using a second biological sample; (B) each test mixture from the second plurality of test mixtures has a concentration of a second antimicrobial agent; and (C) in each test mixture from the second plurality of text mixtures, the concentration of the second antimicrobial agent in that test mixture differs from the concentration of the second antimicrobial agent in each other test mixture from the second plurality of test mixtures; (ii) obtaining a second plurality of machine learning outputs by performing steps comprising, for each test mixture from the second plurality of test mixtures whose concentration of the second antimicrobial agent is greater than zero, providing a plurality of characteristic values determined for that test mixture to the machine learning model; and (b) for each test mixture from the first plurality of test mixtures whose concentration of the first antimicrobial agent is greater than zero and each test mixture from the second plurality of test mixtures whose concentration of the second antimicrobial agent is greater than zero, the machine learning model to which the plurality of characteristic values determined for that test mixture is provided is the same machine learning model.

### Example 10

The method of Example 9, wherein the first antimicrobial agent and the second antimicrobial agent are different.

### Example 11

The method of any of Examples 9-10, wherein the first antimicrobial agent and the second antimicrobial agent are each classified in a group consisting of: (a) beta-lactam; (b) aminoglycoside; (c) fluoroquinolones; (d) carbapenems; and (e) tetracyclines.

### Example 12

The method of any of Examples 9-11, wherein the first antimicrobial agent and the second antimicrobial agent are each from a group consisting of: (a) cefepime; (b) cefotaxime; (c) ceftazidime; (d) gentamicin; (e) levofloxacin; (f) meropenem; and (g) tetracycline.

### Example 13

The method of any of Examples 9-12, wherein: (a) the first biological sample comprises a first microorganism; (b) the second biological sample comprises a second microorganism; and (c) the first microorganism is different from the second microorganism.

### Example 14

The method of any of Examples 9-13, wherein the first and second microorganisms are both gram-negative.

### Example 15

The method of any of Examples 9-14, wherein the first microorganism and the second microorganism are both from the group consisting of: (a) Acinetobacter baumannii; (b) Citrobacter freundii; (c) Enterobacter aerogenes; (d) Enterobacter cloacae; (e) Eschericia coli; (f) Klebsiella oxytoca; (g) Klebsiella pneumonia; (h) Proteus mirabilis; (i) Pseudomonas aeruginosa; (j) Pseudomonas fluorescens; (k) Providendia rettgeri; (1) Serratia marcescens; (m) Salmonella typhi; and (n) Salmonella paratyphi.

### Example 16

The method of any of Examples 1-15, wherein: (a) obtaining the plurality of machine learning outputs comprises, for each test mixture from the first plurality of test mixtures whose concentration of the first antimicrobial agent is greater than zero, after providing the plurality of characteristic values determined for that test mixture to the machine learning model, obtaining an intermediate MIC prediction as a machine learning output for that test mixture; (b) generating the MIC prediction for the first biological sample comprises providing the plurality of machine learning outputs to a MIC creation function.

### Example 17

The method of Example 16, wherein the MIC prediction for the first biological sample is the median intermediate MIC prediction from the plurality of machine learning outputs.

### Example 18

The method of any of Examples 16-17, wherein, for at least one test mixture from the first plurality of test mixtures, the intermediate MIC prediction obtained as the machine learning output for that test mixture is a lower concentration of the first antimicrobial agent than the concentration of the first antimicrobial agent in that test mixture.

### Example 19

The method of any of Examples 16-18, wherein: (a) the machine learning model is a neural network having a plurality of output nodes; (b) each output node from the plurality of output nodes corresponds to a potential MIC; and (c) for each test mixture from the first plurality of test mixtures, the intermediate MIC prediction obtained as the machine learning output for that test mixture is the potential MIC corresponding to the output node having a highest value when the plurality of characteristic values determined for that test mixture are provided to the neural network.

### Example 20

The method of any of Examples 1-19, wherein, for each test mixture from the first plurality of test mixtures whose concentration of the first antimicrobial agent is greater than zero, an identification of the first antimicrobial agent is provided to the machine learning model along with the plurality of characteristic values determined for that test mixture.

### Example 21

The method of any of Examples 1-14, wherein: (a) obtaining the plurality of machine learning outputs comprises, for each test mixture from the first plurality of test mixtures whose concentration of the first antimicrobial agent is greater than zero, after providing the plurality of characteristic values determined for that test mixture to the machine learning model, obtaining a growth prediction as a machine learning output for that test mixture; (b) generating the MIC prediction for the first biological sample comprises identifying the test mixture with a lowest concentration of the first antimicrobial agent for which a growth prediction of inhibition was obtained as the machine learning output for that test mixture.

### Example 22

The method of Example 21, wherein: (a) the machine learning model is an ensemble comprising one or more decision trees, each having a plurality of leaf nodes, each leaf node connected to a parent node by a branch specifying growth or inhibition; and (b) for each test mixture from the first plurality of test mixtures whose concentration of the first antimicrobial agent is greater than zero, obtaining the growth prediction for that test mixture comprises predicting growth or inhibition based on whether there are more leaf nodes connected to parent nodes by branches specifying growth or whether there are more leaf nodes connected to parent nodes by branches specifying inhibition when the plurality of characteristic values for that test mixture are provided to the machine learning model.

### Example 23

The method of Example 21, wherein the machine learning model is a machine learning model trained to provide the growth prediction on an output node.

### Example 24

The method of any of Examples 9-14, wherein the first microorganism and the second microorganism are both from the group consisting of: (a) *Acinetobacter baumannii*; (b) *Citrobacter freundii;* (c) *Enterobacter cloacae;* (d) *Eschericia coli;* (e) *Klebsiella oxytoca*; (f) *Pseudomonas aeruginosa;* (g) *Pseudomonas fluorescens;* (h) *Serratia marcescens*; (i) *Salmonella typhi;* (j) *Salmonella paratyphi;* (k) *Proteus mirabilis;* and (1) *Providencia rettgeri.*

### Example 25

The method of any of Examples 1-21 or 23-24, wherein the set of parameters comprises: (a) microbial area; and (b) ratio of microbial area to microbial count.

### Example 26

The method of any of Examples 1-4 or 21, wherein: (a) the machine learning model is a decision tree classifier; (b) the method comprises: (i) determining an identification of a microbe comprised by the first biological sample; and (ii) selecting the decision tree classifier from a plurality of decision tree classifiers based on the identification of the microbe.

### Example 27

The method of Example 26, wherein the microbe comprised by the first biological sample has a type selected from a group of microbe types consisting of: (a) gram negative fermenters; (b) gram negative non-fermenters; (c) gram positive micrococcacaeae; and (d) gram-positive streptococcaceae.

### Example 28

The method of Example 27, wherein: (a) the type of the microbe comprised by the first biological sample is gram negative fermenters; and (b) the microbe comprised by the first biological sample is selected from a group of microbes consisting of: (i) *Aeromonas hydrophilia*; (ii) *Citrobacter freundii;* (iii) *Citrobacter koseri*; (iv) *Enterobacter aerogenes*; (v) *Enterobacter cloacae;* (vi) *Eschericia coli;* (vii) *Klebsiella oxytoca;* (viii) *Klebsiella pneumoniae*; (ix) *Morganelle morganii*; (x) *Proteus mirabilis;* (xi) *Proteus penneri*; (xii) *Proteus vulgaris*; (xiii) *Providencia stuartii*; (xiv) *Providencia rettgeri*; (xv) *Salmonella paratyphi;* (xvi) *Salmonella typhi;* (xvii) *Serratia marcescens;* (xviii) *Shigella dysenteriae*; (xix) *Shigella flexneri*; (xx) *Shigella sonnei*; (xxi) *Vibrio cholerae*; and (xxii) *Yersinia enterocolitica.*

### Example 29

The method of Example 27, wherein: (a) the type of the microbe comprised by the first biological sample is gram negative non-fermenter; and (b) the microbe comprised by the first biological sample is selected from a group of microbes consisting of: (i) *Acinetobacter baumannii*; (ii) *Acinetobacer haemolyticus*; (iii) *Acinetobacter lwoffii*; (iv) *Moraxella species*; (v) *Pseudomonas aeruginosa;* (vi) *Pseudomonas alcaligenes*; (vii) *Pseudomonas fluorescens;* (viii) *Pseudomonas putida*; and (ix) *Stenotrophamonas maltophilia.*

### Example 30

The method of Example 27, wherein: (a) the type of the microbe comprised by the first biological sample is gram-positive micrococcacaeae; and (b) the microbe comprised by the first biological sample is selected from a group of microbes consisting of: (i) *Staphylococcus aureus*; (ii) *Staphylococcus capitis*; (iii) *Staphylococcus epidermidis*; (iv) *Staphylococcus haemolyticus*; (v) *Staphylococcus saprophyticus*; (vi) *Staphylococcus warneri*; (vii) *Staphylococcus simulans*; and (viii) *Staphylococcus lugdenensis.*

### Example 31

The method of Example 27, wherein: (a) the type of the microbe comprised by the first biological sample is gram-positive streptococcaceae; and (b) the microbe comprised by the first biological sample is selected from a group of microbes consisting of: (i) *Enterococcus avium*; (ii) *Enterococcus faecalis*; (iii) *Enterococcus faecium*; (iv) *Enterococcus gallinarum*; (v) *Enterococcus raffinosus*; (vi) *Streptococcus bovis*; (vii) *Streptococcus pyogenes*; and (viii) *Streptococcus pneumoniae.*

### Example 32

The method of Example 26, wherein the microbe comprised by the first biological sample has a type selected from a group of microbe types consisting of: (a) enterobacteriaceae family; (b) pseudomonas family; (c) acinetobacter family; (d) micrococcacaeae family; and (e) streptococcaceae family.

### Example 33

The method of Example 26, wherein the first antimicrobial agent has a type selected from a group of antimicrobial types consisting of: (a) aminoglycoside; (b) ansamycin; (c) beta-lactam, penicillin class; (d) beta-lactam plus inhibitor combination; (e) carbapenems; (f) cephem; (g) fluoroquinolones; (h) folate pathway inhibitor; (i) fosfomycin; (j) glycopeptide; (k) lincosamide; (1) lipopeptide; (m) macrolide; (n) oxazolidinone; and (o) tetracyclines.

### Example 34

The method of Example 33, wherein: (a) the type of the first antimicrobial agent is aminoglycoside; and (b) the first antimicrobial agent is selected from a group of antimicrobial agents consisting of: (i) amikacin; (ii) gentamicin; and (iii) tobramycin.

### Example 35

The method of Example 33, wherein: (a) the type of the first antimicrobial agent is beta-lactam, penicillin class; and (b) the first antimicrobial agent is selected from a group of antimicrobial agents consisting of: (i) penicillin; and (ii) oxacillin.

### Example 36

The method of Example 33, wherein: (a) the type of the first antimicrobial agent is beta-lactam plus inhibitor combination; and (b) the first antimicrobial agent is selected from a group of antimicrobial agents consisting of: (i) amoxicillin + clavulanic acid; (ii) ampicillin + sulbactam; (iii) ceftazidime + avibactam; (iv) ceftolozane + tazobactam; (v) piperacillin + tazobactam; and (vi) ticarcillin + clavulanic acid.

### Example 37

The method of Example 33, wherein: (a) the type of the first antimicrobial agent is carbapenems; and (b) the first antimicrobial agent is selected from a group of antimicrobial agents consisting of: (i) doripenem; (ii) ertapenem; (iii) imipenem; and (iv) meropenem.

### Example 38

The method of Example 33, wherein: (a) the type of the first antimicrobial agent is cephem; and (b) the first antimicrobial agent is selected from a group of antimicrobial agents consisting of: (i) cefazolin; (ii) cefepime; (iii) cefotaxime; (iv) cefoxitin; (v) ceftaroline; (vi) ceftazidime; (vii) ceftriaxone; (viii) ceftizoxime; and (ix) cefuroxime.

### Example 39

The method of Example 33, wherein: (a) the type of the first antimicrobial agent is fluoroquinolones; and (b) the first antimicrobial agent is selected from a group of antimicrobial agents consisting of: (i) ciprofloxacin; and (ii) levofloxacin.

### Example 40

The method of Example 26, wherein: (a) the plurality of parameters consists of: (i) microbial area; (ii) difference in microbial area; and (iii) microbial growth; (b) the plurality of characteristic values comprises: (i) microbial area at a last imaging time from the plurality of imaging times; (ii) difference in microbial area from a second to last imaging time to the last imaging time; and (iii) microbial growth at the last imaging time.

### Example 41

A non-transitory computer readable medium encoding instructions for performing the method of any of Examples 1-40.

### Example 42

A computer system 49 configured with instructions for causing a biological testing system 1 to perform the method of any of Examples 1-40.

### Example 43

A biological testing system (1) comprising a processor (51) configured with a set of computer instructions operable, when executed, to cause the system (1) to perform a method comprising: (a) incubating a first plurality of test mixtures in a plurality of test wells (20) using an incubator subsystem (7) of the biological testing system (1), wherein: (i) each test mixture from the first plurality of test mixtures is inoculated using a first biological sample; (ii) each test mixture from the first plurality of test mixtures has a concentration of a first antimicrobial agent; (iii) in each test mixture from the first plurality of test mixtures, the concentration of the first antimicrobial solution in that test mixture differs from the concentration of the first antimicrobial solution in each other test mixture from the first plurality of test mixtures; (iv) the same first biological sample is used to inoculate each test mixture from the first plurality of test mixtures; and (v) the first plurality of test mixtures comprises a growth mixture having a concentration of the first antimicrobial agent of zero; (b) at a plurality of imaging times, wherein each of the imaging times takes place after incubation has begun, for each test mixture from the first plurality of test mixtures, capturing an image of that test mixture using an AST camera (35); (c) obtaining a plurality of machine learning outputs by the processor (51) performing steps comprising, for each test mixture from the first plurality of test mixtures whose concentration of the first antimicrobial agent is greater than zero: (i) determining a plurality of characteristic values for that test mixture, wherein: (A) each of the characteristic values corresponds to a parameter from a plurality of parameters; and (B) the plurality of characteristic values for that test mixture are determined based on images captured of that test mixture at the plurality of imaging times; and (ii) providing the plurality of characteristic values determined for that test mixture to a machine learning model; and (d) the processor (51) generating a MIC prediction for the first biological sample based on the plurality of machine learning outputs.

### Example 44

The biological testing system (1) of Example 43, wherein: (a) the plurality of imaging times comprises an earliest imaging time separated from onset of incubation by a first duration; (b) each imaging time from the plurality of imaging times except for the earliest imaging time is separated from its preceding imaging time by a second duration; and (c) the second duration is shorter than the first duration.

### Example 45

The biological testing system (1) of Example 44, wherein, for each test mixture from the first plurality of test mixtures whose concentration of the first antimicrobial agent is greater than zero: (a) the plurality of characteristic values for that test mixture comprises a first set of characteristic values and a second set of characteristic values; (b) the first set of characteristic values is based on images captured at a first time from the plurality of imaging times; (c) the second set of characteristic values is based on images captured at a second time from the plurality of imaging times; and (d) the plurality of parameters comprises a set of parameters, wherein each parameter from the set of parameters corresponds to one value from the first set of characteristic values and to one value from the second set of characteristic values.

### Example 46

The biological testing system of Example 45, wherein, for each test mixture from the first plurality of test mixtures whose concentration of the first antimicrobial agent is greater than zero, the plurality of characteristic values for that test mixture comprises, for each imaging time from the plurality of imaging times, a rate of change value for each parameter from the set of parameters.

### Example 47

The biological testing system (1) of any of Examples 45-46, wherein, for each test mixture from the plurality of test mixtures whose concentration of the first antimicrobial agent is greater than zero: (a) the plurality of characteristic values comprises a growth set of characteristic values; (b) each parameter from the set of parameters corresponds to one characteristic value from the growth set of characteristic values for each imaging time from the plurality of imaging times; and (c) the characteristic values from the growth set of characteristic values are based on images captured of the growth mixture at the plurality of imaging times.

### Example 48

The biological testing system (1) of any of Examples 43-47, wherein: (a) the method further comprises: (i) incubating a second plurality of text mixtures, wherein: (A) each test mixture from the second plurality of test mixtures is inoculated using a second biological sample; (B) each test mixture from the second plurality of test mixtures has a concentration of a second antimicrobial agent; and (C) in each test mixture from the second plurality of text mixtures, the concentration of the second antimicrobial agent in that test mixture differs from the concentration of the second antimicrobial agent in each other test mixture from the second plurality of test mixtures; (ii) obtaining a second plurality of machine learning outputs by performing steps comprising, for each test mixture from the second plurality of test mixtures whose concentration of the second antimicrobial agent is greater than zero, providing a plurality of characteristic values determined for that test mixture to the machine learning model; and (b) for each test mixture from the first plurality of test mixtures whose concentration of the first antimicrobial agent is greater than zero and each test mixture from the second plurality of test mixtures whose concentration of the second antimicrobial agent is greater than zero, the machine learning model to which the plurality of characteristic values determined for that test mixture is provided is the same machine learning model.

### Example 49

The biological testing system (1) of Example 48, wherein the first antimicrobial agent and the second antimicrobial agent are different.

### Example 50

The biological testing system (1) of any of Examples 48-49, wherein: (a) the first biological sample comprises a first microorganism; (b) the second biological sample comprises a second microorganism; and (c) the first microorganism is different from the second microorganism.

### Example 51

The biological testing system (1) of any of Examples 43-50, wherein: (a) obtaining the plurality of machine learning outputs comprises, for each test mixture from the first plurality of test mixtures whose concentration of the first antimicrobial agent is greater than zero, after providing the plurality of characteristic values determined for that test mixture to the machine learning model, obtaining an intermediate MIC prediction as a machine learning output for that test mixture; (b) generating the MIC prediction for the first biological sample comprises providing the plurality of machine learning outputs to a MIC creation function.

### Example 52

The biological testing system (1) of Example 51, wherein, for at least one test mixture from the first plurality of test mixtures, the intermediate MIC prediction obtained as the machine learning output for that test mixture is a lower concentration of the first antimicrobial agent than the concentration of the first antimicrobial agent in that test mixture.

### Example 53

The biological testing system (1) of any of Examples 51-52, wherein: (a) the machine learning model is a neural network having a plurality of output nodes; (b) each output node from the plurality of output nodes corresponds to a potential MIC; and (c) for each test mixture from the first plurality of test mixtures, the intermediate MIC prediction obtained as the machine learning output for that test mixture is the potential MIC corresponding to the output node having a highest value when the plurality of characteristic values determined for that test mixture are provided to the neural network.

### Example 54

The biological testing system (1) of any of Examples 43-53, wherein, for each test mixture from the first plurality of test mixtures whose concentration of the first antimicrobial agent is greater than zero, an identification of the first antimicrobial agent is provided to the machine learning model along with the plurality of characteristic values determined for that test mixture.

### Example 55

The biological testing system (1) of any of Examples 43-54, wherein: (a) obtaining the plurality of machine learning outputs comprises, for each test mixture from the first plurality of test mixtures whose concentration of the first antimicrobial agent is greater than zero, after providing the plurality of characteristic values determined for that test mixture to the machine learning model, obtaining a growth prediction as a machine learning output for that test mixture; (b) generating the MIC prediction for the first biological sample comprises identifying the test mixture with a lowest concentration of the first antimicrobial agent for which a growth prediction of inhibition was obtained as the machine learning output for that test mixture.

### Example 56

The biological testing system (1) of Example 55, wherein: (a) the machine learning model is an ensemble comprising one or more decision trees, each having a plurality of leaf nodes, each leaf node connected to a parent node by a branch specifying growth or inhibition; and (b) for each test mixture from the first plurality of test mixtures whose concentration of the first antimicrobial agent is greater than zero, obtaining the growth prediction for that test mixture comprises predicting growth or inhibition based on whether there are more leaf nodes connected to parent nodes by branches specifying growth or whether there are more leaf nodes connected to parent nodes by branches specifying inhibition when the plurality of characteristic values for that test mixture are provided to the machine learning model.

### Example 57

The biological testing system (1) of Example 55, wherein the machine learning model is a machine learning model trained to provide the growth prediction on an output node.

### Example 58

The biological testing system (1) of Example 43 wherein: (a) the machine learning model is a decision tree classifier; (b) the method comprises: (i) determining an identification of a microbe comprised by the first biological sample; and (ii) selecting the decision tree classifier from a plurality of decision tree classifiers based on the identification of the microbe.

### Example 59

The biological testing system (1) of Example 58, wherein: (a) the plurality of parameters consists of: (i) microbial area; (ii) difference in microbial area; and (iii) microbial growth; (b) the plurality of characteristic values comprises: (i) microbial area at a last imaging time from the plurality of imaging times; (ii) difference in microbial area from a second to last imaging time to the last imaging time; and (iii) microbial growth at the last imaging time.

### V. Miscellaneous

It should be understood that, in the above examples and the claims, a statement that something is "based on" something else should be understood to mean that it is determined at least in part by the thing that it is indicated as being based on. To indicate that something must be completely determined based on something else, it is described as being "based EXCLUSIVELY on" whatever it must be completely determined by.

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various versions of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, versions, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

### Clauses

**Clause 1A.** A method comprising:
(a) incubating a first plurality of test mixtures in a plurality of test wells using an incubator subsystem of a biological testing system, wherein:
   (i) each test mixture from the first plurality of test mixtures is inoculated using a first biological sample;
   (ii) each test mixture from the first plurality of test mixtures has a concentration of a first antimicrobial agent;
   (iii) in each test mixture from the first plurality of test mixtures, the concentration of the first antimicrobial solution in that test mixture differs from the concentration of the first antimicrobial solution in each other test mixture from the first plurality of test mixtures;
   (iv) the same first biological sample is used to inoculate each test mixture from the first plurality of test mixtures; and
   (v) the first plurality of test mixtures comprises a growth mixture having a concentration of the first antimicrobial agent of zero;
(b) at a plurality of imaging times, wherein each of the imaging times takes place after incubation has begun, for each test mixture from the first plurality of test mixtures, capturing an image of that test mixture using an antimicrobic susceptibility testing, AST, camera;
(c) obtaining a plurality of machine learning outputs by a processor of a computer system performing steps comprising, for each test mixture from the first plurality of test mixtures whose concentration of the first antimicrobial agent is greater than zero:
   (i) determining a plurality of characteristic values for that test mixture, wherein:
      (A) each of the characteristic values corresponds to a parameter from a plurality of parameters; and
      (B) the plurality of characteristic values for that test mixture are determined based on images captured of that test mixture at the plurality of imaging times;
         and
   (ii) providing the plurality of characteristic values determined for that test mixture to a machine learning model;
      and
(d) the processor of the computer system generating a minimum inhibitory concentration, MIC, prediction for the first biological sample based on the plurality of machine learning outputs.

**Clause 2A.** The method of **clause 1A, wherein:**
(a) the plurality of imaging times comprises an earliest imaging time separated from onset of incubation by a first duration;
(b) each imaging time from the plurality of imaging times except for the earliest imaging time is separated from its preceding imaging time by a second duration; and
(c) the second duration is shorter than the first duration.

**Clause 3A. The method of clause 2A,** wherein the first duration is 90 minutes, and the second duration is 30 minutes.

**Clause 4A. The method of any of clauses 1A-3A,** wherein the plurality of imaging times comprises a latest imaging time separated from onset of incubation by four hours.

**Clause 5A. The method of any of clauses 1A-4A**, wherein, for each test mixture from the first plurality of test mixtures whose concentration of the first antimicrobial agent is greater than zero:
(a) the plurality of characteristic values for that test mixture comprises a first set of characteristic values and a second set of characteristic values;
(b) the first set of characteristic values is based on images captured at a first time from the plurality of imaging times;
(c) the second set of characteristic values is based on images captured at a second time from the plurality of imaging times; and
(d) the plurality of parameters comprises a set of parameters, wherein each parameter from the set of parameters corresponds to one value from the first set of characteristic values and to one value from the second set of characteristic values.

**Clause 6A. The method of clause 5A,** wherein, for each test mixture from the first plurality of test mixtures whose concentration of the first antimicrobial agent is greater than zero, the plurality of characteristic values for that test mixture comprises, for each imaging time from the plurality of imaging times, a rate of change value for each parameter from the set of parameters.

**Clause 7A. The method of any of clauses 5A-6A,** wherein, for each test mixture from the plurality of test mixtures whose concentration of the first antimicrobial agent is greater than zero:
(a) the plurality of characteristic values comprises a growth set of characteristic values;
(b) each parameter from the set of parameters corresponds to one characteristic value from the growth set of characteristic values for each imaging time from the plurality of imaging times; and
(c) the characteristic values from the growth set of characteristic values are based on images captured of the growth mixture at the plurality of imaging times.

**Clause 8A. The method of any of clauses 5A-7A,** wherein the set of parameters comprises microbial count.

**Clause 9A. The method of any of clauses 1A-8A,** wherein:
(a) the method further comprises:
   (i) incubating a second plurality of text mixtures, wherein:
      (A) each test mixture from the second plurality of test mixtures is inoculated using a second biological sample;
      (B) each test mixture from the second plurality of test mixtures has a concentration of a second antimicrobial agent; and
      (C) in each test mixture from the second plurality of text mixtures, the concentration of the second antimicrobial agent in that test mixture differs from the concentration of the second antimicrobial agent in each other test mixture from the second plurality of test mixtures;
   (ii) obtaining a second plurality of machine learning outputs by performing steps comprising, for each test mixture from the second plurality of test mixtures whose concentration of the second antimicrobial agent is greater than zero, providing a plurality of characteristic values determined for that test mixture to the machine learning model; and
(b) for each test mixture from the first plurality of test mixtures whose concentration of the first antimicrobial agent is greater than zero and each test mixture from the second plurality of test mixtures whose concentration of the second antimicrobial agent is greater than zero, the machine learning model to which the plurality of characteristic values determined for that test mixture is provided is the same machine learning model.

**Clause 10A. The method of clause 9A,** wherein the first antimicrobial agent and the second antimicrobial agent are different.

**Clause 11A. The method of any of clauses 9A-10A,** wherein the first antimicrobial agent and the second antimicrobial agent are each classified in a group consisting of:
(a) beta-lactam;
(b) aminoglycoside;
(c) fluoroquinolones;
(d) carbapenems; and
(e) tetracyclines.

**Clause 12A. The method of any of clauses 9A-11A,** wherein the first antimicrobial agent and the second antimicrobial agent are each from a group consisting of:
(a) cefepime;
(b) cefotaxime;
(c) ceftazidime;
(d) gentamicin;
(e) levofloxacin;
(f) meropenem; and
(g) tetracycline.

**Clause 13A. The method of any of clauses 9A-12A**, wherein:
(a) the first biological sample comprises a first microorganism;
(b) the second biological sample comprises a second microorganism; and
(c) the first microorganism is different from the second microorganism.

**Clause 14A. The method of any of clauses 9A-13A**, wherein the first and second microorganisms are both gram-negative.

**Clause 15A. The method of any of clauses 9A-14A**, wherein the first microorganism and the second microorganism are both from the group consisting of:
(a) *Acinetobacter baumannii*;
(b) *Citrobacter freundii;*
(c) *Enterobacter aerogenes*;
(d) *Enterobacter cloacae;*
(e) *Eschericia coli;*
(f) *Klebsiella oxytoca;*
(g) *Klebsiella pneumoniae*;
(h) *Proteus mirabilis;*
(i) *Pseudomonas aeruginosa;*
(j) *Pseudomonas fluorescens;*
(k) *Providendia rettgeri*;
(l) *Serratia marcescens;*
(m) *Salmonella typhi;* and
(n) *Salmonella paratyphi.*

**Clause 16A. The method of any of clauses 1A-15A**, wherein:
(a) obtaining the plurality of machine learning outputs comprises, for each test mixture from the first plurality of test mixtures whose concentration of the first antimicrobial agent is greater than zero, after providing the plurality of characteristic values determined for that test mixture to the machine learning model, obtaining an intermediate MIC prediction as a machine learning output for that test mixture;
(b) generating the MIC prediction for the first biological sample comprises providing the plurality of machine learning outputs to a MIC creation function.

**Clause 17A. The method of clause 16A**, wherein the MIC prediction for the first biological sample is the median intermediate MIC prediction from the plurality of machine learning outputs.

**Clause 18A. The method of any of clauses 16A-17A**, wherein, for at least one test mixture from the first plurality of test mixtures, the intermediate MIC prediction obtained as the machine learning output for that test mixture is a lower concentration of the first antimicrobial agent than the concentration of the first antimicrobial agent in that test mixture.

**Clause 19A. The method of any of clauses 16A-18A**, wherein:
(a) the machine learning model is a neural network having a plurality of output nodes;
(b) each output node from the plurality of output nodes corresponds to a potential MIC; and
(c) for each test mixture from the first plurality of test mixtures, the intermediate MIC prediction obtained as the machine learning output for that test mixture is the potential MIC corresponding to the output node having a highest value when the plurality of characteristic values determined for that test mixture are provided to the neural network.

**Clause 20A. The method of any of clauses 1A-19A**, wherein, for each test mixture from the first plurality of test mixtures whose concentration of the first antimicrobial agent is greater than zero, an identification of the first antimicrobial agent is provided to the machine learning model along with the plurality of characteristic values determined for that test mixture.

**Clause 21A. The method of any of clauses 1A-14A**, wherein:
(a) obtaining the plurality of machine learning outputs comprises, for each test mixture from the first plurality of test mixtures whose concentration of the first antimicrobial agent is greater than zero, after providing the plurality of characteristic values determined for that test mixture to the machine learning model, obtaining a growth prediction as a machine learning output for that test mixture;
(b) generating the MIC prediction for the first biological sample comprises identifying the test mixture with a lowest concentration of the first antimicrobial agent for which a growth prediction of inhibition was obtained as the machine learning output for that test mixture.

**Clause 22A. The method of clause 21A**, wherein:
(a) the machine learning model is an ensemble comprising one or more decision trees, each having a plurality of leaf nodes, each leaf node connected to a parent node by a branch specifying growth or inhibition; and
(b) for each test mixture from the first plurality of test mixtures whose concentration of the first antimicrobial agent is greater than zero, obtaining the growth prediction for that test mixture comprises predicting growth or inhibition based on whether there are more leaf nodes connected to parent nodes by branches specifying growth or whether there are more leaf nodes connected to parent nodes by branches specifying inhibition when the plurality of characteristic values for that test mixture are provided to the machine learning model.

**Clause 23A. The method of clause 21A**, wherein the machine learning model is a machine learning model trained to provide the growth prediction on an output node.

**Clause 24A. The method of any of clauses 9A-14A**, wherein the first microorganism and the second microorganism are both from the group consisting of:
(a) *Acinetobacter baumannii*;
(b) *Citrobacter freundii;*
(c) *Enterobacter cloacae;*
(d) *Eschericia coli;*
(e) *Klebsiella oxytoca*;
(f) *Pseudomonas aeruginosa;*
(g) *Pseudomonas fluorescens;*
(h) *Serratia marcescens;*
(i) *Salmonella typhi;*
(j) *Salmonella paratyphi*;
(k) *Proteus mirabilis;* and
(l) *Providendia rettgeri.*

**Clause 25A. The method of any of clauses 1A-21A or 23A-24A**, wherein the set of parameters comprises:
(a) microbial area; and
(b) ratio of microbial area to microbial count.

**Clause 26A. The method of any of clauses 1A-4A or 21A** wherein:
(a) the machine learning model is a decision tree classifier;
(b) the method comprises:
   (i) determining an identification of a microbe comprised by the first biological sample; and
   (ii) selecting the decision tree classifier from a plurality of decision tree classifiers based on the identification of the microbe.

**Clause 27A. The method of clause 26A**, wherein the microbe comprised by the first biological sample has a type selected from a group of microbe types consisting of:
(a) gram negative fermenters;
(b) gram negative non-fermenters;
(c) gram positive micrococcacaeae; and
(d) gram-positive streptococcaceae.

**Clause 28A. The method of clause 27A**, wherein:
(a) the type of the microbe comprised by the first biological sample is gram negative fermenters; and
(b) the microbe comprised by the first biological sample is selected from a group of microbes consisting of:
   (i) *Aeromonas hydrophilia*;
   (ii) *Citrobacter freundii;*
   (iii) *Citrobacter koseri*;
   (iv) *Enterobacter aerogenes*;
   (v) *Enterobacter cloacae;*
   (vi) *Eschericia coli;*
   (vii) *Klebsiella oxytoca;*
   (viii) *Klebsiella pneumoniae;*
   (ix) *Morganelle morganii*;
   (x) *Proteus mirabilis;*
   (xi) *Proteus penneri*;
   (xii) *Proteus vulgaris*;
   (xiii) *Providencia stuartii*;
   (xiv) *Providendia rettgeri*;
   (xv) *Salmonella paratyphi*;
   (xvi) *Salmonella typhi;*
   (xvii) *Serratia marcescens;*
   (xviii) *Shigella dysenteriae*;
   (xix) *Shigella flexneri*;
   (xx) *Shigella sonnei*;
   (xxi) *Vibrio cholerae*; and
   (xxii) *Yersinia enterocolitica.*

**Clause 29A. The method of clause 27A**, wherein:
(a) the type of the microbe comprised by the first biological sample is gram negative non-fermenter; and
(b) the microbe comprised by the first biological sample is selected from a group of microbes consisting of:
   (i) *Acinetobacter baumannii*;
   (ii) *Acinetobacer haemolyticus*;
   (iii) *Acinetobacter lwoffii*;
   (iv) *Moraxella species*;
   (v) *Pseudomonas aeruginosa;*
   (vi) *Pseudomonas alcaligenes*;
   (vii) *Pseudomonas fluorescens;*
   (viii) *Pseudomonas putida*; and
   (ix) *Stenotrophamonas maltophilia.*

**Clause 30A. The method of clause 27A**, wherein:
(a) the type of the microbe comprised by the first biological sample is gram-positive micrococcacaeae; and
(b) the microbe comprised by the first biological sample is selected from a group of microbes consisting of:
   (i) *Staphylococcus aureus*;
   (ii) *Staphylococcus capitis*;
   (iii) *Staphylococcus epidermidis*;
   (iv) *Staphylococcus haemolyticus*;
   (v) *Staphylococcus saprophyticus*;
   (vi) *Staphylococcus warneri*;
   (vii) *Staphylococcus simulans*; and
   (viii) *Staphylococcus lugdenensis.*

**Clause 31A. The method of clause 27A**, wherein:
(a) the type of the microbe comprised by the first biological sample is gram-positive streptococcaceae; and
(b) the microbe comprised by the first biological sample is selected from a group of microbes consisting of:
   (i) *Enterococcus avium*;
   (ii) *Enterococcus faecalis*;
   (iii) *Enterococcus faecium*;
   (iv) *Enterococcus gallinarum*;
   (v) *Enterococcus raffinosus*;
   (vi) *Streptococcus bovis*;
   (vii) *Streptococcus pyogenes*; and
   (viii) *Streptococcus pneumoniae.*

**Clause 32A. The method of clause 26A**, wherein the microbe comprised by the first biological sample has a type selected from a group of microbe types consisting of:
(a) enterobacteriaceae family;
(b) pseudomonas family;
(c) acinetobacter family;
(d) micrococcacaeae family; and
(e) streptococcaceae family.

**Clause 33A. The method of clause 26A**, wherein the first antimicrobial agent has a type selected from a group of antimicrobial types consisting of:
(a) aminoglycoside;
(b) ansamycin;
(c) beta-lactam, penicillin class;
(d) beta-lactam plus inhibitor combination;
(e) carbapenems;
(f) cephem;
(g) fluoroquinolones;
(h) folate pathway inhibitor;
(i) fosfomycin;
(j) glycopeptide;
(k) lincosamide;
(l) lipopeptide;
(m) macrolide;
(n) oxazolidinone; and
(o) tetracyclines.

**Clause 34A. The method of clause 33A**, wherein:
(a) the type of the first antimicrobial agent is aminoglycoside; and
(b) the first antimicrobial agent is selected from a group of antimicrobial agents consisting of:
   (i) amikacin;
   (ii) gentamicin; and
   (iii) tobramycin.

**Clause 35A. The method of clause 33A**, wherein:
(a) the type of the first antimicrobial agent is beta-lactam, penicillin class; and
(b) the first antimicrobial agent is selected from a group of antimicrobial agents consisting of:
   (i) penicillin; and
   (ii) oxacillin.

**Clause 36A. The method of clause 33A**, wherein:
(a) the type of the first antimicrobial agent is beta-lactam plus inhibitor combination; and
(b) the first antimicrobial agent is selected from a group of antimicrobial agents consisting of:
   (i) amoxicillin + clavulanic acid;
   (ii) ampicillin + sulbactam;
   (iii) ceftazidime + avibactam;
   (iv) ceftolozane + tazobactam;
   (v) piperacillin + tazobactam; and
   (vi) ticarcillin + clavulanic acid.

**Clause 37A. The method of clause 33A**, wherein:
(a) the type of the first antimicrobial agent is carbapenems; and
(b) the first antimicrobial agent is selected from a group of antimicrobial agents consisting of:
   (i) doripenem;
   (ii) ertapenem;
   (iii) imipenem; and
   (iv) meropenem.

**Clause 38A. The method of clause 33A**, wherein:
(a) the type of the first antimicrobial agent is cephem; and
(b) the first antimicrobial agent is selected from a group of antimicrobial agents consisting of:
   (i) cefazolin;
   (ii) cefepime;
   (iii) cefotaxime;
   (iv) cefoxitin;
   (v) ceftaroline;
   (vi) ceftazidime;
   (vii) ceftriaxone;
   (viii) ceftizoxime; and
   (ix) cefuroxime.

**Clause 39A. The method of clause 33A**, wherein:
(a) the type of the first antimicrobial agent is fluoroquinolones; and
(b) the first antimicrobial agent is selected from a group of antimicrobial agents consisting of:
   (i) ciprofloxacin; and
   (ii) levofloxacin.

**Clause 40A. The method of clause 26A**, wherein:
(a) the plurality of parameters consists of:
   (i) microbial area;
   (ii) difference in microbial area; and
   (iii) microbial growth;
(b) the plurality of characteristic values comprises:
   (i) microbial area at a last imaging time from the plurality of imaging times;
   (ii) difference in microbial area from a second to last imaging time to the last imaging time; and
   (iii) microbial growth at the last imaging time.

**Clause 41A.** A non-transitory computer readable medium encoding instructions for performing the **method of any of clauses 1A-40A.**

**Clause 42A.** A computer system configured with instructions for causing a biological testing system to perform the **method of any of clauses 1A-40A.**

**Clause 43A.** A biological testing system comprising a processor configured with a set of computer instructions operable, when executed, to cause the system to perform a method comprising:
(a) incubating a first plurality of test mixtures in a plurality of test wells using an incubator subsystem of the biological testing system, wherein:
   (i) each test mixture from the first plurality of test mixtures is inoculated using a first biological sample;
   (ii) each test mixture from the first plurality of test mixtures has a concentration of a first antimicrobial agent;
   (iii) in each test mixture from the first plurality of test mixtures, the concentration of the first antimicrobial solution in that test mixture differs from the concentration of the first antimicrobial solution in each other test mixture from the first plurality of test mixtures;
   (iv) the same first biological sample is used to inoculate each test mixture from the first plurality of test mixtures; and
   (v) the first plurality of test mixtures comprises a growth mixture having a concentration of the first antimicrobial agent of zero;
(b) at a plurality of imaging times, wherein each of the imaging times takes place after incubation has begun, for each test mixture from the first plurality of test mixtures, capturing an image of that test mixture using an antimicrobic susceptibility testing, AST, camera;
(c) obtaining a plurality of machine learning outputs by the processor performing steps comprising, for each test mixture from the first plurality of test mixtures whose concentration of the first antimicrobial agent is greater than zero:
   (i) determining a plurality of characteristic values for that test mixture, wherein:
      (A) each of the characteristic values corresponds to a parameter from a plurality of parameters; and
      (B) the plurality of characteristic values for that test mixture are determined based on images captured of that test mixture at the plurality of imaging times;
         and
   (ii) providing the plurality of characteristic values determined for that test mixture to a machine learning model;
      and
(d) the processor generating a minimum inhibitory concentration, MIC, prediction for the first biological sample based on the plurality of machine learning outputs.

**Clause 44A.** The biological testing system of **clause 43A, wherein:**
(a) the plurality of imaging times comprises an earliest imaging time separated from onset of incubation by a first duration;
(b) each imaging time from the plurality of imaging times except for the earliest imaging time is separated from its preceding imaging time by a second duration; and
(c) the second duration is shorter than the first duration.

**Clause 45A.** The biological testing system **of clause 44A,** wherein, for each test mixture from the first plurality of test mixtures whose concentration of the first antimicrobial agent is greater than zero:
(a) the plurality of characteristic values for that test mixture comprises a first set of characteristic values and a second set of characteristic values;
(b) the first set of characteristic values is based on images captured at a first time from the plurality of imaging times;
(c) the second set of characteristic values is based on images captured at a second time from the plurality of imaging times; and
(d) the plurality of parameters comprises a set of parameters, wherein each parameter from the set of parameters corresponds to one value from the first set of characteristic values and to one value from the second set of characteristic values.

**Clause 46A.** The biological testing system **of clause 45A,** wherein, for each test mixture from the first plurality of test mixtures whose concentration of the first antimicrobial agent is greater than zero, the plurality of characteristic values for that test mixture comprises, for each imaging time from the plurality of imaging times, a rate of change value for each parameter from the set of parameters.

**Clause 47A. The biological testing system of any of clauses 45A-46A**, wherein, for each test mixture from the plurality of test mixtures whose concentration of the first antimicrobial agent is greater than zero:
(a) the plurality of characteristic values comprises a growth set of characteristic values;
(b) each parameter from the set of parameters corresponds to one characteristic value from the growth set of characteristic values for each imaging time from the plurality of imaging times; and
(c) the characteristic values from the growth set of characteristic values are based on images captured of the growth mixture at the plurality of imaging times.

**Clause 48A. The biological testing system of any of clauses 43A-47A**, wherein:
(a) the method further comprises:
   (i) incubating a second plurality of text mixtures, wherein:
      (A) each test mixture from the second plurality of test mixtures is inoculated using a second biological sample;
      (B) each test mixture from the second plurality of test mixtures has a concentration of a second antimicrobial agent; and
      (C) in each test mixture from the second plurality of text mixtures, the concentration of the second antimicrobial agent in that test mixture differs from the concentration of the second antimicrobial agent in each other test mixture from the second plurality of test mixtures;
   (ii) obtaining a second plurality of machine learning outputs by performing steps comprising, for each test mixture from the second plurality of test mixtures whose concentration of the second antimicrobial agent is greater than zero, providing a plurality of characteristic values determined for that test mixture to the machine learning model; and
(b) for each test mixture from the first plurality of test mixtures whose concentration of the first antimicrobial agent is greater than zero and each test mixture from the second plurality of test mixtures whose concentration of the second antimicrobial agent is greater than zero, the machine learning model to which the plurality of characteristic values determined for that test mixture is provided is the same machine learning model.

**Clause 49A.** The biological testing system **of clause 48A,** wherein the first antimicrobial agent and the second antimicrobial agent are different.

**Clause 50A. The biological testing system of any of clauses 48A-49A,** wherein:
(a) the first biological sample comprises a first microorganism;
(b) the second biological sample comprises a second microorganism; and
(c) the first microorganism is different from the second microorganism.

**Clause 51A. The biological testing system of any of clauses 43A-50A**, wherein:
(a) obtaining the plurality of machine learning outputs comprises, for each test mixture from the first plurality of test mixtures whose concentration of the first antimicrobial agent is greater than zero, after providing the plurality of characteristic values determined for that test mixture to the machine learning model, obtaining an intermediate MIC prediction as a machine learning output for that test mixture;
(b) generating the MIC prediction for the first biological sample comprises providing the plurality of machine learning outputs to a MIC creation function.

**Clause 52A. The biological testing system of clause 51A**, wherein, for at least one test mixture from the first plurality of test mixtures, the intermediate MIC prediction obtained as the machine learning output for that test mixture is a lower concentration of the first antimicrobial agent than the concentration of the first antimicrobial agent in that test mixture.

**Clause 53A. The biological testing system of any of clauses 51A-52A**, wherein:
(a) the machine learning model is a neural network having a plurality of output nodes;
(b) each output node from the plurality of output nodes corresponds to a potential MIC; and
(c) for each test mixture from the first plurality of test mixtures, the intermediate MIC prediction obtained as the machine learning output for that test mixture is the potential MIC corresponding to the output node having a highest value when the plurality of characteristic values determined for that test mixture are provided to the neural network.

**Clause 54A. The biological testing system of any of clauses 43A-53A**, wherein, for each test mixture from the first plurality of test mixtures whose concentration of the first antimicrobial agent is greater than zero, an identification of the first antimicrobial agent is provided to the machine learning model along with the plurality of characteristic values determined for that test mixture.

**Clause 55A. The biological testing system of any of clauses 43A-54A**, wherein:
(a) obtaining the plurality of machine learning outputs comprises, for each test mixture from the first plurality of test mixtures whose concentration of the first antimicrobial agent is greater than zero, after providing the plurality of characteristic values determined for that test mixture to the machine learning model, obtaining a growth prediction as a machine learning output for that test mixture;
(b) generating the MIC prediction for the first biological sample comprises identifying the test mixture with a lowest concentration of the first antimicrobial agent for which a growth prediction of inhibition was obtained as the machine learning output for that test mixture.

**Clause 56A.** The biological testing system **of clause 55A**, **wherein:**
(a) the machine learning model is an ensemble comprising one or more decision trees, each having a plurality of leaf nodes, each leaf node connected to a parent node by a branch specifying growth or inhibition; and
(b) for each test mixture from the first plurality of test mixtures whose concentration of the first antimicrobial agent is greater than zero, obtaining the growth prediction for that test mixture comprises predicting growth or inhibition based on whether there are more leaf nodes connected to parent nodes by branches specifying growth or whether there are more leaf nodes connected to parent nodes by branches specifying inhibition when the plurality of characteristic values for that test mixture are provided to the machine learning model.

**Clause 57A. The biological testing system of clause 55A**, wherein the machine learning model is a machine learning model trained to provide the growth prediction on an output node.

**Clause 58A. The biological testing system of clause 43A** wherein:
(a) the machine learning model is a decision tree classifier;
(b) the method comprises:
   (i) determining an identification of a microbe comprised by the first biological sample; and
   (ii) selecting the decision tree classifier from a plurality of decision tree classifiers based on the identification of the microbe.

**Clause 59A. The biological testing system of clause 58A**, wherein:
(a) the plurality of parameters consists of:
   (i) microbial area;
   (ii) difference in microbial area; and
   (iii) microbial growth;
(b) the plurality of characteristic values comprises:
   (i) microbial area at a last imaging time from the plurality of imaging times;
   (ii) difference in microbial area from a second to last imaging time to the last imaging time; and
   (iii) microbial growth at the last imaging time.

## Claims

1. A method comprising:
(a) incubating a first plurality of test mixtures (19) in a plurality of test wells (20) using an incubator subsystem (7) of a biological testing system (1), wherein:
(i) each test mixture from the first plurality of test mixtures (19) is inoculated using a first biological sample;
(ii) each test mixture from the first plurality of test mixtures (19) has a concentration of a first antimicrobial agent;
(iii) in each test mixture from the first plurality of test mixtures (19), the concentration of the first antimicrobial solution in that test mixture differs from the concentration of the first antimicrobial solution in each other test mixture from the first plurality of test mixtures (19);
(iv) the same first biological sample is used to inoculate each test mixture from the first plurality of test mixtures (19); and
(v) the first plurality of test mixtures (19) comprises a growth mixture having a concentration of the first antimicrobial agent of zero;
(b) at a plurality of imaging times, wherein each of the imaging times takes place after incubation has begun, for each test mixture from the first plurality of test mixtures (19), capturing an image (119) of that test mixture using an antimicrobic susceptibility testing, AST, camera (35);
(c) obtaining a plurality of machine learning outputs by a processor (51) of a computer system (49) performing steps comprising, for each test mixture from the first plurality of test mixtures (19) whose concentration of the first antimicrobial agent is greater than zero:
(i) determining a plurality of characteristic values for that test mixture, wherein:
(A) each of the characteristic values corresponds to a parameter from a plurality of parameters; and
(B) the plurality of characteristic values for that test mixture are determined based on images (119) captured of that test mixture at the plurality of imaging times;
(ii) providing the plurality of characteristic values determined for that test mixture to a machine learning model, wherein the machine learning model is a neural network having a plurality of output nodes, and each output node from the plurality of output nodes corresponds to a potential MIC; and
(iii) obtaining an intermediate MIC prediction as a machine learning output for that test mixture, wherein the intermediate MIC prediction obtained as the machine learning output for that test mixture is the potential MIC corresponding to the output node having a highest value when the plurality of characteristic values determined for that test mixture are provided to the neural network;
and
(d) the processor (51) of the computer system (49) generating a minimum inhibitory concentration, MIC, prediction for the first biological sample based on the plurality of machine learning outputs, said generating comprising providing the plurality of machine learning outputs to a MIC creation function.

2. The method of claim 1, wherein:
(a) the plurality of imaging times comprises an earliest imaging time separated from onset of incubation by a first duration;
(b) each imaging time from the plurality of imaging times except for the earliest imaging time is separated from its preceding imaging time by a second duration; and
(c) the second duration is shorter than the first duration,
wherein, optionally, the first duration is 90 minutes, and the second duration is 30 minutes.

3. The method of claim 1 or claim 2, wherein the plurality of imaging times comprises a latest imaging time separated from onset of incubation by four hours.

4. The method of any of claims 1 to 3, wherein, for each test mixture from the first plurality of test mixtures (19) whose concentration of the first antimicrobial agent is greater than zero:
(a) the plurality of characteristic values for that test mixture comprises a first set of characteristic values and a second set of characteristic values;
(b) the first set of characteristic values is based on images captured at a first time from the plurality of imaging times;
(c) the second set of characteristic values is based on images captured at a second time from the plurality of imaging times; and
(d) the plurality of parameters comprises a set of parameters, wherein each parameter from the set of parameters corresponds to one value from the first set of characteristic values and to one value from the second set of characteristic values,
wherein, optionally, for each test mixture from the first plurality of test mixtures (19) whose concentration of the first antimicrobial agent is greater than zero, the plurality of characteristic values for that test mixture comprises, for each imaging time from the plurality of imaging times, a rate of change value for each parameter from the set of parameters.

5. The method of claim 4, wherein, for each test mixture from the plurality of test mixtures (19) whose concentration of the first antimicrobial agent is greater than zero:
(a) the plurality of characteristic values comprises a growth set of characteristic values;
(b) each parameter from the set of parameters corresponds to one characteristic value from the growth set of characteristic values for each imaging time from the plurality of imaging times; and
(c) the characteristic values from the growth set of characteristic values are based on images captured of the growth mixture at the plurality of imaging times.

6. The method of claim 4 or claim 5, wherein the set of parameters comprises microbial count.

7. The method of any of claims 1 to 6, wherein:
(a) the method further comprises:
(i) incubating a second plurality of test mixtures, wherein:
(A) each test mixture from the second plurality of test mixtures is inoculated using a second biological sample;
(B) each test mixture from the second plurality of test mixtures has a concentration of a second antimicrobial agent; and
(C) in each test mixture from the second plurality of test mixtures, the concentration of the second antimicrobial agent in that test mixture differs from the concentration of the second antimicrobial agent in each other test mixture from the second plurality of test mixtures;
(ii) obtaining a second plurality of machine learning outputs by performing steps comprising, for each test mixture from the second plurality of test mixtures whose concentration of the second antimicrobial agent is greater than zero, providing a plurality of characteristic values determined for that test mixture to the machine learning model; and
(b) for each test mixture from the first plurality of test mixtures whose concentration of the first antimicrobial agent is greater than zero and each test mixture from the second plurality of test mixtures whose concentration of the second antimicrobial agent is greater than zero, the machine learning model to which the plurality of characteristic values determined for that test mixture is provided is the same machine learning model.

8. The method of claim 7, wherein the first antimicrobial agent and the second antimicrobial agent are different.

9. The method of claim 7 or claim 8, wherein the first antimicrobial agent and the second antimicrobial agent are each classified in a group consisting of:
(a) beta-lactam;
(b) aminoglyco side;
(c) fluoroquinolones;
(d) carbapenems; and
(e) tetracyclines.

10. The method of any of claims 7 to 9, wherein the first antimicrobial agent and the second antimicrobial agent are each from a group consisting of:
(a) cefepime;
(b) cefotaxime;
(c) ceftazidime;
(d) gentamicin;
(e) levofloxacin;
(f) meropenem; and
(g) tetracycline.

11. The method of any of claims 7 to 10, wherein:
(a) the first biological sample comprises a first microorganism;
(b) the second biological sample comprises a second microorganism; and
(c) the first microorganism is different from the second microorganism.

12. The method of any of claims 7 to 11, wherein the first and second microorganisms are both gram-negative.

13. A non-transitory computer readable medium (53, 55), for use in a biological testing system (1), having stored thereon computer program instructions which, when executed by one or more processors (51), cause the one or more processors (51) to perform the method of any of claims 1 to 12.

14. A computer system (49) configured with computer program instructions which, when executed by one or more processors (51) of a biological testing system (1), cause the one or more processors (51) to perform the method of any of claims 1 to 12.

15. A biological testing system (1) comprising one or more processors (51) configured with a set of computer program instructions which, when executed by the one or more processors (51), cause the one or more processors (51) to perform the method of any one of claims 1 to 12.
